# EUROPEAN PATENT SPECIFICATION

(11) **EP 1 337 850 B1**
(45) Date of publication and mention of the grant of the patent: **21.03.2007**
(21) Application number: 01994744.9
(22) Date of filing: 28.11.2001
(51) Int. Cl.: G01N 33/574, A61K 39/395

(54) **METHOD FOR DIAGNOSING A TUMOR IN A PATIENT DETERMINING THE CONCENTRATION OF PIBF**
VERFAHREN DER PIBF-KONZENTRATIONSBESTIMMUNG ZUR DIAGNOSE EINES TUMORS IN EINEM PATIENTEN
PROCEDE DE DIAGNOSTIC D'UNE TUMEUR CHEZ UN PATIENT, DETERMINANT LA CONCENTRATION DE PIBF

(30) Priority: 28.11.2000 AT 19972000
(43) Date of publication of application: 27.08.2003
(73) Proprietor: Intercell AG, 1030 Wien (AT)
(72) Inventor: SZEKERES-BARTHO, Julia, H-7626 Pecs (HU); NAGY, Eszter, A-1030 Vienna (AT); POLGAR, Beata, H-7624 Pecs (HU); PALKOVICS, Tamas, H-8360 Keszthely (HU); KOCH, Margit, A-1110 Vienna (AT); KLADE, Christoph, A-2700 Wr. Neustadt (AT); HENICS, Tamas, A-1030 Vienna (AT)
(74) Representative: Sonn, Helmut
(86) International application number: PCT/EP2001/013876
(87) International publication number: WO 2002/044734

(56) References cited:
- DATABASE EMBL [Online] AC: Y09631, XP002194124
- SZEKERES-BARTHO, J. ET AL: "The mechanism of the inhibitory effect of progesterone on lymphocyte cytotoxicity" AMERICAN JOURNAL OF REPRODUCTIVE IMMUNOLOGY AND MICROBIOLOGY, vol. 9, no. 1, September 1985 (1985-09), pages 15-18, XP001063083 cited in the application
- SZEKERES-BARTHO JULIA ET AL: "The immunological pregnancy protective effect of progesterone is manifested via controlling cytokine production." AMERICAN JOURNAL OF REPRODUCTIVE IMMUNOLOGY, vol. 35, no. 4, 1996, pages 348-351, XP001063023 ISSN: 1046-7408
- SZEKERES-BARTHO, J. ET AL: "The antiabortive effect of progesterone-induced blocking factor in mice is manifested by modulating NK activity" CELLULAR IMMUNOLOGY, vol. 177, no. 2, 1 May 1997 (1997-05-01), pages 194-199, XP001063024 cited in the application
- CHECK, J. H. ET AL: "A model for potential tumor immunotherapy based on knowledge of immune mechanisms responsible for spontaneous abortion." MEDICAL HYPOTHESES, vol. 57, no. 3, September 2001 (2001-09), pages 337-343, XP001063069 ISSN: 0306-9877

## Description

The present invention concerns a recombinant protein with a Progesterone-Induced Immunomodulatory Protein-(PIBF-)activity, a nucleic acid molecule encoding a recombinant protein with a PIBF activity, a nucleic acid vector comprising said nucleic acid sequence, a cell comprising said vector and a method for diagnosing a tumor in a patient.

For the maintenance of normal pregnancy, the production of progesterone - a steroid hormone with wide range of immunosuppressive effects - is absolutely essential. Peripheral lymphocytes from healthy pregnant women express nuclear receptors to sense this hormone (Szekeres-Bartho et al. J.Reprod.Immunol. 16, 239 (1989); Szekeres-Bartho et al. Cell. Immunol. 125, 273 (1990)), and produce a mediator protein named the Progesterone Induced Blocking Factor (PIBF) (Szekeres-Bartho et al. Am.J.Reprod.Immunol.Microbiol. 9, 15 (1985)). The sequence of the PIBF cDNA from human liver showed no significant homology to that of any of the known proteins (HSPIBF, Acc. No. Y09631). The encoded precursor protein is highly hydrophilic and has a molecular weight of 89 kDa. The naturally occuring PIBF as discoverred originally is a 34-36 kDa immunomodulatory protein with a sequence length of 757 amino acids.

It has been determined that the concentration of PIBF in urine samples of healthy persons is about 1-10 ng/ml whereas the concentration of PIBF in pregnant women from the 2^{nd} trimester ranges from about 70-150 ng/ml. These high levels quickly return to normal following abortion or labor.

PIBF which mediates the effects of progesterone is shown to have very potent immune modulatory function, both in vitro, as well as in vivo. Indeed, PIBF is shown to be essential for pregnancy in a mouse model, since PIBF isolated from culture supernatants of mouse lymphocytes protects fetuses from resorption induced by antiprogesterones. In addition, neutralizing antibodies against the mouse PIBF cause resorption of embryos and consequently abortus. The important role of PIBF in human reproduction has also been substantiated by measuring low levels in body fluids from pathologic pregnancies. PIBF plays an important role in the maintenance of pregnancy most likely by inhibiting natural killer lymphocytes. Importantly, by experimentally manipulating the amount of PIBF in vitro, one can modulate the killing activity of peripheral blood lymphocytes containing NK (natural killer) cells. It has been determined that there are at least two mechanisms of action of PIBF on NK cells. One is a direct inhibition of NK cell activity. NK cells kill their target cells by exocytosis of perforin and serine esterase-containing granules in the contact area between effector and target cells. Decidual lymphocytes - of which 60% carry NK surface markers - have high perforin content, however, they exert low cytotoxic activity. Although activated NK cells find and bind their targets in the presence of PIBF, they fail to release perforin from the storage granules, and as a result, there is no lysis of target cells. It seems that PIBF paralyses NK cells and holds the cytotoxic machinery under restraint by inhibiting degranulation and thus the release of killing substances.

There is another, indirect mechanism, by which PIBF exerts its anti-NK effect, through altered cytokine expression. In the presence of PIBF there is a significant decrease in TNFa (Tumor Necrosis Factor α) production by NK cells, which might also be involved in the down-regulation of NK activity. The amount of secreted TNFα is inversely related to PIBF production both in vitro and in vivo.

The second main mechanism of action of PIBF is the induction of T_{H2} cytokine dominance. T_{H2} dominance contributes to decreased cell mediated responses and B cell enhancement, whereas T_{H1} dominance results in decreased humoral responses and favors cellular immunological mechanisms. The secreted PIBF facilitates the production of T_{H2} cytokines, such as IL-3, IL-4 and IL-10, while it suppresses T_{H1} cytokines, such as IL-12 and IFN-y both in vitro and in vivo. Neutralization of PIBF by specific antibodies results in a T_{H1} shift in vivo, which is also a characteristics of failed pregnancies. The effect of PIBF on humoral immune responses is not only a simple enhancement but induction of the production of asymmetric antibodies. This is a population of antibodies (ab) which, owing to the presence of a mannose-rich oligosaccharide residue on one of the Fab arms of the molecule, possesses an asymmetric structure, and has no or low effector functions; however, these ab might act as blocking antibodies. The ratio of asymmetric IgG was significantly higher in supernatants of hybridoma cells cultured in the presence of PIBF than in those cultured in the absence of PIBF. Further studies revealed a positive relationship between asymmetric antibody content of the sera and PIBF expression on lymphocytes. Furthermore, blocking of progesterone receptors by RU 486 or neutralizing endogenous PIBF activity by specific anti-PIBF antibodies significantly reduced the production of asymmetric antibodies in pregnant mice.

Malignant tumors, i.e., cancers, are the second leading cause of death in all developed countries after heart disease and develop in one in three persons. One of every four persons dies of cancer. Cancer is characterized primarily by an increase in the number of abnormal, or neoplastic, cells derived from a normal tissue which proliferate to form a tumor mass, the invasion of adjacent tissues by these neoplastic tumor cells, and the generation of malignant cells which spread via the blood or lymphatic system to regional lymph nodes and to distant sites. The latter progression to malignancy is referred to as metastasis.

Cancer can result from a breakdown in the communication between neoplastic cells and their environment, including their normal neighbouring cells. Signals, both growth-stimulatory and growth-inhibitory, are routinely exchanged between cells within a tissue. Normally, cells do not divide in the absence of stimulatory signals, and, likewise, will cease dividing in the presence of inhibitory signals. In a cancerous, or neoplastic, state, a cell acquires the ability to "override" these signals and to proliferate under conditions in which normal cells would not grow.

Tumor cells must acquire a number of distinct aberrant traits to proliferate. Reflecting this requirement is the fact that the genomes of certain well-studied tumors carry several different independently altered genes, including activated oncogenes and inactivated tumor suppressor genes. Each of these genetic changes appears to be responsible for imparting some of the traits that, in aggregate, represent the full neoplastic phenotype.

Tumor cells carry antigens that can be recognized as being foreign to the body and that is one of the major functions of the immune system to eliminate such cells before they can form large tumors. This immune surveillance is clearly ineffective in patients with progressive malignant diseases. There is a range of protective measures identified that suppresses self-reactivity and may represent a major barrier in the immune system's ability to eradicate tumor cells. There are a number of mechanisms exerted by tumor cells such as 1. non-expression of classical and expression of non-classical self-identifying class I MHC molecules (such as HLA-G), which undermines the killing effect of (tumor)antigen specific class I MHC-restricted CTL_{S}; 2. biasing towards T_{H2} responses, suppressing T_{H1} helper function, and consequently effective cytotoxic anti-tumor responses; and 3. the production of immunosuppressive factors that down-regulate local and systemic immune responses (for example, secretion of TGF-β decreases T cell proliferation and cytotoxicity, expression of fas ligand, which induces apoptosis of CTLs.). As a result of these cumulative effects, tumors enjoy an immunologically privileged state, and grow without or with restricted control of the immune system.

It is fairly well established that many pathological conditions, such as infections, cancer, autoimmune disorders, etc., are characterized by the inappropriate expression of certain molecules. These molecules thus serve as "markers" for a particular pathological or abnormal condition. Apart from their use as diagnostic "targets", i.e., materials to be identified to diagnose these abnormal conditions, the molecules serve as reagents which can be used to generate diagnostic and/or therapeutic agents.

An aim of the present invention is a novel method for diagnosing a tumor in a patient which can be carried out easily and safely, which method does not require high-tech equipment, does not cause any particular trouble to the patient, which can be carried out quickly and which leads to results which allow to distinguish between a patient with a tumor and a healthy patient.

A further object of the present invention is to provide a kit for carrying out the method for diagnosing a tumor in a patient.

A still further object of the present invention is to provide an efficient anti-tumor medicine.

The method according to the present invention for diagnosing a tumor in a patient with which the above object is solved comprises taking a sample from the patient, measuring the concentration of PIBF (Progesterone Induced Blocking Factor) or a derivative thereof or a fragment thereof in the sample and determining whether the concentration of PIBF in the sample is above or below a predetermined threshold value, whereby the concentration above the threshold value identifies a patient with a tumor.

During the characterization of PIBF as an important immunomodulatory molecule for the maintenance of pregnancy, it has surprisingly been shown that tumor cells express PIBF or PIBF related substances, whereas no or low PIBF reactivity is found in the adjacent normal tissues. This indicates that PIBF is involved in the development or maintenance of immunological tolerance towards malignantly transformed cells and constitutes therefore a useful marker for tumor cells.

Therefore the method according to the present invention uses the fact that the concentration of PIBF in a sample taken from the patient to be tested is higher than the concentration of PIBF in a sample taken from a-healthy person.

In the scope of the present invention, the sample taken from the patient may be any kind of sample, fluid or not, from practically any part of the body. The concentration of the PIBF can be measured according to any method known in the art which enables to quantify the concentration of PIBF in a sample. This may comprise chemical, microbiologic, physical techniques, staining, etc. on fluids, tissue samples, etc. Possible methods include in vivo imaging with Computer Tomograph (CT) and Magnetic Resonance Image (MRI), after labeling with radionucleic and paramagnetic (e.g. gadolinium) labels, respectively, etc.

Since the PIBF may be submitted to metabolic or other changes in the body of the patient, the PIBF may comprise modifications depending on which sample has been taken from the patient. The PIBF may for example have been cleaved so that only a fragment of the PIBF is present in the given sample. The PIBF may furthermore have been modified so that a derivative of the PIBF is present in that sample or also a fragment of that derivative. It has also been shown that alternatively processed PIBF mRNA are present in tumor cells in a different concentration compared to normal cells, therefore, proteins or fragments translated from these different forms of mRNA molecules or fragments thereof are also comprised by the the term "fragments". Therefore, also the PIBF derivative, or a fragment of the PIBF or of the PIBF derivative or PIBF related substances (such as e.g. a cleaved product of 34 kDa or an alternatively spliced 14 kDa product) can be used as an indication of the concentration of the PIBF in the patient and therefore the respective concentration can be used for the method for diagnosing a tumor in a patient according to the present invention.
In the scope of the present invention the term "PIBF or fragments thereof" refers to - without being limited - to sequences according to SEQ ID Nos: 1, 3, 4, 6, 8, 10, 14, 15, 17, 19, 20, 23, 25, 27, 29, 31, 32, 34 and 36 or fragments or derivatives thereof. Therefore, examples of PIBF or fragments thereof which can be detected or quantified according to the present invention are these above mentioned sequences. Since it has been shown that exons 17 and 18 are included in almost all mRNA forms which have been identified PIBF fragments comprising exons 17 and 18 (see figs.) are preferably used for the detection or quantification of PIBF in a sample.

In the scope of the present invention, the fragment of the PIBF or of the PIBF-derivative may comprise for example less than 715 amino acids, preferably less than 500 amino acids, still preferred less than 200 amino acids, and most preferred less than 50 amino acids.

In the scope of the present invention, the term "derivative" comprises for example any naturally or even non naturally occurring modifications, e.g. cleavage, glycosylations, methylations, acetylations, amidations, phosphorylations, sulfatations, deletions, substitutions, etc.

Also, in the scope of the present invention, "threshold value" relates to a concentration value which will generally be the median sample concentration of PIBF in healthy sample donors. It is possible to take a known general median PIBF concentration in healthy people according to the literature or also to determine the sample concentration of PIBF in healthy donors when carrying out the present invention. The threshold value may also be determined in healthy (normal) samples taken earlier (in a healthy state) from the same person. Examples of such threshold values may be for example between 1 and 10 ng/ml, preferably between 1 and 5 ng/ml, whereby the concentration depends on the method of detection as well as the type of tumor. Furthermore, the threshold value may be zero in the case that alternatively processed PIBF mRNA products are present only in tumor cells and not in healthy cells. Therefore, the threshold value also depends on the PIBF molecule and must be determined for each specific PIBF molecule individually.

However, when determining the threshold value it is important that the sample from the healthy person is not taken from a pregnant woman since the PIBF concentration in samples of pregnant women is higher than the PIBF concentration in samples of non-pregnant women.

The concentration of PIBF measured in the sample taken from the patient which is above the predetermined threshold value identifies individuals with suspected tumor. A "tumor" as used herein refers to all neoplastic cell growth and proliferation, whether malignant or benign, and all pre-cancerous and cancerous cells and tissues.

Within the term "patient", in the scope of the present invention, patients with a tumor but also patients susceptible of having a tumor as well as healthy people who are having a general routine check are comprised. Of course the term "patient" may comprise also any animal, in particular mouse, rat, guinea pig, monkey, which animal is preferably a laboratory animal used for analyses, e.g. for detecting specific tumors, testing anti-tumor substances or carcinogenic substances. Furthermore, the animal may be a genetically modified animal which has a pre-disposition for developing tumors.

Since pregnancy also results in elevated PIBF levels, sexually active women have to be tested by conventional pregnancy tests (e.g. based on hCG) before considered as patients with tumor. It also means that it is very difficult to use this test to detect tumor growth when the patient is a pregnant woman beyond the first trimester. However, since a significant portion of the pregnancy-related malignancies are related to the uncontrolled growth of pregnancy-related tissues (such as trophoblast cells in mola hydatidosa), extremely high levels (> 150-200 ng/ml) of PIBF might indicate tumor growth with or without a viable baby present.

Preferably, the tumor to be diagnosed by the method according to the present invention is an epithelial carcinoma. Since the vast majority of human tumors (based on world wide morbidity data) are epithelial carcinomas (lung, breast, colon, etc.) the method according to the present invention is particularly advantageous for the diagnosis of this kind of tumor.

The epithelial carcinoma is preferably a lung carcinoma, colon carcinoma and breast carcinoma, respectively. The PIBF concentration in samples taken from patients with the above mentioned tumors is particularly high compared to the PIBF concentration in samples from healthy patients. Therefore, if the method according to the present invention is used for diagnosing one of the above mentioned tumors a concentration above a threshold value identifies individuals with a suspected tumor. However, a concentration below the threshold value does not necessarily exclude the presence of a tumor in certain cases.

According to an advantageous embodiment of the present invention the sample is a body fluid, preferably urine and serum, respectively. This enables a very simple way of taking the sample from the patient without any surgical step and without the necessity of specific high-tech instruments. The body fluid can be taken in any laboratory or even at the patient's home and is especially advantageous for a routine diagnosis, a diagnosis on a patient who is very weak and for regular checking of the progression of the tumor in a patient. The PIBF concentration can for example be measured with a dry chemistry method, e.g. a strip which will change its colour according to the PIBF concentration in a sample into which it is dipped.

Alternatively, the sample is a tissue sample. Even though the taking of this kind of sample from the patient is not as easy as the taking of a body fluid a method according to the present invention using a tissue sample from the patient enables the direct location of the tumor, especially if different tissue samples are taken and compared with each other. Furthermore, it is possible to directly follow the progressing of the tumor. Furthermore, by detecting the tissues with a tumor the method according to the present invention can further be used at least as an additional method for deciding which tissues and which parts of a body of the patient must be surgically removed.

According to preferred embodiment of the present invention the threshold value is the concentration of PIBF in a sample of a healthy person. Of course, the threshold value is particularly precise if it is the median concentration of PIBF of a plurality of samples of healthy persons.

Preferably, the threshold value is determined by measuring the concentration of PIBF in a sample of at least one healthy person parallel to the determination of the concentration of PIBF in a sample of the patient. Since the measured concentration depends on the method of measuring the PIBF concentration, the diagnosis is more specific and exact if the method for measuring the concentration of PIBF in the sample of the patient and in the sample of the healthy person are identical. In order to further enhance the sensitivity of the method the sample from the patient and the sample from the healthy person are preferably measured in parallel, e.g. at the same time in order to eliminate any interfering parameters, e.g. temperature, buffers etc. which have an influence on the result. The sample of the healthy person will preferably be measured in parallel as the "negative sample".

Advantageously, as a positive control the concentration of PIBF or a derivative thereof or a fragment thereof in a sample comprising a defined concentration of PIBF or a derivative thereof or a fragment thereof is measured parallel to the determination of the concentration of PIBF in the sample of the patient. The measuring in parallel of the positive control allows to control the results and detect any divergence in the method.

Preferably, the concentration of PIBF in the sample is measured immunologically, in particlular by a competitive assay, by a sandwich assay, by immunostaining or combinations of these methods. Any immunological method known to the person skilled in the art may be applied. Immunological methods are highly sensitive methods for detecting molecules and therefore particularly advantageous for the measuring of the PIBF concentration in the sample. To carry out the immunological method it is necessary to have at least one anti-PIBF antibody which will specifically bind to PIBF, derivatives thereof or fragments thereof. The antibody may be monoclonal or polyclonal and may further be recombinant. Furthermore, humanized monoclonal or phage encoded single-chain monoclonal antibodies may be used.

Examples of recombinant monoclonal anti-human PIBF antibodies which can be used as described above are deposited at the hybridoma cell bank at the University Medical School of Pécs, Department of Immunology and Biotechnology, Hungary, under the deposition Nos. 11 to 14/2001, cell line codes HYB.255-258.

"Single-chain antibodies" are structurally defined as comprising the binding portion of a first polypeptide from the variable region of an antibody, associated with the binding portion of a second polypeptide from the variable region of an antibody, the two polypeptides being joined by a peptide linker linking the first and second polypeptides into a single polypeptide chain. The single polypeptide chain thus comprises a pair of variable regions connected by a polypeptide linker. The regions may associate to form a functional antigen-binding site, as in the case wherein the regions comprise a light-chain and a heavy-chain variable region pair with appropriately paired complementarity determining regions (CDRs).

The term "humanized antibody" as used herein refers to antibody molecules in which amino acids have been replaced in the known antigen binding reagents in order to more closely resemble a human antibody and still retaining the original binding ability.

The antibodies may be generated using methods that are well known in the art. Such antibodies may include but are not limited to polyclonal, monoclonal, recombinant, chimeric, single chain, Fab fragments, and fragments produced by a Fab expression library. Neutralizing antibodies, (i.e., those which inhibit dimer formation) are especially preferred for therapeutic use.

For the production of antibodies, various hosts including goats, rabbits, rats, mice, chicken (Yab), humans, and others, may be immunized by injection with PIBF natural or recombinant protein or any fragment or oligopeptide thereof which has immunogenic properties or a PIBF-DNA (fragment). Depending on the host species, various adjuvants may be used to increase immunological response. Such adjuvants include but are not limited to Freund's, mineral gels such as aluminium hydroxide, and surface active substances such as lysolecithin, pluronic polyols, polyanions, aluminium, polycations (e.g. polyArg), peptides, oil emulsions, keyhole limpet hemocyanin, and dinitrophenol. Among adjuvants used in humans, BCG (bacilli Calmette-Guerin) and Corynebacterium parvum are especially preferable.

It is preferred that the peptides, fragments, or oligopeptides used to induce antibodies to PIBF have an amino acid sequence consisting of at least five amino acids, and more preferably at least 10 amino acids. It is also preferable that they are identical to a portion of the amino acid sequence of the natural protein. Short stretches of PIBF amino acids may be fused with those of another protein such as keyhole limpet hemocyanin and antibodies will be produced against the chimeric molecule.

Monoclonal antibodies to PIBF may be prepared using any technique which provides for the production of antibody molecules by continuous cell lines in culture. These include but are not limited to the hybridoma technique, the human B-cell hybridoma technique, and the EBV-hybridoma technique.

In addition, techniques developed for the production of "chimeric antibodies", the splicing of mouse antibody genes to human antibody genes to obtain a molecule with appropriate antigen specificity and biological activity can be used. Alternatively, techniques described for the production of single chain antibodies may be adapted, using methods known in the art, to produce PIBF-specific single chain antibodies. Antibodies with related specificity, but of distinct idiotypic composition, maybe generated by chain shuffling from random combinatorial immunoglobulin libraries.

Antibodies may also be produced by inducing in vivo production in the lymphocyte population or by screening recombinant immunoglobulin libraries or panels of highly specific binding reagents.

Antibody fragments which contain specific binding sites for PIBF may also be generated. For example, such fragments include but are not limited to the F(ab')2 fragments which can be produced by pepsin digestion of the antibody molecule and the Fab fragments which can be generated by reducing the disulfide bridges of the F(ab')2 fragments. Alternatively, Fab expression libraries may be constructed to allow rapid and easy identification of monoclonal Fab fragments with the desired specificity.

Various immunoassays may be used for screening to identify antibodies having the desired specificity. Numerous protocols for competitive binding or immunoradiometric assays using either polyclonal or monoclonal antibodies with established specificities are well known in the art. Such immunoassays typically involve the measurement of complex formation between PIBF and its specific antibody. A two-site, monoclonal-based immunoassay utilizing monoclonal antibodies reactive to two non-interfering PIBF epitopes is preferred, but a competitive binding assay may also be employed.

In one preferred form the concentration of PIBF in the sample is measured by a competitive assay. According to this method a solid phase is covered with preferably recombinant human PIBF (or its variants) at a specific concentration. Labelled anti-PIBF antibodies are added together with the examples to be measured. The higher is the PIBF concentration in the sample the lower is the corresponding detected value. Based on these readings the absolute concentration of the PIBF can be determined. This is a particularly precise method especially when the sample is a body fluid and can be carried out for example with an ELISA.

According to a further preferred embodiment of the invention, the concentration of PIBF in a sample is measured by a sandwich assay. For this assay it is necessary to have two anti-PIBF antibodies which each bind to a different epitope of the PIBF molecule. The first anti-PIBF antibody is preferably immobilized to a solid support after which the sample which is to be measured is added so that the PIBF present in the sample binds to the first anti-PIBF antibody. A second anti-PIBF antibody which is preferably labelled is added so that it binds to the bound PIBF. The amount of bound second anti-PIBF antibody is measured and is used as indication for the absolute concentration of the PIBF in the sample. Also this method is preferably used when the sample to be measured is a body fluid of the patient and can be carried out by ELISA.

According to another preferred embodiment to the present invention the concentration of PIBF in a sample is measured by immunostaining. This method is preferably used when the sample which is to be measured is a tissue sample of the patient. According to this method anti-PIBF antibody is directly added to the tissue sample of the patient where it binds to PIBF present in the tissue sample. The bound antibody is quantified there by directly indicating the concentration of PIBF in the tissue sample. This method allows localisation of PIBF in a sample.

Preferably the concentration of PIBF is measured indirectly by measuring the concentration of PIBF-mRNA in the sample. For this polynucleotides may be used, including oligonucleotide sequences, antisense RNA and DNA molecules, and PNAs. The polynucleotides may be used to detect and quantitate gene expression in samples in which expression of PIBF will be correlated with a tumor. Accordingly, a kit can be provided comprising a reagent comprising the above mentioned (labelled) polynucleotides in order to carry out PIBF-mRNA measurement in the given sample. Here again it is further preferable to measure the concentration of alternatively processed mRNA. Also, the presence or absence of a specific mRNA molecule can give information with respect to whether or not the cells are tumor cells.

In one aspect, hybridization with nucleotide probes may be used to identify PIBF-mRNA sequences. Nucleotide sequences complementary to the PIBF-mRNA may be labelled by standard methods, and added to a fluid or tissue sample from a patient under conditions suitable for the formation of hybridization complexes. After a suitable incubation period, the sample is washed and the signal is quantitated and compared with the threshold value.

The specificity of the probe, whether it is made from a highly specific region or a less specific region and the stringency of the hybridization (maximal, high, intermediate, or low) will determine whether the probe identifies only naturally occurring sequences encoding PIBF, alleles, or related sequences.

Probes which are used for the hybridization of PIBF-mRNA (related) sequences should preferably show at least 50%, preferably 70%, still preferred 90%, homology to the PIBF encoding sequence or fragments thereof. The hybridization probes of the subject invention may be DNA or RNA and derived from the nucleotide sequence of SEQ ID. NO 3 or 5 (PIBF-cDNA).

Examples of such PIBF-mRNA molecules to be detected and/or quantified are for example those detected by DNA or RNA derived from the nucleotide sequence of SEQ ID NO: 5, 7, 9, 11, 12, 13, 16, 18, 21, 22, 24, 26, 28, 30, 33, 35 and 37. Since it has been shown that exons 17 and 18 are included in almost all mRNA forms which have been identified DNA or RNA derived from a sequence coding exons 17 and 18 (see figs.) are preferably used for the detection or quantification of PIBF-mRNA in a sample.

Hybridization probes may be labelled by a variety of reporter groups, for example, radionucleotides such as 32P or 35S, or enzymatic labels, such as alkaline phosphatase coupled to the probe via avidin/biotin coupling systems, and the like.

The polynucleotide sequences encoding PIBF may further be used in northern blot analysis, dot blot, or other membrane-based technologies; in dip stick, pin, ELISA or (micro-) chip assays utilizing fluids or tissues from patient biopsies to detect PIBF-mRNAs. Such methods are well known in the art.

Additionally, PIBF-mRNA can be detected and measured by RT-PCR: In a first step the mRNA is transcribed by reverse transcriptase into cDNA after which the cDNA is detected and quantified by PCR. The oligomers for the PCR may be chemically synthesized, generated enzymatically, or produced from a recombinant source. Oligomers will preferably consist of two nucleotide sequences, one with sense orientation and another with antisense, employed under optimized conditions for identification of the specific sequence. The same two oligomers, nested sets of oligomers, or even a degenerate pool of oligomers may be employed under less stringent conditions for detection and/or quantitation of closely related sequences.

A further aspect of the present invention relates to a method for determining the positive or negative progression of a tumor in a patient comprising diagnosing a tumor in a patient according to one of the above mentioned methods according to the present invention and determining whether the measured concentration of PIBF or a derivative thereof or a fragment thereof in the sample is above or below at least one previously measured concentration of PIBF or a derivative thereof or a fragment thereof in at least one sample previously taken from the same patient, whereby the concentration above the previously measured concentration identifies a positive progression. Since the concentration of PIBF in a sample is directly proportional to the progression of the tumor, e.g. size, development etc., the method according to the present invention allows direct analysing of the course of the illness. For a complete characterisation of the progression of the tumor it is of course advantageous to take many samples over a period of time in particular before and after a specific treatment, e.g. with a substance or by removing tumor tissue completely or partly, in which case the effectiveness of the specific treatment can be analysed. The term "positive progression" used herein means that the tumor is further developing.

A further aspect of the present invention relates to the use of an anti-PIBF antibody or a fragment thereof in an above described method according to the present invention. As mentioned above the anti-PIBF antibody may be monoclonal, polyclonal, it further may be recombinant, humanized or phage encoded single-chain antibody. If only a fragment of the antibody is used, this fragment comprises the epitope of the anti-PIBF antibody which recognizes the PIBF.

It is preferred to use a monoclonal antibody in order to achieve a most specific and precise result. The monoclonal antibody may be produced as mentioned above, and the examples given above also apply here.

A further aspect of the present invention relates to the use of PIBF or a derivative thereof or a fragment thereof in one of the above mentioned methods according to the present invention. As already mentioned above the fragment may be a fragment of the PIBF or a fragment of the PIBF derivative. Here, the same definitions and preferred embodiments or examples as mentioned above apply.

Preferably the PIBF is recombinant, meaning that also the derivative or the fragment may be recombinant.

A further aspect of the present invention relates to a kit comprising a first reagent comprising at least one anti-PIBF antibody or a fragment thereof and a second reagent comprising PIBF or a derivative thereof or a fragment thereof at a defined concentration. Of course, the anti-PIBF antibody and the PIBF are present in a form which allows their storage, e.g. in dry, lyophilized, frozen or dissolved form. Further, the kit may comprise any further buffers, enzymes, salts etc., which are necessary for the above mentioned method to be carried out.

Preferably the kit comprises a solid phase to which the at least one anti-PIBF antibody or the fragment thereof or the PIBF or the derivative thereof or the fragment thereof is bound. The solid phase may be any solid phase known to the person skilled in the art, e.g. any insoluble material which can provide a substrate upon which to immobilize proteins or peptides, for example in the form of a dry strip. Such substrates may include nylon, amino acids, glass, cellulose and the like. This kit may preferably be used for competitive or a sandwich assay whereby the further reagent comprising either the antibody or the PIBF, depending on which is immobilized to the solid phase, and the sample are added to the solid phase.

Preferably the PIBF present in the above mentioned kit is recombinant meaning of course that also the derivative thereof and the fragment thereof, respectively, are recombinant.

A preferred kit comprises a further reagent comprising a second anti-PIBF antibody or a fragment thereof which binds to an epitope of the PIBF which is distinct to the epitope recognized by the first anti-PIBF antibody or the fragment thereof. This kit is particularly advantageous in order to carry out a sandwich assay.

The above mentioned kit according to the present invention is preferably for diagnosing a tumor in a patient and for determining the progression of a tumor in a patient, respectively. The methods are the same as described above, whereby the reagent comprising the PIBF or a derivative thereof or a fragment thereof at the defined concentration is used either as a positive control as described above or to carry out a competitive assay as described above (whereby it is used in competition to the PIBF present in the sample of the patient) or both.

A further aspect of the present invention concerns the use of an anti-PIBF antibody or a fragment thereof for the preparation of an anti-tumor medicine. The anti-PIBF antibody or the fragment thereof specifically blocks or neutralizes PIBF, thereby specifically abolishing PIBF activity in tumors thus rendering the tumors susceptible to NK (and potentially CD8+ and other T cell mediated lysis). Furthermore, mono and bi-specific antibodies can specifically recognize PIBF on a surface of tumor cells and can be used to deliver toxic substances to the tumorous compartment of the body of the patient. The main strategy of the anti-tumor medicine is the use of the knowledge that tumor cells produce higher concentrations of PIBF. With this information which is the basis of the present invention various strategies can be developed for fighting a tumor in a patient.

According to a preferred embodiment, the antibody is a monoclonal, humanized, and single chain antibody, respectively. The above mentioned deposited antibodies may also be used for this aspect of the present invention.

Preferably the antibody has attached thereto a molecule. In this case the anti-PIBF antibody is used as a targeting or delivery mechanism for bringing a molecule, e.g. a pharmaceutical agent, to cells or tissues which express PIBF. The antibody which is administered to the patient binds to the tumor expressing PIBF and thereby brings the molecule which is toxic for the tumor into direct contact with the tumor. There are various methods and molecules which are used which are known to the person skilled in the art. For example a toxic molecule can be used which will enter the tumorous cells and interfere with for example essential metabolic steps thereby killing the cells. Also, the toxic molecule may induce cell lysis or act as a receptor for other toxic substances or enzymes which will kill the tumorous cells. However, independently of the way the toxic molecule functions, the main point is that the molecule is specifically directed to the tumorous cells by the anti-PIBF antibody and does not interfere with healthy cells.

The molecule may preferably be a toxic substance and a prodrug, respectively, in particular a radionuclide, a toxin and a chemotherapeutic drug, respectively. By delivering the substance to the tumorous target an effective anti-tumor medicine is achieved.

A further aspect of the present invention concerns the use of PIBF or a derivative thereof or a fragment thereof for the preparation of an anti-tumor medicine. There are two strategies for these anti-tumor medicines according to the present invention: - A PIBF-derivative or a fragment thereof is used as inhibitory protein or peptide which interferes with the PIBF action through binding and thereby blocking or inactivating putative receptors for PIBF present on cells, e.g. NK cells, or inhibit signalling components down-stream of receptor binding.

According to a preferred embodiment of a present invention the medicine is a vaccine. The PIBF derivative or the fragment thereof comprises the immunogenic peptide of PIBF and can be used for vaccination either to induce antigen specific anti-tumor cytotoxic T cell responses and/or to stimulate the production of neutralizing antibodies by the immune system of the cancer patient himself which would release the NK cells from suppression by PIBF.

Preferably the vaccine comprises an adjuvant. Such an adjuvant may be but is not limited to for example Freund's mineral gels such as aluminiumhydroxide and surface active substances such as lysolecitin, pluronic polyols, polyanions, polycations (e.g. polyArg), peptides, oil emulsions, keyhole limpet hemocyanin, and dinitrophenol. Among adjuvants preferably used in humans are BCG (bacilli Calmette-Guerin) and Corynebacterium parvum. Preferably the PIBF or the derivative thereof or the fragment thereof is recombinant and a chemically synthesized molecule, respectively.

An advantageous-aspect of the present invention concerns the use of a polynucleotide encoding PIBF or a derivative thereof or a fragment thereof or PIBF-antisense molecule for the preparation of an anti-tumor medicine. In the scope of the present application the term "polynucleotide encoding PIBF" or "nucleotide sequences complementary to PIBF-mRNA" relates to a sequence derived from a sequence preferably selected from the group consisting of SEQ ID No. 3, 5, 7, 9, 11, 12, 13, 16, 18, 21, 22, 24,26,28,30,33,35,37 or fragemnts or derivatives thereof.

Genes encoding PIBF can be turned off by transforming a cell or tissue with expression vectors which express high levels of a polynucleotide or a derivative thereof or a fragment thereof which encodes PIBF. Such constructs may be used to introduce untranslatable sense or antisense sequences into a cell. Even in the absence of integration into the DNA, such vectors may continue to transcribe RNA molecules until they are disabled by endogenous nucleases. Transient expression may last for a month or more with a non-replicating vector and even longer if appropriate replication elements are part of the vector system.

Modifications of gene expression can be obtained by designing antisense molecules, DNA, RNA, or PNA, to the control regions of the gene encoding PIBF, i.e., the promoters, enhancers, and introns. Oligonucleotides derived from the transcription initiation site, e.g., between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or regulatory molecules. The antisense molecules may also be designed to block translation of mRNA by preventing the transcript from binding to ribosomes.

The term "antisense" as used herein refers to nucleotide sequences which are complementary to a specific DNA or RNA sequence. Antisense molecules may be produced by any method including synthesis by ligating the gene(s) of interest in a reverse orientation to a viral promoter which permits the synthesis of a complementary strand. Once introduced into a cell this transcribed strand combines with natural sequences produced by the cell to form duplexes. These duplexes then block either the further transcription or translation.

In one aspect, antisense molecules to the polynucleotide encoding PIBF may be used in situations in which it would be desirable to block the transcription of the mRNA. In particular, cells may be transformed with sequences complementary to polynucleotides encoding PIBF. Thus, antisense molecules may be used to modulate PIBF activity, or to achieve regulation of gene function. Such technology is now well known in the art, and sense or antisense oligomers or larger fragments, can be designed from various locations along the coding or control regions of sequences encoding PIBF.

Expression vectors derived from retroviruses, adenovirus, herpes or vaccinia viruses, or from various bacterial plasmids may be used for delivery of nucleotide sequences to the targeted tumorous organ, tissue or cell population. Methods which are well known to the person skilled in the art can be used to construct recombinant vectors which will express antisense molecules complementary to the polynucleotides of the gene encoding PIBF.

Ribozymes, enzymatic RNA molecules, may also be used to catalyze the specific cleavage of RNA. The mechanism of ribozyme action involves sequence-specific hybridization of the ribozyme molecule to complementary target RNA, followed by endonucleolytic cleavage. Examples which may be used include engineered hammerhead motif ribozyme molecules that can specifically and efficiently catalyze endonucleolytic cleavage of sequences encoding PIBF.

Specific ribozyme cleavage sites within any potential RNA target are initially identified by scanning the target molecule for ribozyme cleavage sites which include the following sequences: GUA, GUU, and GUC. Once identified, short RNA sequences of between 15 and ,20 ribonucleotides corresponding to the region of the target gene containing the cleavage site may be evaluated for secondary structural features which may render the oligonucleotide inoperable. The suitability of candidate targets may also be evaluated by testing accessibility to hybridization with complementary oligonucleotides using ribonuclease protection assays.

Antisense molecules and ribozymes of the invention may be prepared by any method known in the art for the synthesis of nucleic acid molecules. These include techniques for chemically synthesizing oligonucleotides such as solid phase phosphoramidite chemical synthesis. Alternatively, RNA molecules may be generated by in vitro and in vivo transcription of DNA sequences encoding PIBF. Such DNA sequences may be incorporated into a wide variety of vectors with suitable RNA polymerase promoters such as T7 or SP6. Alternatively, these cDNA constructs that synthesize antisense RNA constitutively or inducibly can be introduced into cell lines, cells, or tissues.

RNA molecules may be modified to increase intracellular stability and half-life. Possible modifications include but are not limited to the addition of flanking sequences at the 5' and/or 3' ends of the molecule or the use of phosphorothioate or 2' O-methyl rather than phosphodiesterase linkages within the backbone of the molecule. This concept is inherent in the production of PNAs and can be extended in all of these molecules by the inclusion of non-traditional bases such as inosine, queosine, and wybutosine, as well as acetyl-, methyl-, thio-, and similarly modified forms of adenine, cytidine, guanine, thymine, and uridine which are not as easily recognized by endogenous endonucleases.

Many methods for introducing vectors into cells or tissues are available and equally suitable for use in vivo, in vitro, and ex vivo. For ex vivo therapy, vectors may be introduced into stem cells taken from the patient and clonally propagated for autologous transplant back into that same patient (allogeneic stem cell transplantation). Delivery by transfection and by liposome injections may be achieved using methods which are well known in the art..

Any of the anti-tumor medicines described above may be applied to any suitable subject including, for example, mammals such as dogs, cats, cows, horses, rabbits, monkeys, and most preferably, humans.

A further aspect of the present invention is a method for treating a patient with a tumor said method comprising administering to the patient an effective amount of an anti-PIBF antibody or a fragment thereof.
In two publications (Szekeres-Bartho et al., Am.J.Reprod.Immuno. 24, 105, 1990; Szekeres-Bartho et al., Cell.Immunol.177, 194, 1997) it was demonstrated that addition of neutralizing anti-PIBF antibody interferes with a successful pregnancy outcome in mice. Moreover, PIBF isolated from culture supernatants of progesterone treated murine lymphocytes when injected in vivo, prevented the abortive effect of anti-progesterone drugs. These data suggest that these reagents could act in a similar way in patients with cancer or autoimmune diseases.

Another aspect of the present invention concerns a method for the treatment of a tumor in a patient said method comprising administering an effective amount of PIBF or a derivative thereof or a fragment thereof.

Another preferred aspect of the present invention relates to a method for the treatment of a tumor in a patient said method comprising administering an effective amount of a polynucleotide encoding PIBF or a derivative thereof or a fragment thereof or PIBF antisense molecule.

Another aspect of the present invention refers to a pharmaceutical preparation for the treatment of a tumor in a patient said preparation comprising anti-PIBF antibody or a fragment thereof, PIBF or a derivative thereof or a fragment thereof, and polynucleotide encoded PIBF or a derivative thereof or a fragment thereof or PIBF antisense molecule, respectively. Of course, here again, the same definitions and preferred embodiments as mentioned above apply.

The pharmaceutical preparation may be administered alone or in combination with at least one other agent such as a stabilizing compound which may be administered in any sterile biocompatible pharmaceutical carrier including but not limited to saline, buffered saline, dextrose and water. The pharmaceutical preparations may be administered to a patient alone or in combination with other agents, drugs or hormones. The pharmaceutical preparations utilized for the method for the treatment of a tumor in a patient may be administered by any number of routes including but not limited to oral intravenous, intramuscular, intra-arterial, intramedullary, intrathecal, intraventricular, transdermal, subcutaneous, intraperitonial, intranasal, enteral, topical, sublingual or rectal means.

In addition to the active ingredients these pharmaceutical compositions may comprise suitable pharmaceutically acceptable carriers comprising excipients and auxiliaries which facilitate processing of the active compounds into preparations which can be used pharmaceutically. The carriers enable the pharmaceutical preparations to be formulated as tablets, pills, dragees, capsules, liquids, gels, syrups, slurries, suspensions and the like.

According to another aspect, the present invention also relates to a recombinant protein with a PIBF activity according to SEQ.ID.NO 1 and derivatives thereof. This PIBF protein according to the present invention has full PIBF activity comparable to natural PIBF (comprising the sequence according to SEQ.ID.NO 2) but does not show the exact amino acids 595 to 614 as well as amino acid No. 333 according to the natural PIBF sequence (SEQ.ID.NO 2). The protein sequence of the recombinant PIBF protein according to the present invention (SEQ. ID. NO 1) comprises 757 amino acid residues. The present invention therefore provides for novel recombinant PIBF proteins having the sequence of SEQ.ID.NO 1 or derivatives or homologs thereof. Therefore, the recombinant protein with a PIBF activity according to the present invention comprises
- the amino acid sequence according to SEQ.ID.NO 1 or
- an amino acid sequence with an amino acid identity of at least 98% to the sequence according to SEQ.ID.NO 1 as determined by FAST/A algorithm or
- an amino acid sequence from amino acid identity of at least 95% to the sequence from amino acid residue 580 to 630 of SEQ.ID.NO 1 as determined by FAST/A algorithm and
- a PIBF activity of at least 50% of the natural human PIBF molecule.

The PIBF activity may be defined and quantified as NK or CTL inhibition. NK inhibition is considered when in the presence of PIBF the otherwise efficient effector cells (tested in the absence of PIBF) are paralized, that is either the recognition and binding (conjugation) or killing of the target cells is reduced as a function of PIBF concentration. The activity can be expressed as percentage inhibition/µg PIBF or similar substances compared to no PIBF. This similarly applies to CTL inhibitory activity (Szekeres-Bartho et al., Cell.Immunol.177 (1997), 194-199), Szekeres-Bartho et al., Am.J.Reprod.Immunol. 24, 105, (1990)). Furthermore, the PIBF activity may be defined and quantified as Th2 enhancement which is measured by quantifying Th2 (IL-3, IL-4, IL-6, IL-10) vs. Th1 (IL-12, IFN-g) lymphokines, either at the protein or at the mRNA level, and then taking the ratio of the Th2 vs. Th1 signals. An increase in the Th2 and a concomitant decrease in the Th1 cytokines indicate a Th2 enhancement. It can be expressed as an increase in the percentage of Th2 cytokine positive or a decrease in the percentage of Th1 cytokine positive peripheral blood mononuclear cells (PBMCs)/µg PIBF. It is also appropriate to measure the absolute amounts of these cytokines (according to standard methods from the literature) secreted into the culture supernatant or into body fluids as a function of PIBF concentration. The cytokine mRNAs can be measured by standard quantitative RT-PCR based assays, Szekeres-Bartho et al., AJRI 35 (1996), 348-351, Szekeres-Bartho et al., Am.J.Reprod.Immunol. 23, 26, (1990), Szekeres-Bartho et al., Am.J.Ob.Gyn. 163, 1320, (1990).

Despite the significant differences in amino acid sequence of the PIBF protein according to the present invention compared to the natural human PIBF sequence it is possible to produce a recombinant protein with a sequence as defined above (SEQ. ID.NO 1) which recombinant protein shows particularly high functional similarities to the natural protein.

According to a preferred embodiment of the present invention the recombinant protein comprises an amino acid sequence as given from amino acid residues 300 to 350 in SEQ.ID.NO 1. Amino acid No. 333 in the natural human PIBF protein (SEQ.ID.NO 2) is Cys instead of Arg in the recombinant PIBF protein according to the present invention (SEQ.ID.NO 1). Therefore, the recombinant protein according to the present invention preferably comprises an Arg as amino acid No. 333 according to SEQ.ID.NO 1 and a considerable PIBF activity (>50%). However, it may comprise on either one or both ends further amino acid residues which are identical to, homologue to or different from the amino acid residues in SEQ.ID.NO 1 as long as the recombinant protein shows a PIBF activity of at least 50% of the natural human PIBF molecule.

According to a preferred embodiment of the present invention the recombinant protein comprises an amino acid sequence as given from amino acid residues 580 to 630 in SEQ.ID.NO 1 and a considerable PIBF activity (>50%). This recombinant protein therefore comprises the sequence of the inventive PIBF between amino acid residues 580 to 630 in SEQ.ID.NO 1. It may further comprise on either one or both ends further amino acid residues which are identical to, homologue to or different from the amino acid residues in SEQ.ID.NO 1 as long as the recombinant protein shows a PIBF activity of at least 50% of a natural human PIBF molecule.

According to a further aspect of the present invention a protein with a PIBF activity comprising
- the amino acid sequence according to SEQ.ID.NO 4 or
- an amino acid with an amino acid identity of at least 90%, still preferred at least 95%, most preferred 99%, to the sequence according to SEQ.ID.No 4 as determined by FAST/A algorithm
is provided. This protein has shown to be a 89-kDA protein with a PIBF activity isolated from a mouse. This amino acid sequence is particularly advantageous with respect to aspects of detecting, diagnosing and analyzing tumors, anti-tumor substances, carcinogenic substances in mice but also other laboratory animals. Furthermore, with the help of this inventive protein tests can be carried out on animals, e.g. mice, guinea-pigs, hamsters, rats, with a predisposition for a tumor. Another aspect relates to animals, in particular mice, in which this protein is inhibited or its activity blocked. This may be carried out for example by providing analogues of binding partners of this protein.

A preferred aspect of the present invention relates to a protein comprising an amino acid sequence with an identity of at least 85%,preferably at least 90%, still preferred at least 95% as determined by FAST/A algorithm to a sequence selected from the group consisting of SEQ.ID.NOs 6, 8, 10, 14, 15, 17, 19, 20, 23, 25, 27, 29, 31, 32, 34 and 36 said protein being an alternatively processed PIBF protein. It has been shown that alternatively processed mRNA molecules are present in different tissues and therefore express also alternatively processed proteins. Surprisingly, these alternatively processed proteins are present in tumor tissues at another concentration compared to the healthy tissues. This can be advantageously used for detecting and analyzing tumors in a sample whereby particularly SEQ.ID. NOs 6 and 8 are preferred since these are two smaller PIBF forms found in human primary tumors, SEQ.ID.NO 6 in gastric adenocarcinoma and SE-Q.ID.NO 8 in endometrial adenocarcinoma. Normal tissue counterparts from the same patients did not express detectable levels of these PIBF-mRNA splice variants. It has, however, also been shown that exons 17 and 18 are included in almost all forms identified. Therefore, peptides comprising these exons are particularly advantageous. The term "alternatively spliced PIBF proteins" refers to proteins which are derived from proteins with PIBF activity.

According to a further aspect the present invention provides a nucleic acid molecule encoding the above described recombinant protein with a PIBF activity according to the present invention. Of course, it is further possible for the nucleic acid molecule to comprise an additional sequence encoding at least one second protein other than the PIBF protein thereby providing a nucleic acid sequence encoding a fusion protein comprising at least in one part a peptide with PIBF activity.

With respect to the mouse nucleic acid molecule, e.g. SEQ ID No 5 or a fragment thereof, this is preferably used to produce knock out mice, e.g. mice, in which the expression of the PIBF gene or a fragment thereof is blocked or inhibited. This will for example be carried out by providing an antisense mouse PIBF nucleic acid molecule or fragment thereof which strategy is described above. Another aspect of the present invention therefore relates to knock out mice which show an inhibited or reduced expression of active PIBF protein.

Preferably, a nucleic acid molecule encoding an alternatively processed PIBF protein are provided comprising a nucleic acid sequence with an identity of at least 80%, preferably at least 90%, still preferred at least 95% to a sequence elected from the group comprising SEQ.ID. NOs 7, 9, 11, 12, 13, 16, 18, 21, 22, 24, 26, 28, 30, 33, 35 and 37 or
- a sequence which hybridizes under stringent conditions to one of the above sequences or
- a sequence which is degenerated due to the genetic code of one of the above sequences.
These are nucleic acid sequences which correspond to alternatively spliced mRNA molecules found in various tissues whereby particularly SEQ.ID. NO 7 and SEQ.ID.NO 9 relate to alternatively spliced mRNA molecules which were found only in tumor tissues, however normal tissues did not comprise these mRNA sequences. Therefore, particularly these are advantageous when detecting or analyzing tumors as well as healthy cells and tissues.

According to a further aspect the present invention relates to a nucleic acid vector comprising an inventive nucleic acid sequence.

When the above mentioned vector according to the present invention is introduced into a suitable host mRNA is produced which provides an RNA strand for the translation of a recombinant protein with PIBF activity according to the present invention or an inventive protein.

The regulatory element can be any suitable element known by the skilled person in the art in particular a specific promotor which is chosen in accordance with the specific host into which the vector is to be introduced in order to achieve a maximum production of recombinant protein. The regulatory element can further comprise enhancers which enhance the transcription.

Preferably the nucleic acid vector further comprises a selection marker. The selection marker can be any suitable marker which is well-known by the skilled person in the art in order to select cells or host organisms into which the vector has been introduced. Such selection markers may be for example any gene encoding an antibiotic resistance conferring protein, or a gene encoding a protein necessary for the cell metabolism, whereby the cells or host organisms in which the above mentioned vector is to be introduced show a deficiency for this protein. The selection marker may further be any gene which will change the phenotype of the cell or host organism which has taken up the above mentioned vector, e.g. the colour.

According to a further aspect the present invention relates to a cell comprising the above mentioned vector according to the present application. The vector may be integrated into the genome of the cell or also present as exogenous DNA in the cytoplasma as long as transcription of the complementary nucleic acid molecule is provided. In the scope of the present invention the term "cell" comprises any procaryotic or eucaryotic cell. These cells will be preferably used to produce recombinant proteins with PIBF activity according to the present invention. These produced recombinant proteins can be isolated and purified according to methods well known in the art and used further, e.g. for the production of pharmaceutical preparations comprising recombinant proteins with PIBF activity according to the present invention.

The invention will be described in more detail by the following examples and figures but the invention is of course not limited thereto.
Fig. 1 shows an alignment of recombinant and (natural) mouse PIBF amino acid sequences,
Fig. 2 shows a schematic representation of the exons and introns in the PIBF gene region on chromosome 13,
Fig. 3 shows a northern blot for the detection of PIBF-mRNA in different tissues.
Fig. 4 shows the immunohistochemical analysis of human primary tumors.
Figures 5A - 5D show the influence of anti-PIBF treatment on NK cell target killing of tumor cells.
Figures 6A - 6C show the effect of the recombinant PIBF on IL-10 and IL-12 expression of non-pregnancy lymphocytes.
Fig. 7 shows PIBF levels in urine samples of patients with non-adenocarcinoma tumors and non-solid tumors.
Fig. 8 shows the detection of elevated PIBF levels with the help of monoclonal and polyclonal antibodies.
Fog. 9 shows the normalization of PIBF levels after surgery or chemotherapy.
Fig. 10 shows the different PIBF-mRNAs overexpressed in human primary tumors.
Fig. 11 shows the PIBF binding to human PBMC.
Fig. 12 shows alternatively spliced PIBF-mRNA.

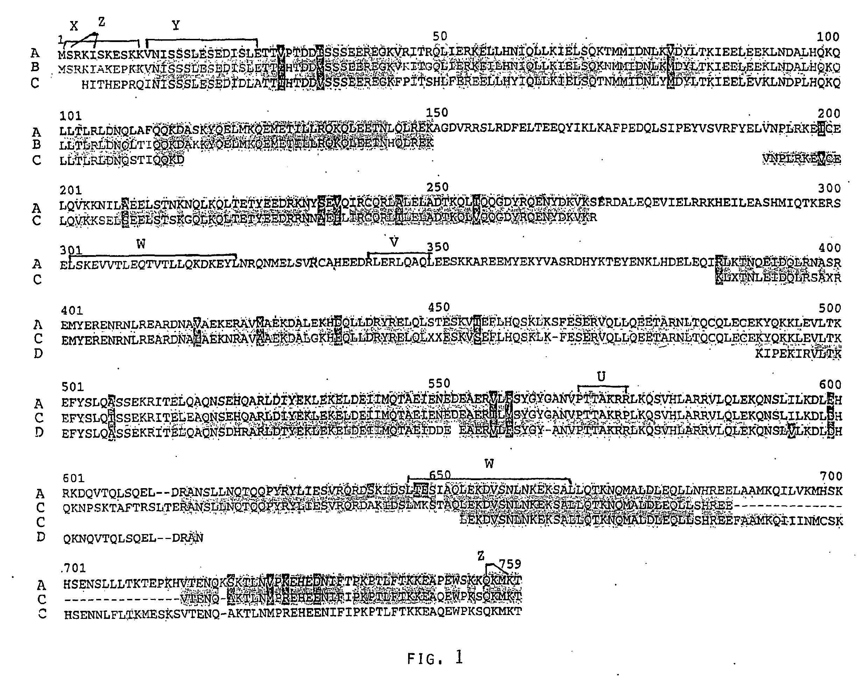

Fig. 1 shows the alignment of recombinant (human) and mouse (natural) PIBF, A being the recombinant sequence, B the IC mouse sequence (cloned from mouse testis library), C being the EST mouse, pieced together from DEST libraries based on the human sequences, and D being the bovine sequence. X represents the signal sequence according to the PSG prediction method, y the signal sequence according to the GvH prediction, z the ER membrane retention signal, w the leucine zipper pattern - DNA binding motif, v the peroxisomal targeting signal, and u the nuclear localisation signal.

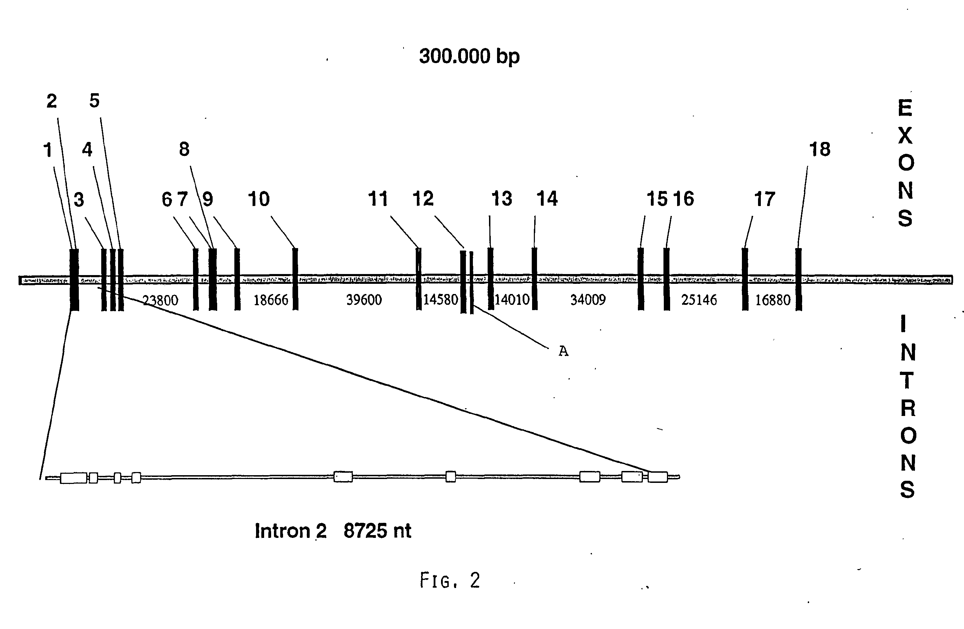

The PIBF gene is located to chromosome 13. A number of introns are present in the PIBF gene (s. Fig. 2), whereby in intron 2 there are multiple copies of the Alu repeat element which serves as a site for alternative splicing. A shows a gap between genomic contigs.

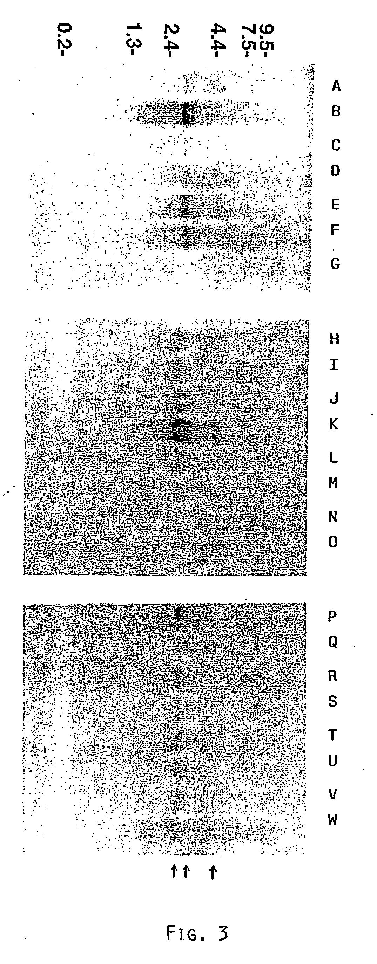

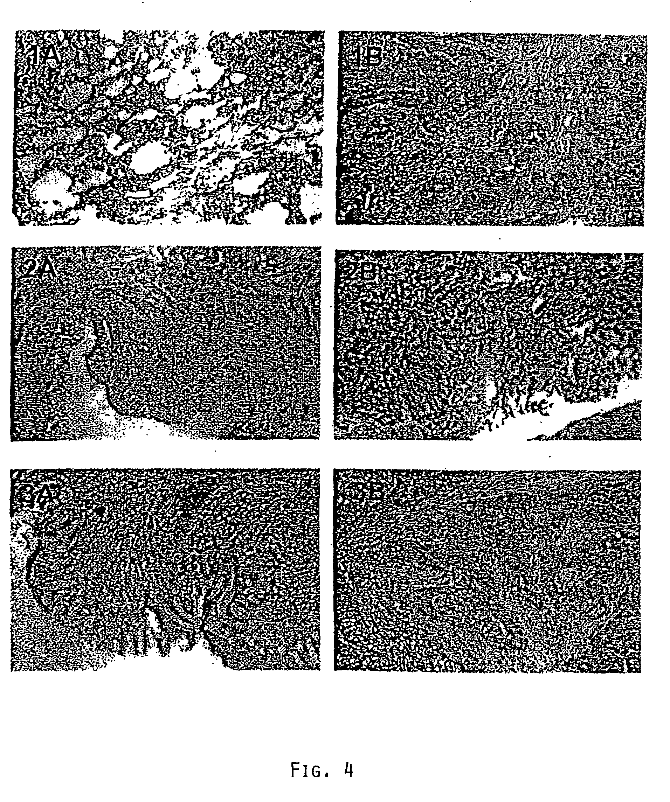

Fig. 3 shows a nothern blot for the detection of PIBF-mRNA in various normal tissues: stomach (A), thyroid gland (B), spinal cord (C), lymph node (D), trachea (E), adrenal gland (F), bone marrow (G), spleen (H), thymus (I), prostate (J), testis (K), uterus (L), small intestine (M), colon (N), PBL (O), heart (P), brain (Q), placenta (R), lung (S), liver (T), skeletal muscle (U), kidney (V), pancreas (W). The arrows in Fig. 4 indicate 3 different mRNA forms.

### Example 1: ESTs entries matching the human PIBF sequence

ESTs entries in human cDNA libraries were searched which match the human PIBF sequence. 43 entries having PIBF sequences were found out of 2.2 million dESTs deposited in 3776 human cDNA libraries. These 43 entries belong to'27 different libraries. 7 of the 27 (25%) libraries originate for normal (non-pregnant, non-tumors adult) tissues. Importantly, testis, which is a immuno-privileged tissue, frequently indicated the presence of PIBF mRNA. 13 of the 27 libraries contain mRNAs expressed in tumorous tissues (-50%). The rest is from foetal or pregnant tissues. This shows that PIBF is preferentially expressed during development, pregnancy and malignancy. However, the number of matching ESTs can correlate with the mRNA abundance but heavily depend on the quality of the library. For that reason one can not take it as a measure of expression level directly (see table I).

**TABLE I**

| | ACC # | Origin of cDNA library | | |
|---|---|---|---|---|
| | | Organ | Embyonic, normal, tumor | Maching parts (nt) |
| 1 | AA099685 | Uterus | Normal, pregnant | 860-1321 |
| 2 | AI188926 | Placenta, pooled (2) | Normal, 8-9 weeks pregn. | 2409-2763(87) |
| 3 | AI200713 | Placenta, pooled (2) | Normal, 8-9 weeks pregn. | 2418-2763(86) |
| 4 | N27300 | Placenta, pooled (2) | Normal, 8-9 weeks pregn. | 2367-2763(84) |
| 5 | N40036 | Placenta, pooled (2) | Normal, 8-9 weeks pregn. | 1657-2089(20) |
| 6 | AA251149 | Tonsilla, germ. center | Normal - enriched B cells | 2440-2763(84) |
| 7 | AA251594 | Tonsilla, germ. center | Normal - enriched B cells | 307-633 |
| 8 | AA806027 | Tonsilla, germ. center | Normal - enriched B cells | 1644-2025 |
| 9 | AA610068 | testis | normal | 2455-2763(86) |
| 10 | AI126269 | testis | normal | 2385-2763(91) |
| 11 | AI758409 | Kidney, bulk tissue Kid11 | Normal | 2491-2763(83) |
| 12 | H64996 | Nose (Olfac epithel) | Normal (female) | (75) 1669-1837(98) |
| 13 | AW793587 | Uterus (exp ORFs) L | adult | (224)1989-2346 |
| 14 | AW818553 | Stomach ORF | adult | 2544-2667(8) |
| 15 | BE165549 | Head-neck | adult | 1881-2264 |
| 16 | AA913693 | Lung-testis-B cell | normal + fetal (lung) | 2501-2763(8) |
| 17 | AA971010 | Lunc-testis-B cell | normal + fetal (lung) | 2531-2763(102) |
| 18 | AI014561 | Luna-testis-B cell | normal + fetal (lung) | (41)1616-2116 |
| 19 | AI222385 | Lung-testis-B cell | normal + fetal:(lung) | 2361-2763(8) |
| 20 | AI809069 | Lung-testis-B cell | normal + fetal (lung) | 1644-2179 |
| 21 | AW085186 | Lung-testis-B cell | normal + fetal (lung) | 2328-2763 (86) |
| 22 | AW269537 | Lung-testis-B cell | normal + fetal (lung) | 2376-2763(83) |
| 23 | AW572968 | Lung-testis-B cell | normal + fetal (lung) | 2515-2616 6 |
| 24 | AI350620 | whole body | Fetus (8-9 weeks) | 2565-2763(87) |
| 25 | D31319 9 | Lung, bulk tissue | fetal | 1394-1765 |
| 26 | AA004593 | Liver + spleen | Fetal (20weeks) | (175)900-1019(54) |
| 27 | AI741044 | 5 pooled libaries* | Fetal, placenta tumor | 2349-2763(95) |
| 28 | AI808795 | 5 pooled libaries* | Fetal, placenta, tumor | 2406-2725(146) |
| 29 | AW978222 | Colon | Tumor, metastasis | 1656-2135 |
| 30 | AI254231 | Colon | adenocarcinoma | (140)2482-2763(101) |
| 31 | AA307364 | Colon, cell line | Carcinoma (HCC) | 2017-2391(55) |
| 32 | AA603710 | Germ cell | Mixed tumors | 2404-2763(84) |
| 33 | AI350870 | Germ cell | Mixed type tumors (3) | 2511-2763(84) |
| 34 | AI990811 | Germ cell (GC_6) | Pooled tumors | 2283-2763(96) |
| 35 | AI278790 | Lung, neuroendocrin | carcinoid | 2514-2763(92) |
| 36 | AI554801 | Uterus, pooled (2) Ut3 | Endometrial carcinoma | 2407-2763(88) |
| 37 | AI915158 | Uterus, pooled (3) (ut2) | Serous papill. cc. high gr | 1714-1837(109) |
| 38 | AW273347 | Uterus (pooled (2) | Serous papill. cc, high gr | 1785-2311(50) |
| 39 | AW 169084 | Uterus, pooled (3) (ut2) | Endom. carcinoma | 2200-2763(84) |
| 40 | AI769755 | Kidney pool of 2 Kid12 | Tumor, clear cell | 2671-2763(403) |
| 41 | AW769371 | Kidney (pool of 2) Kid 13 | Wilms tumors (pr. + meta) | 2310-2763(102) |
| 42 | N59340 | Brain (male) | Mult. Scler. lesion | (102)2506-2763(83) |
| 43 | N77149 | Brain (male) | Mult. Scler. lesion | 1858-2175(12) |

### Example 2: Determination of PIBF concentration in urine samples from cancer patients

A competitive ELISA-based assay to measure PIBF in the urine of cancer patients was established. ELISA plates were coated with recombinant human PIBF at a concentration of 2 µg/ml. Biotin labelled polyclonal anti-PIBF IgG was added together with the samples to be determined for PIBF content. The higher is the PIBF concentration in the sample, the lower is the corresponding ELISA value. Based on these ELISA readings the absolute concentration of the PIBF can be determined.

Urine samples were collected from cancer patients, and used fresh or frozen shortly after collection and stored at -20°C until analysis. It was determined previously that PIBF levels in the serum are significantly higher in healthy pregnant women relative to non-pregnant or pathologic pregnancy levels. To validate the assay on urinary PIBF from cancer patients, urine samples from healthy pregnant women and normal healthy non-pregnant individuals served as positive and negative controls, respectively. The results are summarized in Table II. Normal (healthy, non-pregnant) individuals have low urinary PIBF concentrations (5 ng/ml). Urine from pregnant women was characterized by an average of 110 ng/ml PIBF concentration. Importantly, the high PIBF levels quickly returned to normal following abortion or labor. Analysis of urine samples from 65 tumor patients clearly showed that tumor bearing patients had significantly higher amount of PIBF in their urine, than healthy non-pregnant individuals, ranging from 5 to 180 ng/ml. Patients with advanced cancer (big size primary tumor and/or metastasis) seemed to have higher values, examplified by the data on urine samples from lung tumor patients having an average of 28 vs. 43 ng/ml concentration with or without metastasis, respectively.

This data indicates that PIBF concentration is related to tumor mass, and detection of PIBF in the urine can be used for monitoring of disease progression and relapses. The rise in urine PIBF concentration as a consequence of the presence of PIBF producing tumor is even more pronounced, since not all tumor types are PIBF positive (~70-80% of tumors tested so far).

The most prevalent tumor type among the patients was lung cancer with 23 cases. Most of the lung cancer patients had high PIBF concentration. The lack of high PIBF in the urine could be correlated with clinical disease status, namely after the removal of the primary tumor and in remission PIBF concentrations were significantly lower or even normal.

**TABLE II**

| **Control (a)** | **Pregnant (b)** | **Threatened preterm labour (c)** | **Tumor patients (e)** | |
|---|---|---|---|---|
| n=48 | n=23 | n=19 | n=65 | |
| x=5.5 +/- 1.8 | x=110+/36 | x=6+/-4.7 | x=27.7+/-5.3 | |
| | | | Lung (n=23) | |
| a-b p<0.001 1 | b-c <0.02 | | Without metastasis (n=12) 29.4 | With metastasis (n=11) 43.1 |
| a-c NS | | | | |
| a-d p<0.001 | | | | |
| a-e p<0.001 | | | | |

### Example 3: Detection of PIBF in tumor tissues

Following the demonstration of PIBF expression by MCF-7 (human mammary epithelial carcinoma) cell line, a series of human primary tumors were investigated for the expression of PIBF. It became obvious that PIBF appears in the culture supernatant of MCF-7 cells, suggesting that this protein is expressed and secreted similarly what was found with pregnant or activated lymphocytes in culture. According to proteomic analysis, anti-PIBF antibodies recognize proteins with two different sizes in the cellular lysate by 2D Western analysis. A 34-kDa spot is likely to correspond to the secreted form. Another major, 60-62 kDa doublet is detected, which might be the major cell associated PIBF form.

A variety of formalin-fixed human primary tumors were analyzed ex vivo by immunohistology using polyclonal rabbit anti-serum generated by immunization with human natural PIBF (34-kDa) or recombinant PIBF (89-kDa). The results show that a lot of tumor types tested express PIBF or PIBF related substances, e.g. PIBF molecules of different lenghts, PIBF molecules from differently spliced mRNA, truncated molecules, fusion proteins, etc. (Table III). 15 out of the 27 tumors (55%) showed strong positive staining. The lack of specific immunostaining of the normal tissue counterparts proves that transformed tumor cells differentially express PIBF (see Fig. 4). In Fig. 4 left column, designated "A", the normal tissue is shown, in the right column, designated "B", tumor tissues are shown. In the first row (designated "1") lung cancer (small cell), in the second row (designated "2") urinary bladder carcinoma (transitorial cell), and in row "3" stomach cancer (adenocarcinoma) are shown. These data also substantiate that PIBF positivity is a result of expression by the tumor cells themselves, and not the binding of PIBF from the extracellular fluid (secreted by infiltrating lymphocytes).

**TABLE III**

| **Organ** | **Tissue and Tumor type** | **No of PIBF positive (No of tested)** |
|---|---|---|
| Stomach | Adenocarcinoma | 1 (2) |
| Gall bladder | Adenocarcinoma | 0 (1) |
| Prostate | Adenocarcinoma | 0 (1) |
| Colon | Adenocarcinoma Primaer tu. | 1 (1) |
| Ovarium | Cystadenocarcinoma | 2 (3) |
| Thyroid gland | Carcinoma papillare | 2 (2) |
| Breast | Invasive ductal cc | 1 (1) |
| Breast | Invasive lobular cc | 1 (1) |
| Uterus | Cc. endometrioides | 0 (1) |
| Uterus | stromal carcinoma | 0 (1) |
| Uterus | Leiomyosarcoma | 0 (1) |
| Septum nasi | Leiomyosarcoma | 0 (1) |
| Skin | Melanoma | 0 (2) |
| Skin | Epithelial cc. | 1 (1) |
| Lung | Epithelial cc. | 2 (2) |
| Oesophagus | Adenocarcinoma | 1 (1) |
| Urinary bladder | Transitorial cell cc. | 1 (1) |
| Metastasis (skin) | Kidney clear cell cc. | 0 (1) |
| Metastasis (lymph node) | Squamous epithelium | 1 (1) |
| Metastasis (lymph node) | Adenocc. coli | 1 (2) |

Based on the above mentioned results PIBF production is a quite general phenomenon of the malignant or undifferentiated state, and as a consequence, PIBF can serve as a tumor marker.

### Example 4: Modulating NK activity by the presence of PIBF

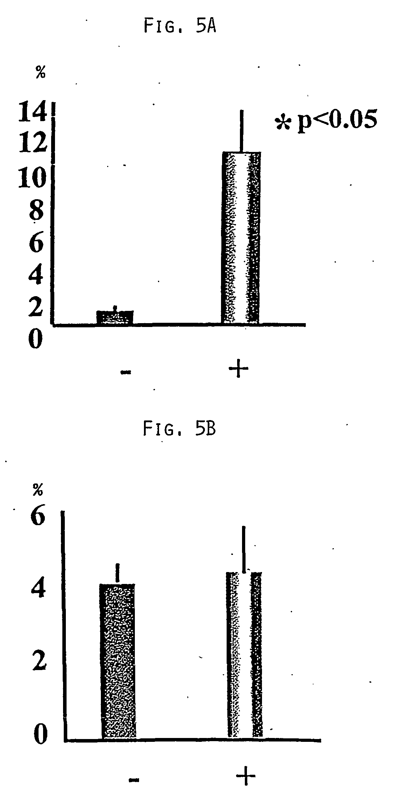

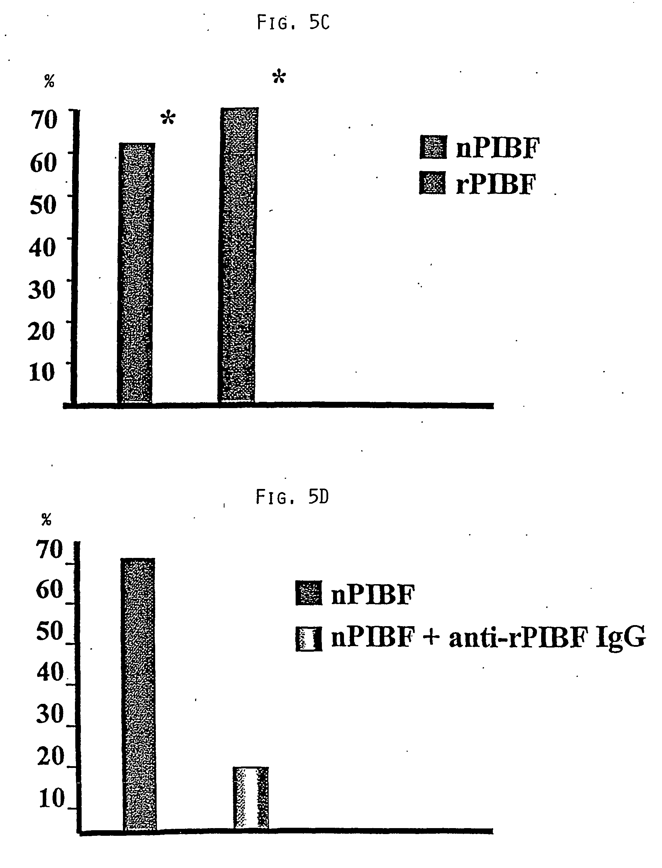

Taken all the data together on the potential importance of PIBF in the suppression of anti-tumor responses,'it is plausible that PIBF produced - secreted or cell surface expressed - by tumor cells will inhibit killer cell activity systemically or locally. It has been long known that there are cell lines which are good targets in NK assays, others are not. The human tumor cell line, MCF-7 belongs to the poor target category. It is certainly a possibility that the low killing activity against these cells is the result of PIBF production, which inhibits NK activity, since the MCF-7 cell line is shown to produce PIBF. To test this possibility, PIBF-expressing MCF-7 cells were used as targets in a 4 hours single cell cytotoxicity assay, according to Grimm and Bonavida "Frequency determination of killer cells by a single-cell cytotoxic assay"; Methods Enzymol 93, 270 (1983). In Fig. 5 it is shown that anti-PIBF treatment enhances NK cell target killing of tumor cells: The minus and the plus indicate the treatment with or without anti-PIBF IgG, the numbers are the percentage of NK activity for Figures 5A and 5B and the percentage of inhibition of NK activity for Figures 5C and 5D. The source of NK cells were freshly isolated PBMCs from healthy individuals. In fact, treatment of these tumor cells with anti-PIBF IgGs increased their sensitivity to NK-mediated lysis dramatically, approximately 8-10fold (Fig. 5A). The basic killing activity against MCF-7 cells is very low (1-2%), whereas high values (50-80%) can be measured when K562 cells are used as target cells in parallel assays. The same anti-PIBF treatment, which seems to be effective in increasing the target activity of tumor cells, however, had no effect on a non-tumor cell line (McCoy, human embryonic fibroblast) (Fig. 5B). By characterizing representative members (good vs. poor target) from both groups, one can make a correlation between expression of PIBF and NK cell activity. Moreover, this assay allows to test the effectiveness of exogenous PIBF to reduce PIBF- target cell killing, and more importantly to evaluate the power of neutralizing anti-PIBF antibodies in stimulating the lysis of PIBF+ tumor cells. Neutralizing rabbit anti-human and also anti-mouse antisera are generated whereby these polyclonal antibodies are able to inactivate natural PIBF in vitro (human lymphocyte cultures in NK assay) or in vivo (pregnant mice, as animal model). By addition of recombinant PIBF to K562 cells (as targets) and PBMCs (as NK cell source), it was possible to reduce the basal killing activity by 60-70% (Fig. 5C). Antibodies generated against the recombinant PIBF removed that inhibition on killing activity almost completely (Fig. 5D).

### Example 5: Modulation of cytokine balance

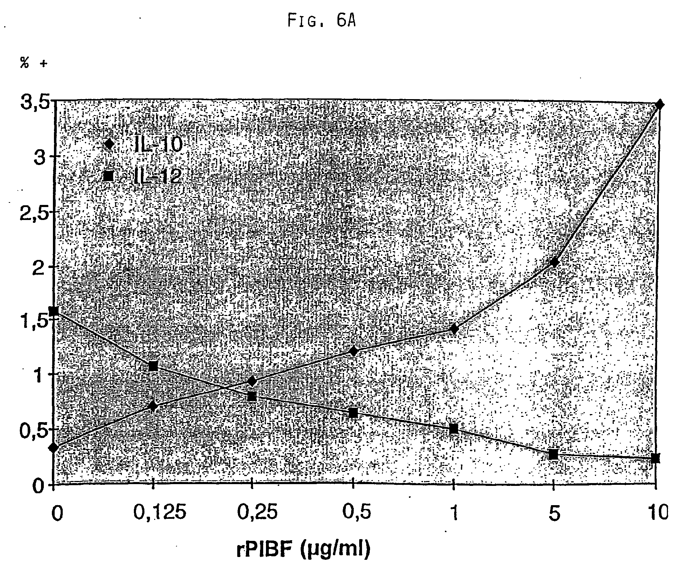

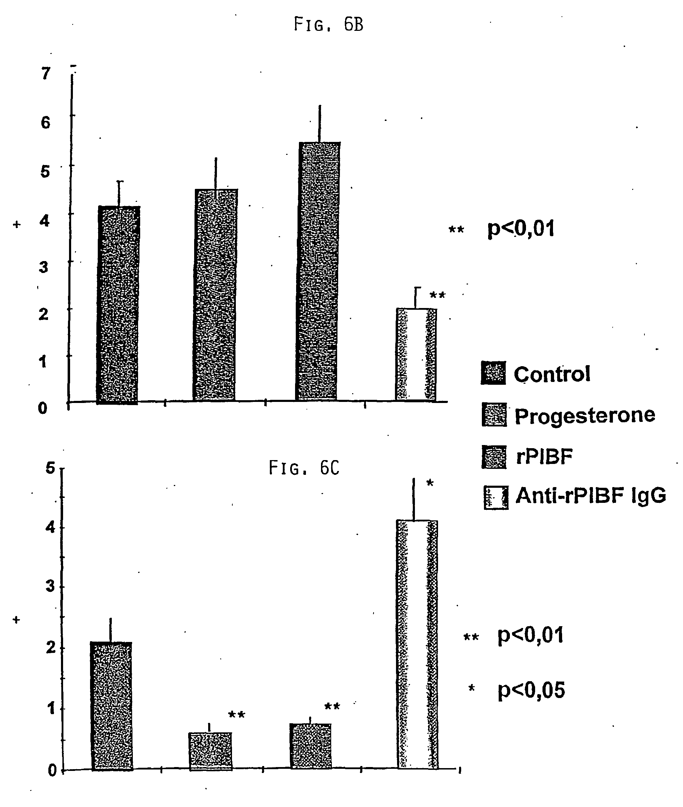

One of the main mechanism of the pregnancy promoting action of PIBF is the induction of the T_{H2} cytokines. There is evidence now that the recombinant form of PIBF is also active to modulate cytokine expression by peripheral blood lymphocytes in vitro. To test the functionality of the recombinant human PIBF which was expressed in E. coli and purified by the GST-tag, rPIBF was added to non-pregnancy peripheral lymphocytes isolated by Ficoll-Paque gradient and cultured at 10⁶/ml cell density. The production of the prototype T_{H2} lymphokine, IL-10, was measured by detecting and counting the number of IL-10 positive lymphocytes (by immunohistochemistry on cytospins) after 24 h treatment. The percent of IL-10 positive lymphocytes increased as the function of rPIBF concentration from 0.35 +/- 0.15 to 3.5 +/- 1.5%. At the highest rPIBF concentration (10 µg/ml) 10x more IL-10 positive lymphocytes were present than in control cultures (Fig. 6A). The opposite effect was seen on IL-12 (T_{H1} lymphokine)-producing lymphocytes by the same treatment. The number of IL-12 positive lymphocytes decreased as the function of PIBF concentration resulting in an approximately 8fold reduction at the highest amount of PIBF. Neutralization of the effect of natural PIBF on cytokine production was also successful. Treatment of pregnancy lymphocytes (producing PIBF) with anti-PIBF IgGs for 3 h resulted in a significant decrease in the number of IL-10 positive and a significant increase in the number of IL-12 positive cells (Figures 6B and C). These results prove that the recombinant form of PIBF is active in inducing T_{H2} cytokine expression. More importantly, neutralizing antibodies can remove active PIBF produced by cells in vivo and consequently enhance TH₁ cytokines.

### Example 6: Diagnostic assay

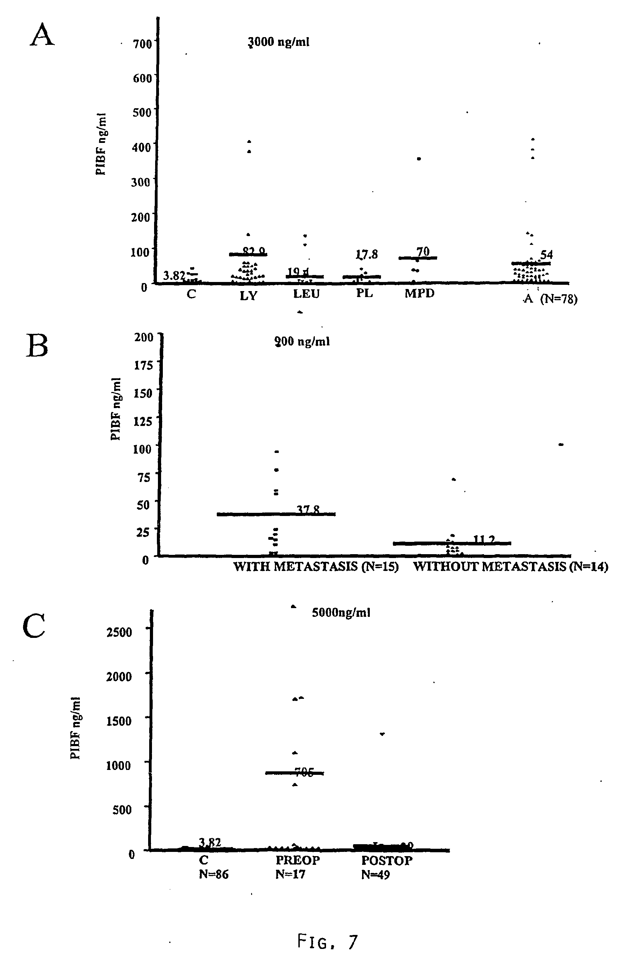

The diagnostic value of urinary PIBF levels in malignancies is further exemplified by using urine samples of patients with non-adenocarcinoma tumors and nonsolid tumors (Fig. 7); dots represent results with individual sera. Average numbers are shown. N means number of patients.

### Examples:

Different hematologic malignancies, especially lymphomas (LY, N:36), and also leukaemias (Leu, N=18), plasmocytomas (PL, N=11) and myeloproliferative diseases (MOP, N=7), are characterized by a higher than normal concentration (control C, N=86) of urinary PIBF (Fig. 7A); A = all tumors). Other examples are head and neck tumors (Fig. 7C) and the malignancies of the urinary tract (Fig.7B) + = with metastasis, N = 15; - = without metastasis, N = 14. It is also obvious that mass of the tumor tissue - that is disease with or without metastasis, removal of tumor - greatly effects the PIBF concentration (Fig. 7B, C).

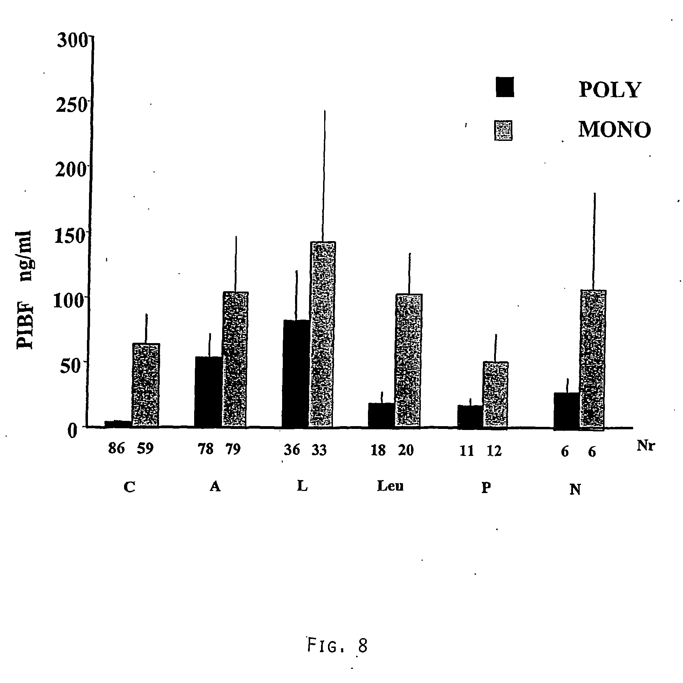

The polyclonal antibodies are replaced in a similar antigen capture sandwich assay with a pair of monoclonal antibodies (ab) produced in mice using the N-terminal 48-kDa rPIBF as antigen. Approximately twenty different hybridoma clones were tested, then four stable and well producing clones selected for ELISA assay, and also for other diagnostic methods (immunohistochemistry). These hybridoma clones are deposited at the Hybridoma Cell Bank at the University Medical School of Pécs. (Deposition numbers are 11-14/2001, cell line codes: HYB255-258). The elevated PIBF levels in urine samples of tumor patients are detected with the monoclonal antibody pairs similarly to the polyclonal antibodies (Fig.8), whereby C = control, A = all haematological tumors, L = lymphoma, Leu = leukemia, P = plasmacytoma, N = non defined; No. = number of patients; POLY = polyclonal ab, MONO = monoclonal ab.

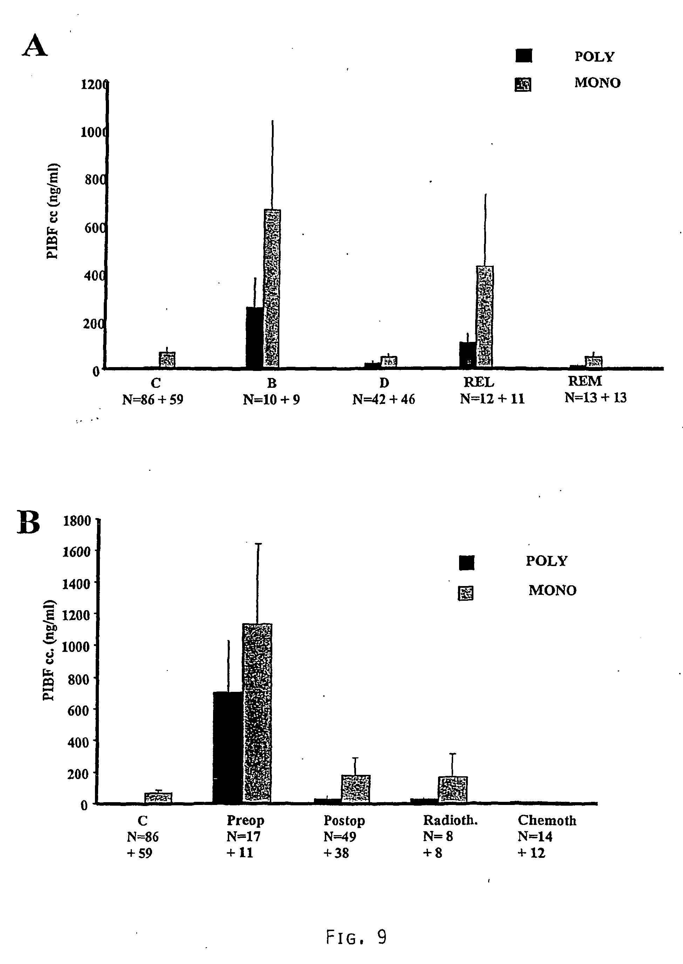

The more excessive data also strengthen not only the diagnostic, but especially the tumor monitoring potential of urinary and serum PIBF ELISA. The test is able to detect and predict therapeutic success and failure, as it is shown by normalization of PIBF levels after surgery or chemotherapy, and a significant rise in relapsing patients (Fig. 9A:haematological, B:urinary track tumors) both with the polyclonal and the monoclonal anti-PIBF antibodies, whereby C = control, B = before treatment, REL = relapse, REM = remission.

### Example 7: Different forms of PIBF

In addtion to the full length human PIBF, the mouse full length PIBF mRNA and protein sequence was identified (SEQ.ID.No 4,5). The mouse PIBF is also organized into 18 exons, and shows an amino acid homology of 89%.

During the attempt to compare PIBF mRNA levels in normal and tumor tissues with RT-PCR, a number of alternatively spliced PIBF mRNA forms were discovered. The structure of the alternatively spliced mRNAs and the corresponding protein products are summerized in Table IV. All the forms identified in one species may occur and can have similar functions in other species. It is examplified by the homologues 35-kDa PIBF protein forms. With the exception of one form of mRNA containing an alternative exon 14' DNA which is intronic sequence in the predominant pre-mRNA, all of them were generated by perfect exon skipping missing several exons relative to the full length. The exons 17 and 18 sequences are included in almost all forms identified. It suggests that the amino acid sequences encoded by these exons are essential for PIBF function. Moreover, several mRNA forms with different sequences result in this same C-terminal PIBF polypeptide with predicted 10-kDa molecular weight. The truncated forms of PIBF identified by RT-PCR analyses of RNA samples were isolated from different human (SEQ.ID.No 6,No 10-20) and mouse tissues and cell lines (SEQ.ID.No8,9,23-37).

The different,PIBF forms are differentially expressed and have different functional attributes.

### Examples:

The full length mRNA (exons 1-18) encodes for a nuclear 89-kDa protein. Bioinformatics predicts a nuclear compartmentalization based on the two nuclear localization signals in exons 7 and 13, and a nucleic acid binding domain in exons 14-16. According to cell fractionation and Western blotting with monoclonal anti-PIBF antibodies, the 89-kDa protein indeed localizes exclusively to the nuclear fractions. Smaller molecular weight forms are present in the cytosolic and secreted fractions of different human and mouse primary tumors, embryo and cell lines.

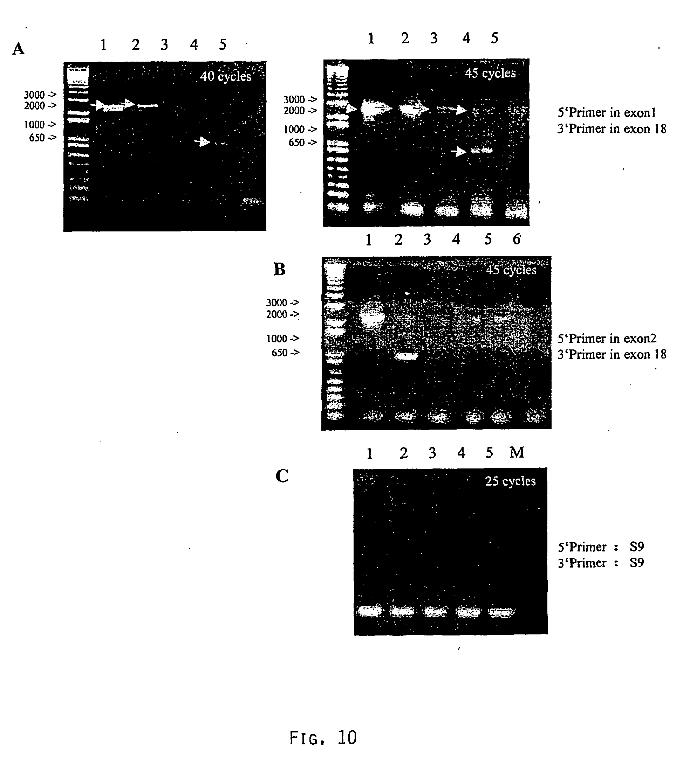

The full length mRNA can be found in almost all tissues based on Northern (Fig. 3) and RT-PCR analysis (Fig. 10), however, in different amounts. Semiquantitative RT-PCR was performed on matched tumor/normal tissue pairs. The same amount of.RNA was amplified with either PIBF specific exon 1/exon 18 (Fig. 10A) and exon 2/exon 18 primer pairs (Fig. 10B) or with ribosomal protein S9 specific primers (for loading control) of the same samples. The samples are: 1 = placenta cDNA, 2 = stomach tumor, 3 = stomach normal, 4 = uterus tumor, 5 = uterus normal, 6 = Neg. Ctrl: w/o template. Fast growing cell, e.g. in tumors and embryo, cell of immune priviledged tissues (testis, placenta) contain more full length PIBF mRNA. RT-PCR analysis of human primary tumors and subsequent cloning and DNA sequencing of PIBF cDNA reveal that the alternatively processed PIBF mRNA forms are expressed differently. It is exemplified by two smaller PIBF forms found in human primary tumors. Expression of the exons (1-5)-(17-18) form in gastric adenocarcinoma and that of the exons 1-(13-18) form in endometrial adenocarcinoma is restricted to the tumor tissues, since the normal tissue counterparts from the same patients do not express detectable levels of these PIBF mRNA splice variants. Importantly, both of these and other alternatively processed PIBF mRNAs are also found in tissues of immune priviledge (placenta, embryo, testis) and in immune cells.

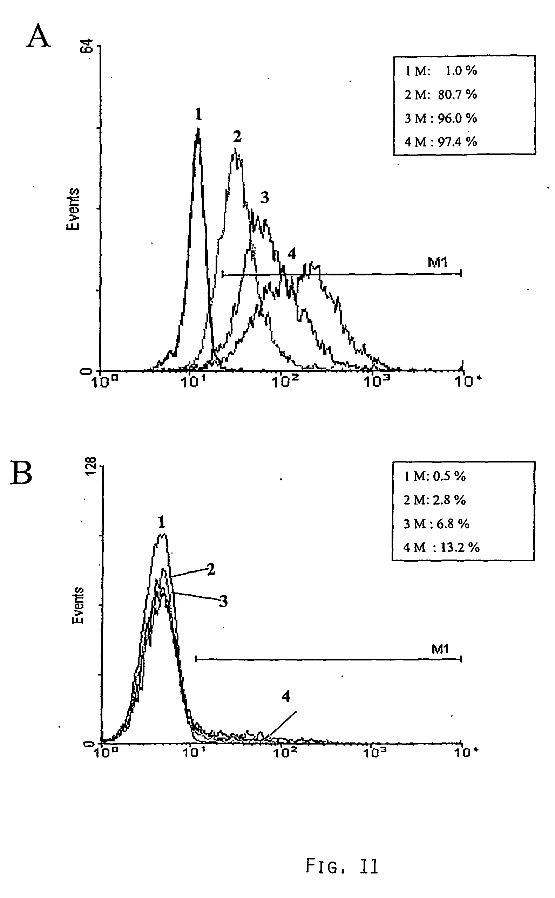

The function of PIBF also depends on the structure of the mature protein from. One of the most interesting forms is encoded by exons 2-3-4-5-17-18 mRNA frequently found in human and mouse tissues, such as human and mouse placenta, human lymphocytes, mouse embryo and human gastric tumor. It encodes for a 298 and 297 amino acid long polypeptide with predicted 35-kDa molecular weight. (SEQ.ID.No 6,7 for human, SEQ.ID.No 8,9 for mouse). The two proteins are 86% homologous. FACS analysis reveals that the human 35-kDa form binds specifically to human immune cells (Fig. 11): FACS staining of human PBMCs with 35-kDa PIBF and α-PibF antibodies (anti-rabbit-FITC). Cells are shown in the monocyte cell region (Fig. 11A) and in the lymphocyte gate (Fig. 11B). M is the number of cells in the increased fluorescence gate expressed in percentage (%), the numbers refer to: 1 = PBMC + anti-exon 17 PIBF, 2 = PMBC + PIBF-35kDa (1 µg) + anti-exon 17, 3 = PBMC + PIBF-35kDa (5 µg) + anti-exon 17, 4 = PBMC + PIBF-35kDa (15 µg) + anti-exon 17. Binding to human lymphocytes and monocytes is suggestive for a PIBF receptor on these immune cells. A truncated version of rPIBF having exon 1-9 (48-kDa), having the N-terminus part of this functional PIBF forms and lacking the C-terminal part (exon 17-18) is not functional in terms of binding to immune cells (data not shown).

**Table IV.**

| **SEQ.ID. Nos** | Name/Definition | Tissues found | Exon structure of mRNA (nt) | Size of protein |
|---|---|---|---|---|
| No 1 | Published human full length PIBF | | Exon1-18 | 758aa |
| | | | | 89-kDa |
| No 2,3 | Alternative human full length PIBF | Every cell (nuclear) | Exon 1-18 | 758aa |
| | | | | 89-kDa |
| No 4,5 | mouse full length PIBF | Every cell (nuclear) | Exon 1-18 | 757aa |
| | | | | 89-kDa |
| No 6,7 | Alternatively spliced | Placenta | Exon (1-5)-(17-18) | 298aa |
| | | Lymphocytes | | 35-kDa |
| | Human 35-kDa | Tumor (gastric adenocc.) | | |
| No 8,9 | Alternatively spliced mouse 35-kDa (homologue of No4) | Embryo placenta | Exon (1-5)-(17-18) | 297aa |
| | | | | 35-kDa |
| No 10,11 12,13 | Alternatively spliced human 10-kDa | Tumor (endomet. adenocc) | Exon 1-(13-18) | 87aa |
| | | | | 10-kDa |
| | | Pregnancy lymphocytes | | |
| | | MCF-7 human mammary adenocc. cell line | Exon.1-(15-18) | |
| | | Leukocytes | Exon 14'-15-18 | |
| No 14 | Alternatively spliced human 14-kDa | Pregnancy lymphocytes | Exon 1-(9-10)-(12-15)-(17-18) | 118aa |
| | | | | 14-kDa |
| | | MCF-7 tumor cell line | | |
| No 15,16 | Alternatively spliced human 8-kDa | Pregnancy lymphocytes | Exon 1-(9-10)-(12-15)-(17-18) | 70aa |
| | | | | 8-kDa |
| | | MCF-7 tumor cell line | | |
| No 17,18 | Alternatively spliced human 20-kDa | MCF-7 tumor cell line | Exon 1-(3-7)-(9-10)-12-(17-18) | 185aa |
| | | | | 20-kDa |
| No 19,21, 22 | Alternatively spliced human 37-kDa | MCF-7 tumor cell line | Exon (1-7)-(9-15)-(17-18) | 308aa |
| | | | | 37-kDa |
| | | | Exon (1-7)-(9-10)-12-(17-18) | |
| No 20 | Alternatively spliced human 31-kDa | MCF-7 tumor cell line | Exon (1-7)-(9-15)-(17-18) | 258aa |
| | | | | 31-kDa |
| No 23,24 | Alternatively spliced mouse 18-kDa | Embryo | Exon (1-2)-(17-18) | 157aa |
| | | Placenta | | 18-kDa |
| No 25, 26 | Alternatively spliced mouse 30-kDa | Placenta | Exon (1-4)-(16-18) | 256aa |
| | | | | 30-kDa |
| No 27, 28 | Alternatively spliced mouse 27-kDa | Adult testis | Exon (1-2)-(15-18) | 229aa |
| | | | | 27-kDa |
| No 29, 30 | Alternatively spliced mouse 61-kDa | Embryo | Exon (1-11)-(17-18) | 512aa |
| | | | | 61-kDa |
| No 31, 33 | Alternatively spliced mouse 68-kDa | Embryo | Exon (1-11)-18 | 569aa |
| | | | | 68-kDa |
| No 32 | Alternatively spliced mouse 10.5-kDa | Embryo | Exon (1-11)-18 | 86aa |
| | | | | 10.5-kDa |
| No 34, 35 | Alternatively spliced mouse 37-kDa | Embryo | Exon 1-(8-14)-(17-18) | 309aa |
| | | | | 37-kDa |
| No 36, 37 | Alternatively spliced mouse 76-kDa | Adult testis | Exon (1-11)-(15-18) | 641aa |
| | | | | 76-kDa |

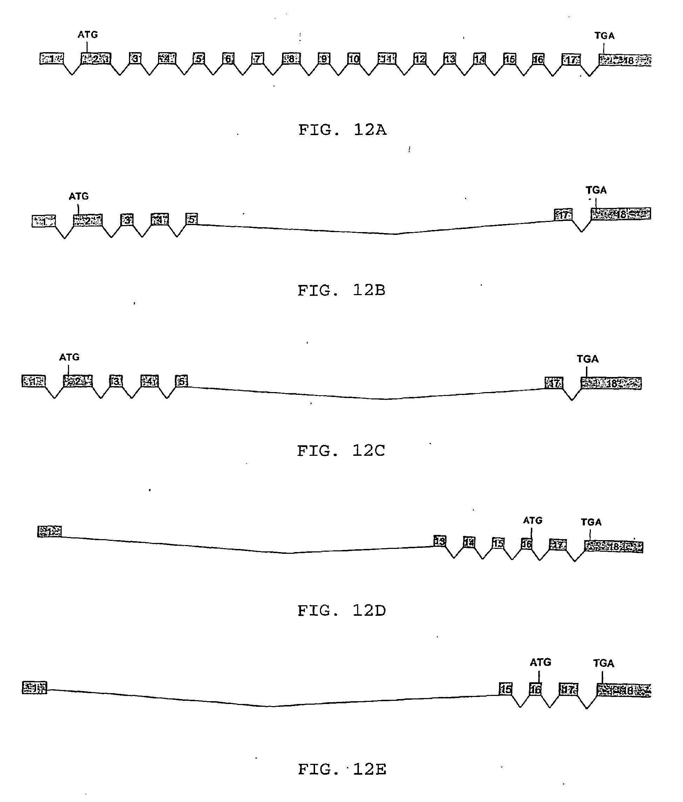

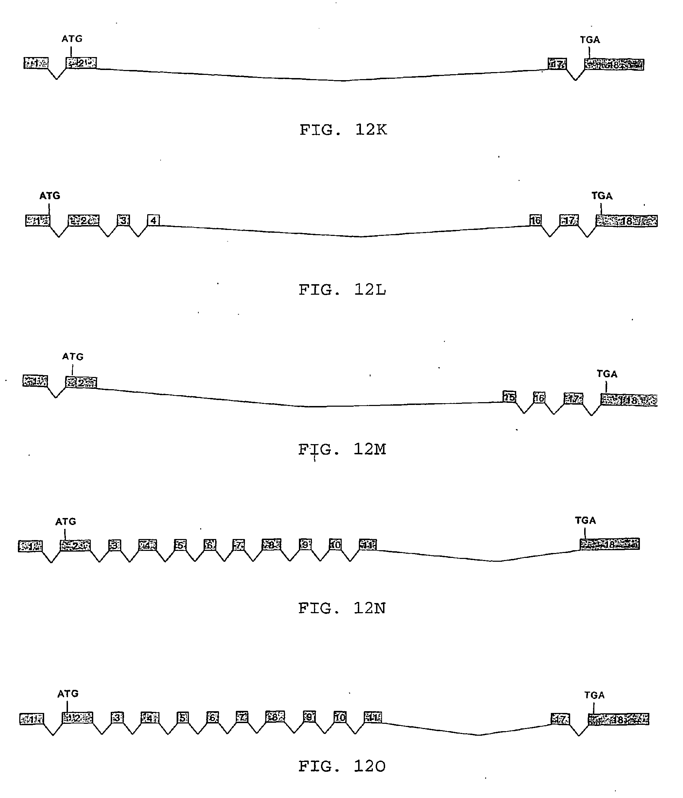

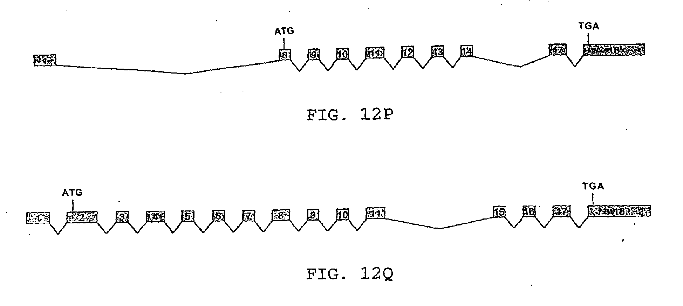

Figs. 12A to 12Q show the alternatively processed PIBF proteins whereby the exons are schematically represented:

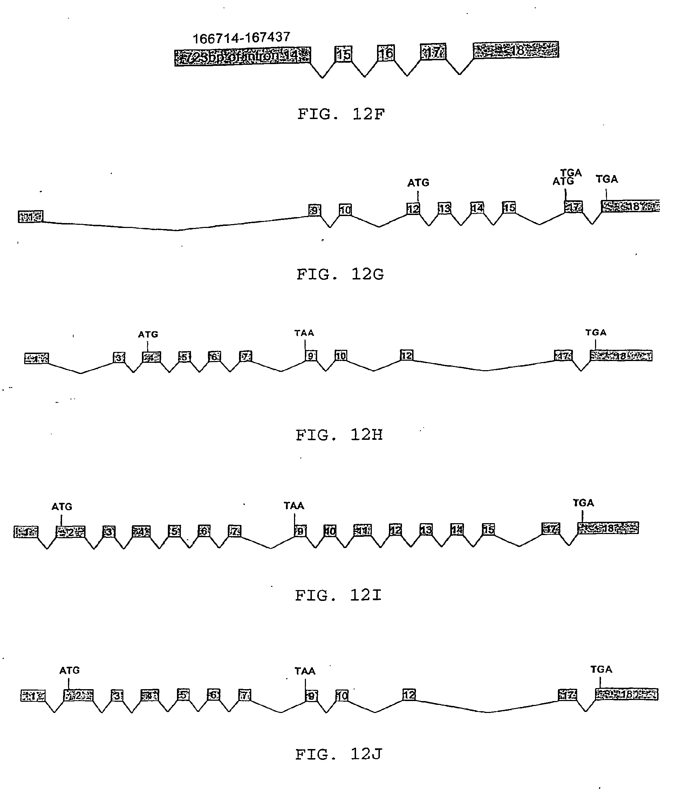

Fig. 12A shows mouse full length 89-kDA PIBF (SEQ ID No. 4), Fig. 12B shows exons (1-5)-(17-18) which is found in stomach tumor, human terminal placenta, male and female lymphocytes, female pregnancy lymphocytes (SEQ ID No. 6), Fig. 12C shows exons (1-5)-(17-18), found in mouse placenta and embryo (SEQ.ID No. 8), Fig. 12D shows exons 1-(13-18) (SEQ. ID No. 11), Fig. 12E shows exons 1-(15-18), found in MCF-7 cells and pregnancy lymphocytes (SEQ.ID No. 12), Fig. 12F shows a part of intron 14 and exons 15-18, found in leukocyte cDNA library (SEQ. ID No. 13), Fig. 12G shows exons 1+(9-10)+(12-15)+(17-18), found in MCF-7 cells and pregnancy lymphocytes (SEQ. ID No. 14), Fig. 12H shows exons 1+(3-7)+(9-10)+12+(17-18), found in MCF-7 cells - human mammary tumor cell line (SEQ. ID No. 17), Fig. 12I shows exon (1-7)+(9-15)+(17-18), found in MCF-7 cells - human mammary tumor cell line (SEQ. ID No. 19), Fig. 12J shows exon (1-7)+(9-10)+12+(17-18), found in MCF-7 cells - human mammary tumor cell line (SEQ. ID No. 22), Fig. 12K shows exon (1-2)-(17-18) found in mouse embryo and placenta (SEQ. ID No. 23), Fig. 12L shows exon (1-4)-(16-18) found in mouse placenta (SEQ. ID No. 25), Fig. 12M shows exons (1-2)-(15-18) found in mouse testis (SEQ. ID No. 27), Fig. 12N shows exon 1-11 18 found in mouse embryo (SEQ. ID No. 29), Fig. 120 shows exons (1-11)-18 found in mouse embryo (SEQ. ID No. 31), Fig. 12P shows exons 1-(8-14)-(17-18) found in mouse embryo (SEQ. ID No. 34) and Fig. 12Q shows exons (1-1)-(15-18) found in mouse testis (SEQ. ID No. 36).

### References

1. Szekeres-Bartho, J., Kinsky, R., Kapovic, M., Vargan P., Chaouat, G. Immuno-endocrine networks in pregnancy. In: Gergely et. al Ed. Progress in Immunology X. Springer Verlag 1993 p.861
2. Szekeres-Bartho, J., Reznikoff-Etievant, M.F., Varga, P., Varga, Z., Chaouat, G. Lymphocytic progesterone receptors in human pregnancy. *J. Reprod Immunol.* 16, 239, 1989.
3. Szekeres-Bartho, J.,Szekeres, Gy., Debre, P., Autran, B., Chaouat, G. Reactivity of Iymphocytes to a progesterone receptor-specific monoclonal antibody. *Cell. Immunol.* 125, 273, 1990.
4. Szekeres-Bartho, J., F. Kilar, G. Falkay, V. Csernus, A. Torok, and A. S. Pacsa. The mechanism of the inhibitory effect of progesterone on lymphocyte cytotoxicity: I. Progesterone-treated lymphocytes release a substance inhibiting cytotoxicity and prostaglandin synthesis. *Am.* J. *Reprod. Immunol. Microbiol. 9, 15,* 1985.
5. Szekeres-Bartho, J., Autran, B., Debre, P., Andreu, G., Denver, L., Chaouat, G. Immunoregulatory effects of a suppressor factor from healthy pregnant women's lymphocytes after progesterone induction. *Cell. Immunol.* 122,281,1989.
6. Szekeres-Bartho, J., Kinsky, R, Chaouat, G. A progesterone-induced immunologic blocking factor corrects high resorption rate in mice treated with antiprogesterone. *Am. J. Ob. Gyn.* 163, 1320, 1 990.
7. Szekeres-Bartho, J., Chaouat, G. Lymphocyte-derived progesterone induced blocking factor corrects resorption in a murine abortion system. *Am. J. Reprod. Immunol.* 23, 26, 1990.
8. Szekeres-Bartho, J., Faust, Zs., Varga, P. Progesterone-induced blocking factor (PIBF) in normal and pathological pregnancy. *Am. J. Reprod. Immunol.* 34, 342, 1995.
9. Check, J., Arwitz, M., Gross, J., Szekeres-Bartho, J., Chung, H. Wu. Evidence that the expression of a progesterone-induced blocking factor by maternal T-lymphocytes is positively correlated with conception. *Am. J. Reprod. Immunol 38,* 6, 1997.
10. Szekeres-Bartho, J., Kinsky, R., Chaouat, G. The effect of a progesterone induced immunologic blocking factor on NK-mediated resorption. *Am. J. Reprod. Immunol. 24,* 105. 1990.
11. Szekeres-Bartho, J., G. Par, Gy. Dombay, Smart, Y. C. Z. Volgyi. The anti-abortive effect of PIBF in mice is manifested by modulating NK activity. *Cell. Immunol.* 177, 194, 1997.
12. Faust, Zs.. G. Laskarin~ D. Rukavina, J. Szekeres-Bartho Progesterone Induced Blocking Factor Inhibits Degranulation ofNK Cells *Am. J. Reprod. Immunol.* 42, 71, 1999.
13. Szekeres-Bartho, J., Wegmann, T.G., A progesterne-dependent immunomodulatory protein alters the Th1/Th2 balanced. *Reprod. Immunol. 31*, **81-95,** 1996.
14. Szekeres-Bartho, J., Wegmann, T.G. Kelemen, K., Bognar, I., Faust, Zs., Varga, P. Interaction of progesterone and cytokine-mediated immunomodulatory mechanisms in favor of successful gestation *Regional lmmunolo∼v,* 6,315, 1995.
15. Szekeres-Bartho, J., Faust, Zs., Varga, P., Szereday, L., Kelemen, K. The immunological pregnancy protective effect of progesterone is manifested via controlling cytokine production. *Am. J. Reprod. Immunol.* 35, 348, 1996.
16. Kelemen, K., Bognar, I., Paal, M., and Szekeres-Bartho, J. A progesterone-induced protein increases the synthesis of asymmetric antibodies. *Cell. Immunol.* 167, 129, 1996.
17. Algarra I, Collado A, Garrido F. Altered MHC class I antigens in tumors. *Int. J. Clin. Lal7. Res.* 27, 95, 1997.
18. Rees RC, Mian S. Selective MHC expression in tumors modulates adaptive and innate antitumor responses. *Cancer Immunol. Immunother.* 48, 374, 1999.
19. Whiteside, T. L., and Herberman, R B. The role of natural killer cells in immune surveillance of cancer. *C717'r. Opin. Immunol.* 7. 704. 1995.
20. Karlhofer FM, Ribaudo RK, and Yokoyama WM. MHC class I alloantigen specificity of Ly-49+ IL-2activated natural killer cells. *Nahlre.358.* pp.66-70. 1992.
21. Phillips JH, Gumperz JE, Parham P. and Lanier LL. Superantigen-dependent, cell-mediated cytotoxicity inhibited by MHC class I receptors on T lymphocytes. *Science* 268. pp.403-405. 1995.
22. Cabestre FA, Lefebvre S. Moreau P. Rouas-Friess N. Dausset J. Carosella ED, Paul P. HLA-G expression: immune privilege for tumour cells?Semin. *Cancer Biol. 9*(1)pp.27-36. 1999.
23. Kagi D, Lederman B: Burki K et al. Cytotoxicity mediated by T cells and natural killer cells is greatly impaired in perforin-deficient mice. *Nature* 369. pp.31-37. 1994.
24. Smyth, M. J. et al. Perforin is a major contributor to NK cell control of tumor metastasis. *J. Immunol.* 162, 6658, 1999.
25. Kollias G. Douni E, Kassiotis G. Kontoyiannis D. On the role of tumor necrosis factor and receptors in models of multiorgan failure, rheumatoid arthritis, multiple sclerosis and inflammatory bowel disease *Imm unol Rev.* 169, 175, 1999
26. Manilay JO, Sykes M. Natural killer cells and their role in graft rejection. *Cu7r Op7n Immunol.* 10, 532, 1998.
27. Young NT. Kir genes, killer cells and clinical transplantation. *Transplantation* 68, 1626, 1999
28. Beaman K, Angkachatchai V, Gilman-Sachs A. TJ6: the pregnancy-associated cytokine. *Am J Reprod Immunol.* 35, 338, 1996.
29. Aslakson CJ, Lee G. Boomer JS, Gilman-Sachs A, Kucuk O. Beaman KD. Expression of regeneration and tolerance factor on B cell chronic lymphocytic leukemias: a possible mechanism for escaping immune surveillance. *Am JHematol* 61, 46, 199..
30. Bonavida B. Bradley TP' Grimm EA. Frequency determination of killer cells by a single-cell cytotoxic assay. *Methods Enzvmol* 93, 270, 1983.

## Claims

1. A method for diagnosing a tumor in a patient said method comprising measuring the concentration of PIBF (Progesterone Induced Blocking Factor) or a derivative thereof or a fragment thereof in a sample taken from the patient and determining whether the concentration of PIBF in the sample is above or below a predetermined threshold value, whereby the concentration above the threshold value identifies a patient with a tumor.

2. The method according to claim 1 **characterized in that** the tumor is an epithelial carcinoma, especially a lung carcinoma, a colon carcinoma or a breast carcinoma.

3. The method according to claim 1 or 2 **characterized in that** the sample is a body fluid, especially urine or serum.

4. The method according to claim 1 or 2 **characterized in that** the sample is a tissue sample.

5. The method according to any one of claims 1 to 4 **characterized in that** the threshold value is the concentration of PIBF in a sample of a healthy person.

6. The method according to any one of claims 1 to 5 **characterized in that** the threshold value is determined by measuring the concentration of PIBF in at least one healthy person parallel to the determination of the concentration of PIBF in a sample of the patient.

7. The method according to any one of claims 1 to 6 **characterized in that** as a positive control the concentration of PIBF or a derivative thereof or a fragment thereof in the sample comprising a defined concentration of PIBF or a derivative thereof or a fragment thereof is measured parallel to the determination of the concentration of PIBF in the sample of the patient.

8. The method according to any one of claims 1 to 7 **characterized in that** the concentration of PIBF in the sample is measured immunologically, in particular by a competitive assay, by a sandwich assay, by immunostaining or combinations of these methods.

9. The method according to any one of claims 1 to 8 **characterized in that** the concentration of PIBF in the sample is measured indirectly by measuring the concentration of PIBF-mRNA in the sample.

10. A method for determining the positive or negative progression of a tumor in a patient comprising diagnosing a tumor in a patient according to a method according to any one of claims 1 to 9 and determining whether the measured concentration of PIBF or a derivative thereof or a fragment thereof in the sample is above or below at least one previously measured concentration of PIBF or a derivative thereof or a fragment thereof in at least one sample previously taken from the same patient, whereby the concentration above the previously measured concentration identifies a positive progression.

11. Use of an anti-PIBF antibody, preferably a monoclonal antibody, or a fragment thereof in a method according to any one of claims 8 to 10.

12. Use of PIBF or a derivative thereof or a fragment thereof in a method according to any one of claims 7 to 10.

13. The use according to claim 12 **characterized in that** the PIBF is a recombinant PIBF.

14. A kit comprising a first reagent comprising at least one anti-PIBF antibody or a fragment thereof and a second reagent comprising PIBF or a derivative thereof or a fragment thereof at a defined concentration.

15. The kit according to claim 14 **characterized in that** it comprises a solid phase to which the at least one anti-PIBF antibody or the fragment thereof or the PIBF or the derivative thereof or the fragment thereof is bound.

16. The kit according to claim 14 or 15 **characterized in that** the PIBF is recombinant.

17. The kit according to any one of claims 14 to 16 **characterized in that** it comprises a further reagent comprising a second anti-PIBF antibody or a fragment thereof which binds to an epitope of the PIBF which is distinct to the epitope recognized by the first anti-PIBF antibody or the fragment thereof.

18. The kit according to any one of claims 14 to 17 for diagnosing a tumor in a patient and for determining the progression of a tumor in a patient, respectively.

19. Use of an anti-PIBF antibody, preferably a monoclonal anti-PIBF antibody, especially humanized anti-PIBF antibody, or a fragment thereof for the preparation of an anti-tumor medicine.

20. The use according to claim 19 **characterized in that** the antibody is a single chain antibody.

21. The use according to claim 19 or 20 **characterized in that** the antibody has attached thereto a molecule.

22. The use according to claim 21 **characterized in that** the molecule is a toxic substance, especially a radionuclide a chemotherapeutic drug or a toxin, and a prodrug, respectively.

23. Use of PIBF or a derivative thereof or a fragment thereof for the preparation of an anti-tumor medicine.

24. The use according to claim 23 **characterized in that** the medicine is a vaccine.

25. The use according to claim 24 **characterized in that** the vaccine further comprises an adjuvant.

26. The use according to claim 23 or 24 **characterized in that** the PIBF or the derivative thereof or the fragment thereof is recombinant.

27. The use according to claim 23 to 24 **characterized in that** the PIBF or the derivative or the fragment thereof is a chemically synthesized molecule.

28. The use of a polynucleotide encoding PIBF or a derivative thereof or a fragment thereof or PIBF-antisense molecule for the preparation of an anti-tumor medicine.

29. Recombinant protein with a Progesterone-Induced Immunomodulatory Protein- (PIBF-) activity, comprising
- the amino acid sequence according to SEQ.ID.NO 1 or
- an amino acid sequence with an amino acid identity of at least 98% to the sequence according to SEQ.ID.NO 1 as determined by FAST/A algorithm or
- an amino acid sequence with an amino acid identity of at least 95% to the sequence from amino acid residue 580 to 630 of SEQ.ID.NO 1 as determined by FAST/A algorithm and
- a PIBF activity of at least 50% of the natural human PIBF molecule.

30. Recombinant protein according to claim 29, **characterized in that** it comprises an amino acid sequence as given from amino acid residues 300 to 350 in SEQ-ID.NO 1.

31. Recombinant protein according to claim 29 or 30, **characterized in that** it comprises an amino acid sequence as given from amino acid residues 580 to 630 in SEQ.ID-NO 1.

32. Protein with a PIBF activity comprising
- the amino acid sequence according to SEQ.ID.NO 4 or
- an amino acid with an amino acid identity of at least 90%, preferably at least 95%, still preferred at least 99%, to the sequence according to SEQ.ID.No 4 as determined by FAST/A algorithm.

33. Protein **characterized in that** it comprises an amino acid sequence with an identity of at least 85%, preferably at least 90%, still preferred at least 95% as determined by FAST/A algorithm to a sequence selected from the group consisting of SEQ.ID.NOs 6, 8, 10, 14, 15, 17, 19, 20, 23, 25, 27, 29, 31, 32, 34 and 36 said protein being an alternatively processed PIBF protein.

34. Nucleic acid molecule encoding a recombinant protein with a PIBF activity according to any one of claims 29 to 32.

35. Nucleic acid molecule encoding an alternatively processed PIBF protein **characterized in that** it comprises a nucleic acid sequence with an identity of at least 80%, preferably at least 90%, still preferred at least 95% to
- a sequence elected from the group consisting of SEQ.ID. NOs 7, 9, 11, 12, 13, 16, 18, 21, 22, 24, 26, 28, 30, 33, 35 and 37 or
- a sequence which hybridizes under stringent conditions to one of the above sequences or
- a sequence which is degenerated due to the genetic code to one of the above sequences.

36. Nucleic acid vector comprising a nucleic acid sequence according to claim 34 or 35 and a suitable regulatory element.

37. Nucleic acid vector according to claim 36, **characterized in that** it further comprises a selection marker.

38. Cell, comprising a vector according to any one of claims 34 to 36.

## Patentansprüche

1. Verfahren zur Diagnostizierung eines Tumors bei einem Patienten, welches Verfahren das Messen der Konzentration von PIBF (Progesteron-induzierter Blockierungs-Faktor) oder eines Derivats davon oder Fragments davon in einer vom Patienten entnommenen Probe und die Bestimmung, ob die PIBF-Konzentration in der Probe über oder unter einem vorbestimmten Schwellenwert liegt, umfasst, wobei die Konzentration über dem Schwellenwert einen Patienten mit einem Tumor identifiziert.

2. Verfahren nach Anspruch 1, **dadurch gekennzeichnet, dass** der Tumor ein Epithel-Carcinom, insbesondere ein Lungen-Carcinom, ein Colon-Carcinom oder ein Brust-Carcinom ist.

3. Verfahren nach Anspruch 1 oder 2, **dadurch gekennzeichnet, dass** die Probe eine Körperflüssigkeit, insbesondere Harn oder Serum ist.

4. Verfahren nach Anspruch 1 oder 2, **dadurch gekennzeichnet, dass** die Probe eine Gewebsprobe ist.

5. Verfahren nach einem der Ansprüche 1 bis.4, **dadurch gekennzeichnet, dass** der Schwellenwert die Konzentration des PIBF in einer Probe einer gesunden Person ist.

6. Verfahren nach einem der Ansprüche 1 bis 5, **dadurch gekennzeichnet, dass** der Schwellenwert durch Messen der PIBF-Konzentration in mindestens einer gesunden Person parallel zur Bestimmung der PIBF-Konzentration in einer Probe des Patienten bestimmt wird.

7. Verfahren nach einem der Ansprüche 1 bis 6, **dadurch gekennzeichnet, dass** als positive Kontrolle die Konzentration von PIBF oder eines Derivats davon oder eines Fragments davon in der Probe, die eine festgelegte Konzentration von PIBF oder eines Derivats davon oder eines Fragments davon aufweist, gemessen wird, parallel zur Bestimmung der Konzentration des PIBF in der Probe des Patienten.

8. Verfahren nach einem der Ansprüche 1 bis 7, **dadurch gekennzeichnet, dass** die PIBF-Konzentration in der Probe immunologisch, insbesondere durch einen konkurrierenden Test, durch einen Sandwich-Test, durch Immunfärbung oder durch Kombinationen dieser Methoden gemessen wird.

9. Verfahren nach einem der Ansprüche 1 bis 8, **dadurch gekennzeichnet, dass** die PIBF-Konzentration in der Probe indirekt, durch Messung der Konzentration der PIBF-mRNA in der Probe, gemessen wird.

10. Verfahren zur Bestimmung der positiven oder negativen Progression eines Tumors bei einem Patienten, welches die Diagnostizierung eines Tumors bei einem Patienten gemäß einem Verfahren nach einem der Ansprüche 1 bis 9 und die Feststellung, ob die gemessene Konzentration des PIBF oder eines Derivats davon oder eines Fragments davon in der Probe über oder unter mindestens einer zuvor gemessenen Konzentration von PIBF oder einem Derivat davon oder einem Fragment davon in mindestens einer zuvor vom selben Patienten genommenen Probe liegt, wobei eine über der zuvor gemessenen Konzentration liegende Konzentration eine positive Progression identifiziert.

11. Verwendung eines anti-PIBF-Antikörpers, vorzugsweise eines monoklonalen Antikörpers oder eines Fragments davon bei einem Verfahren nach einem der Ansprüche 8 bis 10.

12. Verwendung von PIBF oder einem Derivat davon oder einem Fragment davon bei einem Verfahren nach einem der Ansprüche 7 bis 10.

13. Verwendung nach Anspruch 12, **dadurch gekennzeichnet, dass** der PIBF ein rekombinanter PIBF ist.

14. Set, umfassend ein erstes Reagens, das mindestens einen anti-PIBF-Antikörper oder ein Fragment desselben aufweist, und ein zweites Reagens, das PIBF oder ein Derivat davon oder ein Fragment davon mit einer bestimmten Konzentration aufweist.

15. Set nach Anspruch 14, **dadurch gekennzeichnet, dass** es eine feste Phase aufweist, an die der mindestens eine anti-PIBF-Antikörper oder das Fragment davon oder das PIBF oder das Derivat davon oder das Fragment davon gebunden ist.

16. Set nach Anspruch 14 oder 15, **dadurch gekennzeichnet, dass** der PIBF rekombinant ist.

17. Set nach einem der Ansprüche 14 bis 16, **dadurch gekennzeichnet, dass** es mindestens ein weiteres Reagens umfasst, welches einen zweiten anti-PIBF-Antikörper oder ein Fragment davon aufweist, der (das) an ein Epitop des PIBF bindet, welches von dem vom ersten anti-PIBF-Antikörper oder seinem Fragment erkannten Epitop verschieden ist.

18. Set nach einem der Ansprüche 14 bis 17 zur Diagnostizierung eines Tumors bei einem Patienten bzw. zur Feststellung der Progression eines Tumors bei einem Patienten.

19. Verwendung eines anti-PIBF-Antikörpers, vorzugsweise eines monoklonalen anti-PIBF-Antikörpers, insbesondere eines humanisierten anti-PIBF-Antikörpers oder eines Fragments davon zur Herstellung eines anti-Tumor-Medikaments.

20. Verwendung nach Anspruch 19, **dadurch gekennzeichnet, dass** der Antikörper ein Einzelketten-Antikörper ist.

21. Verwendung nach Anspruch 19 oder 20, **dadurch gekennzeichnet, dass** der Antikörper ein an ihm haftendes Molekül aufweist.

22. Verwendung nach Anspruch 21, **dadurch gekennzeichnet, dass** das Molekül eine toxische Substanz ist, insbesondere ein Radionuklid, ein chemotherapeutischer Arzneistoff oder ein Toxin, bzw. ein Prodrug.

23. Verwendung von PIBF oder eines Derivats davon oder eines Fragments davon zur Herstellung eines Anti-Tumor-Medikaments.

24. Verwendung nach Anspruch 23, **dadurch gekennzeichnet, dass** das Medikament ein Vakzin ist.

25. Verwendung nach Anspruch 24, **dadurch gekennzeichnet, dass** das Vakzin weiters ein Adjuvans aufweist.

26. Verwendung nach Anspruch 23 oder 24, **dadurch gekennzeichnet, dass** der PIBF oder das Derivat davon oder das Fragment davon rekombinant ist.

27. Verwendung nach Anspruch 23 bis 24, **dadurch gekennzeichnet, dass** der PIBF oder das Derivat davon oder das Fragment davon ein chemisch synthetisiertes Molekül ist.

28. Verwendung eines Polynukleotids, das für PIBF oder ein Derivat davon oder Fragment davon oder für ein PIBF-antisense-Molekül codiert, zur Herstellung eines Anti-Tumor-Medikaments.

29. Rekombinantes Protein mit einer Progesteron-induzierten, immunmodulierenden Protein(PIBF-)-Aktivität, welches
- die Aminosäuresequenz gemäß SEQ.ID.NO 1 oder
- eine Aminosäuresequenz mit einer Aminosäure-Identität von mindestens 98% mit der Sequenz gemäß SEQ.ID.NO 1, wie mittels FAST/A-Algorithmus bestimmt, oder
- eine Aminosäuresequenz mit einer Aminosäure-Identität von mindestens 95% mit der Sequenz von Aminosäurerest 580 bis 630 von SEQ.ID.NO 1, wie mittels FAST/A-Algorithmus bestimmt, und
- eine PIBF-Aktivität von mindestens 50% des natürlichen Human-PIBF-Moleküls aufweist.

30. Rekombinantes Protein nach Anspruch 29, **dadurch gekennzeichnet, dass** es eine Aminosäuresequenz, wie durch die Aminosäurereste 300 bis 350 in SEQ.ID.NO 1 angegeben, aufweist.

31. Rekombinantes Protein nach Anspruch 29 oder 30, **dadurch gekennzeichnet, dass** es eine Aminosäuresequenz, wie durch die Aminosäurereste 580 bis 630 in SEQ.ID.NO 1 angegeben, aufweist.

32. Protein mit PIBF-Aktivität, welches umfasst:
- die Aminosäuresequenz gemäß SEQ.ID.NO 4 oder
- eine Aminosäure mit einer Aminosäure-Identität von mindestens 90%, vorzugsweise mindestens 95%, noch mehr bevorzugt mindestens 99% mit der Sequenz gemäß SEQ.ID.NO 4, wie mittels FAST/A-Algorithmus bestimmt.

33. Protein, **dadurch gekennzeichnet, dass** es eine Aminosäuesequenz mit einer Identität von mindestens 85%, vorzugsweise mindestens 90%, noch mehr bevorzugt mindestens 95%, wie mittels FAST/A-Algorithmus bestimmt, mit einer Sequenz ausgewählt aus der Gruppe bestehend aus SEQ.ID.NO. 6, 8, 10, 14, 15, 17, 19, 20, 23, 25, 27, 29, 31, 32, 34 und 36 aufweist, wobei dieses Protein ein alternativ prozessiertes PIBF-Protein ist.

34. Nukleinsäure-Molekül, das für ein rekombinantes Protein mit einer PIBF-Aktivität nach einem der Ansprüche 29 bis 32 codiert.

35. Nukleinsäure-Molekül, das für ein alternativ prozessiertes PIBF-Protein codiert, **dadurch gekennzeichnet, dass** es eine Nukleinsäuresequenz aufweist, die eine Identität von mindestens 80%, vorzugsweise mindestens 90%, noch mehr bevorzugt mindestens 95% hat mit
- einer Sequenz gewählt aus der Gruppe bestehend aus SEQ.ID.NO. 7, 9, 11, 12, 13, 16, 18, 21, 22, 24, 26, 28, 30, 33, 35 und 37 oder
- einer Sequenz, die unter stringenten Bedingungen mit einer der obigen Sequenzen hybridisiert, oder
- einer Sequenz, die infolge des genetischen Codes zu einer der obigen Sequenzen degeneriert ist.

36. Nukleinsäure-Vektor, umfassend eine Nukleinsäuresequenz nach Anspruch 34 oder 35 und ein geeignetes Regulierungselement.

37. Nukleinsäure-Vektor nach Anspruch 36, **dadurch gekennzeichnet, dass** er weiters einen Selektions-Marker aufweist.

38. Zelle, umfassend einen Vektor nach einem der Ansprüche 34 bis 36.

## Revendications

1. Procédé de diagnostic d'une tumeur chez un patient, ledit procédé comprenant: mesurer la concentration de PIBF (facteur bloquant induit par la progestérone) ou d'un dérivé de celui-ci ou encore d'un fragment de celui-ci dans un échantillon prélevé à partir dudit patient, et déterminer si la concentration de PIBF dans l'échantillon est supérieure ou inférieure à une valeur seuil prédéterminée, une concentration supérieure à la valeur seuil identifiant un patient avec une tumeur.

2. Procédé selon la revendication 1 **caractérisé en ce que** la tumeur est un carcinome épithélial, en particulier un carcinome pulmonaire, un carcinome du côlon ou un carcinome du sein.

3. Procédé selon la revendication 1 ou 2 **caractérisé en ce que** l'échantillon est un liquide biologique, en particulier de l'urine ou du sérum.

4. Procédé selon la revendication 1 ou 2 **caractérisé en ce que** l'échantillon est un échantillon tissulaire.

5. Procédé selon l'une quelconque des revendications 1 à 4 **caractérisé en ce que** la valeur seuil correspond à la concentration de PIBF dans un échantillon d'une personne saine.

6. Procédé selon l'une quelconque des revendications 1 à 5 **caractérisé en ce que** la valeur seuil est déterminée en mesurant la concentration de PIBF chez au moins une personne saine parallèlement à la détermination de la concentration de PIBF dans un échantillon du patient.

7. Procédé selon l'une quelconque des revendications 1 à 6 **caractérisé en ce que**, comme contrôle positif, la concentration de PIBF ou d'un dérivé de celui-ci ou encore d'un fragment de celui-ci dans l'échantillon comprenant une concentration définie de PIBF ou d'un dérivé de celui-ci ou encore d'un fragment de celui-ci est mesurée parallèlement à la détermination de la concentration de PIBF dans l'échantillon du patient.

8. Procédé selon l'une quelconque des revendications 1 à 7 **caractérisé en ce que** la concentration de PIBF dans l'échantillon est mesurée par voie immunologique, en particulier par un dosage par compétition, par un dosage en sandwich, par immunocoloration, ou par des combinaisons de ces procédés.

9. Procédé selon l'une quelconque des revendications 1 à 8 **caractérisé en ce que** la concentration de PIBF dans l'échantillon est mesurée indirectement en mesurant la concentration d'ARNm de PIBF dans l'échantillon.

10. Procédé visant à déterminer l'évolution positive ou négative d'une tumeur chez un patient comprenant : diagnostiquer une tumeur chez un patient par un procédé selon l'une quelconque des revendications 1 à 9 et déterminer si la concentration de PIBF mesurée ou d'un dérivé de celui-ci ou encore d'un fragment de celui-ci dans l'échantillon est supérieure ou inférieure à au moins une concentration de PIBF préalablement mesurée ou d'un dérivé de celui-ci ou encore d'un fragment de celui-ci dans au moins un échantillon préalablement prélevé à partir du même patient, une concentration supérieure à la concentration préalablement mesurée identifiant une évolution positive.

11. Utilisation d'un anticorps anti-PIBF, de préférence un anticorps monoclonal, ou un fragment de ceux-ci, dans un procédé selon l'une quelconque des revendications 8 à 10.

12. Utilisation de PIBF ou d'un dérivé de celui-ci ou encore d'un fragment de celui-ci dans un procédé selon l'une quelconque des revendications 7 à 10.

13. Utilisation selon la revendication 12 **caractérisée en ce que** le PIBF est un PIBF recombinant.

14. Kit comprenant un premier réactif comprenant au moins un anticorps anti-PIBF ou un fragment de celui-ci et un deuxième réactif comprenant du PIBF ou un dérivé de celui-ci ou un fragment de celui-ci à une concentration définie.

15. Kit selon la revendication 14 **caractérisé en ce qu'**il comprend une phase solide à laquelle le au moins un anticorps anti-PIBF ou le fragment de celui-ci ou le PIBF ou le dérivé de celui-ci ou le fragment de celui-ci est lié.

16. Kit selon la revendication 14 ou 15 **caractérisé en ce que** le PIBF est recombinant.

17. Kit selon l'une quelconque des revendications 14 à 16 **caractérisé en ce qu'**il comprend un autre réactif comprenant un deuxième anticorps anti-PIBF ou un fragment de celui-ci qui se lie à un épitope du PIBF qui est distinct de l'épitope reconnu par le premier anticorps anti-PIBF ou le fragment de celui-ci.

18. Kit selon l'une quelconque des revendications 14 à 17 pour diagnostiquer une tumeur chez un patient et pour déterminer l'évolution d'une tumeur chez un patient, respectivement.

19. Utilisation d'un anticorps anti-PIBF, de préférence un anticorps anti-PIBF monoclonal, en particulier un anticorps anti-PIBF humanisé, ou un fragment de ceux-ci, pour la préparation d'un médicament antitumoral.

20. Utilisation selon la revendication 19 **caractérisée en ce que** l'anticorps est un anticorps monocaténaire.

21. Utilisation selon la revendication 19 ou 20 **caractérisée en ce que** l'anticorps comporte, attachée à celui-ci, une molécule.

22. Utilisation selon la revendication 21 **caractérisée en ce que** la molécule est une substance toxique, en particulier un radionucléide, un médicament chimiothérapeutique ou une toxine, et un promédicament, respectivement.

23. Utilisation de PIBF ou d'un dérivé de celui-ci ou encore d'un fragment de celui-ci pour la préparation d'un médicament antitumoral.

24. Utilisation selon la revendication 23 **caractérisée en ce que** le médicament est un vaccin.

25. Utilisation selon la revendication 24 **caractérisée en ce que** le vaccin comprend en outre un adjuvant.

26. Utilisation selon la revendication 23 ou 24 **caractérisée en ce que** le PIBF ou le dérivé de celui-ci ou encore le fragment de celui-ci est recombinant.

27. Utilisation selon la revendication 23 ou 24 **caractérisée en ce que** le PIBF ou le dérivé ou fragment de celui-ci est une molécule synthétisée chimiquement.

28. Utilisation d'un polynucléotide codant pour le PIBF ou un dérivé de celui-ci ou encore un fragment de celui-ci ou d'une molécule antisens de PIBF pour la préparation d'un médicament antitumoral.

29. Protéine recombinante avec une activité de protéine immunomodulatrice induite par la progestérone (PIBF), comprenant
- la séquence d'acides aminés selon SEQ ID N° : 1, ou
- une séquence d'acides aminés avec une identité d'acides aminés d'au moins 98 % à la séquence selon SEQ ID N°: 1, telle que déterminée par l'algorithme FAST/A, ou
- une séquence d'acides aminés avec une identité d'acides aminés d'au moins 95 % à la séquence des résidus d'acides aminés 580 à 630 de SEQ ID N° : 1, telle que déterminée par l'algorithme FAST/A, et
- une activité PIBF d'au moins 50 % de la molécule de PIBF humaine naturelle.

30. Protéine recombinante selon la revendication 29, **caractérisée en ce qu'**elle comprend une séquence d'acides aminés telle que définie, des résidus d'acides aminés 300 à 350 de SEQ ID N° : 1.

31. Protéine recombinante selon la revendication 29 ou 30, **caractérisée en ce qu'**elle comprend une séquence d'acides aminés telle que définie, des résidus d'acides aminés 580 à 630 de SEQ ID N°: 1.

32. Protéine avec une activité PIBF comprenant
- la séquence d'acides aminés selon SEQ ID N° _{:} 4, ou
- une séquence d'acides aminés avec une identité d'acides aminés d'au moins 90 %, de préférence au moins 95 %, de préférence encore au moins 99 %, à la séquence selon SEQ ID N° : 4, telle que déterminée par l'algorithme FAST/A.

33. Protéine **caractérisée en ce qu'**elle comprend une séquence d'acides aminés avec une identité d'au moins 85 %, de préférence au moins 90 %, de préférence encore au moins 95 %, telle que déterminée par l'algorithme FAST/A, à une séquence choisie dans le groupe constitué par SEQ ID N° : 6, 8, 10, 14, 15, 17, 19, 20, 23, 25, 27, 29, 31, 32, 34 et 36, ladite protéine étant une protéine PIBF résultant d'une maturation alternative.

34. Molécule d'acide nucléique codant pour une protéine recombinante avec une activité PIBF selon l'une quelconque des revendications 29 à 32.

35. Molécule d'acide nucléique codant pour une protéine PIBF résultant d'une maturation alternative, **caractérisée en ce qu'**elle comprend une séquence d'acide nucléique avec une identité d'au moins 80 %, de préférence au moins 90 %, de préférence encore au moins 95 %, à
- une séquence choisie dans le groupe constitué par SEQ ID N° : 7, 9, 11, 12, 13, 16, 18, 21, 22, 24, 26, 28, 30, 33, 35 et 37, ou
- une séquence qui hybride dans des conditions stringentes avec l'une des séquences ci-dessus, ou
- une séquence qui est dégénérée à cause du code génétique de l'une des séquences ci-dessus.

36. Vecteur d'acide nucléique comprenant une séquence d'acide nucléique selon la revendication 34 ou 35 et un élément régulateur approprié.

37. Vecteur d'acide nucléique selon la revendication 36, **caractérisé en ce qu'**il comprend en outre un marqueur de sélection.

38. Cellule comprenant un vecteur selon l'une quelconque des revendications 34 à 36.


### SEQUENCE LISTING

<110> Cistem Biotechnologies GmbH
<120> PIBF
<130> R 38719
<140>
   <141>
<160> 37
<170> PatentIn Ver. 2.1
<210> 1
   <211> 757
   <212> PRT
   <213> Artificial Sequence
<220>
   <223> Description of Artificial Sequence:recombinant protein
<400> 1
   Met Ser Arg Lys Ile Ser Lys Glu Ser Lys Lys Val Asn Ile Ser Ser
    1               5                  10                  15
   Ser Leu Glu Ser Glu Asp Ile Ser Leu Glu Thr Thr Val Pro Thr Asp
               20                  25                  30
   Asp Ile Ser Ser Ser Glu Glu Arg Glu Gly Lys Val Arg Ile Thr Arg
           35                  40                  45
   Gln Leu Ile Glu Arg Lys Glu Leu Leu His Asn Ile Gln Leu Leu Lys
       50                  55                  60
   Ile Glu Leu Ser Gln Lys Thr Met Met Ile Asp Asn Leu Lys Val Asp
   65                  70                  75                  80
   Tyr Leu Thr Lys Ile Glu Glu Leu Glu Glu Lys Leu Asn Asp Ala Leu
                   85                  90                  95
   His Gln Lys Gln Leu Leu Thr Leu Arg Leu Asp Asn Gln Leu Ala Phe
               100                 105                 110
   Gln Gln Lys Asp Ala Ser Lys Tyr Gln Glu Leu Met Lys Gln Glu Met
           115                 120                 125
   Glu Thr Ile Leu Leu Arg Gln Lys Gln Leu Glu Glu Thr Asn Leu Gln
   130 135 140
   Leu Arg Glu Lys Ala Gly Asp Val Arg Arg Ser Leu Arg Asp Phe Glu
   145 150 155 160
   Leu Thr Glu Glu Gln Tyr Ile Lys Leu Lys Ala Phe Pro Glu Asp Gln
   165 170 175
   Leu Ser Ile Pro Glu Tyr Val Ser Val Arg Phe Tyr Glu Leu Val Asn
   180 185 190
   Pro Leu Arg Lys Glu Ile Cys Glu Leu Gln Val Lys Lys Asn Ile Leu
   195 200 205
   Ala Glu Glu Leu Ser Thr Asn Lys Asn Gln Leu Lys Gln Leu Thr Glu
   210 215 220
   Thr Tyr Glu Glu Asp Arg Lys Asn Tyr Ser Glu Val Gln Ile Arg Cys
   225 230 235 240
   Gln Arg Leu Ala Leu Glu Leu Ala Asp Thr Lys Gln Leu Ile Gln Gln
   245 250 255
   Gly Asp Tyr Arg Gln Glu Asn Tyr Asp Lys Val Lys Ser Glu Arg Asp
   260 265 270
   Ala Leu Glu Gln Glu Val Ile Glu Leu Arg Arg Lys His Glu Ile Leu
   275 280 285
   Glu Ala Ser His Met Ile Gln Thr Lys Glu Arg Ser Glu Leu Ser Lys
   290 295 300
   Glu Val Val Thr Leu Glu Gln Thr Val Thr Leu Leu Gln Lys Asp Lys
   305 310 315 320
   Glu Tyr Leu Asn Arg Gln Asn Met Glu Leu Ser Val Arg Cys Ala His
   325 330 335
   Glu Glu Asp Arg Leu Glu Arg Leu Gln Ala Gln Leu Glu Glu Ser Lys
   340 345 350
   Lys Ala Arg Glu Glu Met Tyr Glu Lys Tyr Val Ala Ser Arg Asp His
   355 360 365
   Tyr Lys Thr Glu Tyr Glu Asn Lys Leu His Asp Glu Leu Glu Gln Ile
   370 375 380
   Arg Leu Lys Thr Asn Gln Glu Ile Asp Gln Leu Arg Asn Ala Ser Arg
   385 390 395 400
   Glu Met Tyr Glu Arg Glu Asn Arg Asn Leu Arg Glu Ala Arg Asp Asn
   405 410 415
   Ala Val Ala Glu Lys Glu Arg Ala Val Met Ala Glu Lys Asp Ala Leu
   420 425 430
   Glu Lys His Asp Gln Leu Leu Asp Arg Tyr Arg Glu Leu Gln Leu Ser
   435 440 445
   Thr Glu Ser Lys Val Thr Glu Phe Leu His Gln Ser Lys Leu Lys Ser
   450 455 460
   Phe Glu Ser Glu Arg Val Gln Leu Leu Gln Glu Glu Thr Ala Arg Asn
   465 470 475 480
   Leu Thr Gln Cys Gln Leu Glu Cys Glu Lys Tyr Gln Lys Lys Leu Glu
   485 490 495
   Val Leu Thr Lys Glu Phe Tyr Ser Leu Gln Ala Ser Ser Glu Lys Arg
   500 505 510
   Ile Thr Glu Leu Gln Ala Gln Asn Ser Glu His Gln Ala Arg Leu Asp
   515 520 525
   Ile Tyr Glu Lys Leu Glu Lys Glu Leu Asp Glu Ile Ile Met Gln Thr
   530 535 540
   Ala Glu Ile Glu Asn Glu Asp Glu Ala Glu Arg Val Leu Phe Ser Tyr
   545 550 555 560
   Gly Tyr Gly Ala Asn Val Pro Thr Thr Ala Lys Arg Arg Leu Lys Gln
   565 570 575
   Ser Val His Leu Ala Arg Arg Val Leu Gln Leu Glu Lys Gln Asn Ser
   580 585 590
   Leu Ile Leu Lys Asp Leu Glu His Arg Lys Asp Gln Val Thr Gln Leu
   595 600 605
   Ser Gln Glu Leu Asp Arg Ala Asn Ser Leu Leu Asn Gln Thr Gln Gln
   610 615 620
   Pro Tyr Arg Tyr Leu Ile Glu Ser Val Arg Gln Arg Asp Ser Lys Ile
   625 630 635 640
   Asp Ser Leu Thr Glu Ser Ile Ala Gln Leu Glu Lys Asp Val Ser Asn
   645 650 655
   Leu Asn Lys Glu Lys Ser Ala Leu Leu Gln Thr Lys Asn Gln Met Ala
   660 665 670
   Leu Asp Leu Glu Gln Leu Leu Asn His Arg Glu Glu Leu Ala Ala Met
   675 680 685
   Lys Gln Ile Leu Val Lys Met His Ser Lys His Ser Glu Asn Ser Leu
   690 695 700
   Leu Leu Thr Lys Thr Glu Pro Lys His Val Thr Glu Asn Gln Lys Ser
   705 710 715 720
   Lys Thr Leu Asn Val Pro Lys Glu His Glu Asp Asn Ile Phe Thr Pro
   725 730 735
   Lys Pro Thr Leu Phe Thr Lys Lys Glu Ala Pro Glu Trp Ser Lys Lys
   740 745 750
   Gln Lys Met Lys Thr
   755
<210> 2
   <211> 758
   <212> PRT
   <213> Homo sapiens
<400> 2
   Met Ser Arg Lys Ile Ser Lys Glu Ser Lys Lys Val Asn Ile Ser Ser
   1 5 10 15
   Ser Leu Glu Ser Glu Asp Ile Ser Leu Glu Thr Thr Val Pro Thr Asp
   20 25 30
   Asp Ile Ser Ser Ser Glu Glu Arg Glu Gly Lys Val Arg Ile Thr Arg
   35 40 45
   Gln Leu Ile Glu Arg Lys Glu Leu Leu His Asn Ile Gln Leu Leu Lys
   50 55 60
   Ile Glu Leu Ser Gln Lys Thr Met Met Ile Asp Asn Leu Lys Val Asp
   65 70 75 80
   Tyr Leu Thr Lys Ile Glu Glu Leu Glu Glu Lys Leu Asn Asp Ala Leu
   85 90 95
   His Gln Lys Gln Leu Leu Thr Leu Arg Leu Asp Asn Gln Leu Ala Phe
   100 105 110
   Gln Gln Lys Asp Ala Ser Lys Tyr Gln Glu Leu Met Lys Gln Glu Met
   115 120 125
   Glu Thr Ile Leu Leu Arg Gln Lys Gln Leu Glu Glu Thr Asn Leu Gln
   130 135 140
   Leu Arg Glu Lys Ala Gly Asp Val Arg Arg Ser Leu Arg Asp Phe Glu
   145 150 155 160
   Leu Thr Glu Glu Gln Tyr Ile Lys Leu Lys Ala Phe Pro Glu Asp Gln
   165 170 175
   Leu Ser Ile Pro Glu Tyr Val Ser Val Arg Phe Tyr Glu Leu Val Asn
   180 185 190
   Pro Leu Arg Lys Glu Ile Cys Glu Leu Gln Val Lys Lys Asn Ile Leu
   195 200 205
   Ala Glu Glu Leu Ser Thr Asn Lys Asn Gln Leu Lys Gln Leu Thr Glu
   210 215 220
   Thr Tyr Glu Glu Asp Arg Lys Asn Tyr Ser Glu Val Gln Ile Arg Cys
   225 230 235 240
   Gln Arg Leu Ala Leu Glu Leu Ala Asp Thr Lys Gln Leu Ile Gln Gln
   245 250 255
   Gly Asp Tyr Arg Gln Glu Asn Tyr Asp Lys Val Lys Ser Glu Arg Asp
   260 265 270
   Ala Leu Glu Gln Glu Val Ile Glu Leu Arg Arg Lys His Glu Ile Leu
   275 280 285
   Glu Ala Ser His Met Ile Gln Thr Lys Glu Arg Ser Glu Leu Ser Lys
   290 295 300
   Glu Val Val Thr Leu Glu Gln Thr Val Thr Leu Leu Gln Lys Asp Lys
   305 310 315 320
   Glu Tyr Leu Asn Arg Gln Asn Met Glu Leu Ser Val Cys Cys Ala His
   325 330 335
   Glu Glu Asp Arg Leu Glu Arg Leu Gln Ala Gln Leu Glu Glu Ser Lys
               340                 345                 350
   Lys Ala Arg Glu Glu Met Tyr Glu Lys Tyr Val Ala Ser Arg Asp His
           355                 360                 365
   Tyr Lys Thr Glu Tyr Glu Asn Lys Leu His Asp Glu Leu Glu Gln Ile
       370                 375                 380
   Arg Leu Lys Thr Asn Gln Glu Ile Asp Gln Leu Arg Asn Ala Ser Arg
   385                 390                 395                 400
   Glu Met Tyr Glu Arg Glu Asn Arg Asn Leu Arg Glu Ala Arg Asp Asn
                   405                 410                 415
   Ala Val Ala Glu Lys Glu Arg Ala Val Met Ala Glu Lys Asp Ala Leu
               420                 425                 430
   Glu Lys His Asp Gln Leu Leu Asp Arg Tyr Arg Glu Leu Gln Leu Ser
           435                 440                 445
   Thr Glu Ser Lys Val Thr Glu Phe Leu His Gln Ser Lys Leu Lys Ser
       450                 455                 460
   Phe Glu Ser Glu Arg Val Gln Leu Leu Gln Glu Glu Thr Ala Arg Asn
   465                 470                 475                 480
   Leu Thr Gln Cys Gln Leu Glu Cys Glu Lys Tyr Gln Lys Lys Leu Glu
                   485                 490                 495
   Val Leu Thr Lys Glu Phe Tyr Ser Leu Gln Ala Ser Ser Glu Lys Arg
               500                 505                 510
   Ile Thr Glu Leu Gln Ala Gln Asn Ser Glu His Gln Ala Arg Leu Asp
           515                 520                 525
   Ile Tyr Glu Lys Leu Glu Lys Glu Leu Asp Glu Ile Ile Met Gln Thr
       530                 535                 540
   Ala Glu Ile Glu Asn Glu Asp Glu Ala Glu Arg Val Leu Phe Ser Tyr
   545                 550                 555                 560
   Gly Tyr Gly Ala Asn Val Pro Thr Thr Ala Lys Arg Arg Leu Lys Gln
                   565                 570                 575
   Ser Val His Leu Ala Arg Arg Val Leu Gln Leu Glu Lys Gln Asn Ser
               580                 585                 590
   Leu Ile Xaa Lys Arg Ser Gly Thr Ser Lys Gly Pro Ser Asn Thr Ala
   595 600 605
   Phe Thr Arg Ser Leu Thr Glu Ala Asn Ser Leu Leu Asn Gln Thr Gln
   610 615 620
   Gln Pro Tyr Arg Tyr Leu Ile Glu Ser Val Arg Gln Arg Asp Ser Lys
   625 630 635 640
   Ile Asp Ser Leu Thr Glu Ser Ile Ala Gln Leu Glu Lys Asp Val Ser
   645 650 655
   Asn Leu Asn Lys Glu Lys Ser Ala Leu Leu Gln Thr Lys Asn Gln Met
   660 665 670
   Ala Leu Asp Leu Glu Gln Leu Leu Asn His Arg Glu Glu Leu Ala Ala
   675 680 685
   Met Lys Gln Ile Leu Val Lys Met His Ser Lys His Ser Glu Asn Ser
   690 695 700
   Leu Leu Leu Thr Lys Thr Glu Pro Lys His Val Thr Glu Asn Gln Lys
   705 710 715 720
   Ser Lys Thr Leu Asn Val Pro Lys Glu His Glu Asp Asn Ile Phe Thr
   725 730 735
   Pro Lys Pro Thr Leu Phe Thr Lys Lys Glu Ala Pro Glu Trp Ser Lys
   740 745 750
   Lys Gln Lys Met Lys Thr
   755
<210> 3
   <211> 2715
   <212> DNA
   <213> Artificial Sequence
<220>
   <223> Description of Artificial Sequence:recombinant DNA
<400> 3
   cgtgttttgc tttccgctcc tcggaacatc cgggagagtt gacttccggc ggcttgtggg 60
   agtgctggtt ctgtcctcct tgcgggtgcg gagatggttg tcttggttac gggtcctaac 120
   ggtcccctgc cttgaaatcc cttgttgagg gcctgcaacc ttgtgcttcc gactggagac 180
   gcctttggtc cctcggtgtc tgcactggct gctggtcaag gcttcagtgt ggacgaattg 240
   acactttcga gaatattaaa atcaaattag agaagaaaac tgatccataa taataaaaat 300
   gtctcgaaaa atttcaaagg agtcaaaaaa agtgaacatc tctagttctc tggaatctga 360
   agatattagt ttagaaacaa cagttcctac ggatgatatt tcctcatcag aagagcgaga 420
   gggcaaagtc agaatcacca ggcagctaat tgaacgaaaa gaactacttc ataatattca 480
   gttactaaaa attgagctat cccagaaaac tatgatgatc gacaatttga aagtggatta 540
   tcttacaaag attgaagaat tggaggagaa acttaatgat gcacttcacc agaagcagct 600
   actaacattg agattagaca accaattggc ttttcaacag aaagatgcca gcaaatatca 660
   agaattaatg aaacaagaaa tggaaaccat tttgttgaga cagaaacaac tagaagagac 720
   aaatcttcag ctaagagaaa aagctggaga tgttcgtcga agcctgcgtg actttgagtt 780
   gacagaagag caatatatta aattaaaagc ttttcctgaa gatcagcttt ctattcctga 840
   atatgtatct gttcgcttct atgagctagt gaatccatta agaaaggaaa tctgtgaact 900
   acaagtgaaa aagaatatcc tagcagaaga attaagtaca aacaaaaacc aactgaagca 960
   gctgacagag acatatgagg aagatcgaaa aaactactct gaagttcaaa ttagatgtca 1020
   acgtttggcc ttagaattag cagacacaaa acagttaatt cagcaaggtg actaccgtca 1080
   agagaactat gataaagtca agagtgaacg tgatgcactt gaacaggaag taattgagct 1140
   taggagaaaa catgaaatac ttgaagcctc tcacatgatt caaacaaaag aacgaagtga 1200
   attatcaaaa gaggtagtca ccttagagca aactgttact ttactgcaaa aggataaaga 1260
   atatcttaat cgccaaaaca tggagcttag tgttcgctgt gctcatgaag aggatcgcct 1320
   tgaaagactt caagctcaac tggaagaaag caaaaaggct agagaagaga tgtatgaaaa 1380
   atatgtagca tccagagacc attataaaac agaatatgaa aataaactac atgatgaact 1440
   agaacaaatc agattgaaaa ccaaccaaga aattgatcaa cttcgaaatg cctctaggga 1500
   aatgtatgaa cgagaaaaca gaaatctccg agaagcaagg gataatgctg tggctgaaaa 1560
   ggaacgagca gtgatggctg aaaaggatgc tttagaaaaa cacgatcagc tcttagacag 1620
   gtacagagaa ctacaactta gtacagaaag caaagtaaca gaatttctcc atcaaagtaa 1680
   attaaaatct tttgaaagtg agcgtgttca acttctgcaa gaggaaacag caagaaatct 1740
   cacacagtgt caattggaat gtgaaaaata tcagaaaaaa ttggaggttt taaccaaaga 1800
   attttatagt ctccaagcct cttctgaaaa acgcattact gaacttcaag cacagaactc 1860
   agagcatcaa gcaaggctag acatttatga gaaactggaa aaagagcttg atgaaataat 1920
   aatgcaaact gcagaaattg aaaatgaaga tgaggctgaa agggttcttt tttcctacgg 1980
   ctatggtgct aatgttccca caacagccaa aagacgacta aagcaaagtg ttcacttggc 2040
   aagaagagtg cttcaattag aaaaacaaaa ctcgctgatt ttaaaagatc tggaacatcg 2100
   aaaggaccaa gtaacacagc tttcacaaga gcttgacaga gccaattcgc tattaaacca 2160
   gactcaacag ccttacaggt atctcattga atcagtgcgt cagagagatt ctaagattga 2220
   ttcactgacg gaatctattg cacaacttga gaaagatgtc agcaacttaa ataaagaaaa 2280
   gtcagcttta ctacagacga agaatcaaat ggcattagat ttagaacaac ttctaaatca 2340
   tcgtgaggaa ttggcagcaa tgaaacagat tctcgttaag atgcatagta aacattctga 2400
   gaacagctta cttctcacta aaacagaacc aaaacatgtg acagaaaatc agaaatcaaa 2460
   gactttgaat gtgcctaaag agcatgaaga caatatattt acacctaaac caacactctt 2520
   tactaaaaaa gaagcacctg agtggtctaa gaaacaaaag atgaagacct agtgttttgg 2580
   atgggaagca cctgtagacc attatatact cctgaagttc tttttctgat ggaaaacaaa 2640
   attcagctta atcgtgtact cagcattttt taaataacaa tgtttatttg aactaatatt 2700
   aaattaacaa attcg                                                  2715
<210> 4
   <211> 756
   <212> PRT
   <213> mouse
<400> 4
   Met Ser Arg Lys Ile Ala Lys Glu Pro Lys Lys Val Asn Ile Ser Ser
   1               5                   10                  15
   Ser Leu Glu Ser Glu Asp Ile Ser Leu Glu Thr Thr Ile His Thr Asp
               20                  25                  30
   Asp Val Ser Ser Ser Glu Glu Arg Glu Gly Lys Val Lys Ile Thr Arg
           35                  40                  45
   Gln Leu Ile Glu Arg Lys Glu Ile Leu His Asn Ile Gln Leu Leu Lys
       50                  55                  60
   Ile Glu Leu Ser Gln Lys Asn Met Met Ile Asp Asn Leu Lys Met Asp
   65                  70                  75                  80
   Tyr Leu Thr Lys Ile Glu Glu Leu Glu Glu Lys Leu Asn Asp Ala Leu
                   85                  90                  95
   His Gln Lys Gln Leu Leu Thr Leu Arg Leu Asp Asn Gln Leu Thr Ile
               100                 105                 110
   Gln Gln Lys Asp Ala Lys Lys Tyr Gln Glu Leu Met Lys Gln Glu Met
           115                 120                 125
   Glu Thr Ile Leu Leu Arg Gln Lys Gln Leu Glu Glu Thr Asn His Gln
       130                 135                 140
   Leu Arg Glu Lys Ala Gly Asp Val Arg Arg Asn Leu Arg Asp Phe Glu
   145                 150                 155                 160
   Leu Thr Glu Glu Gln Tyr Val Lys Leu Lys Ser Phe Pro Glu Asp Gln
                   165                 170                 175
   Leu Ser Ile Pro Glu Tyr Val Ser Ile Arg Phe Tyr Glu Leu Val Asn
               180                 185                 190
   Pro Leu Arg Lys Glu Val Cys Glu Leu Gln Val Lys Lys Ser Glu Leu
           195                 200                 205
   Ser Glu Glu Leu Ser Thr Ser Lys Gly Gln Leu Lys Gln Leu Thr Glu
       210                 215                 220
   Thr Tyr Glu Glu Asp Arg Arg Asn Asn Ala Glu Leu Leu Ile Arg Cys
   225                 230                 235                 240
   Gln Arg Leu Thr Leu Glu Leu Ala Asp Thr Lys Gln Leu Val Gln Gln
                   245                 250                 255
   Gly Asp Tyr Arg Gln Glu Asn Tyr Asp Lys Val Lys Ser Glu Arg Asp
   260 265 270
   Ala Leu Glu Gln Asp Val Leu Glu Leu Arg Arg Lys His Glu Val Leu
   275 280 285
   Glu Ala Ser His Ile Ala Gln Ala Lys Glu Arg Asn Glu Leu Ser Lys
   290 295 300
   Glu Val Ser Ser Leu Gln Gln Thr Val Thr Leu Leu Gln Lys Asp Lys
   305 310 315 320
   Asp Tyr Leu Asn Arg Gln Asn Met Glu Leu Ser Val Arg Cys Ala His
   325 330 335
   Glu Glu Asp Arg Leu Glu Arg Leu Gln Val Gln Leu Glu Asp Thr Lys
   340 345 350
   Lys Ala Arg Glu Glu Met Tyr Glu Lys Tyr Val Thr Ser Arg Asp His
   355 360 365
   Tyr Lys Thr Glu Tyr Glu Asn Lys Leu His Asp Glu Leu Glu Gln Ile
   370 375 380
   Lys Leu Lys Thr Asn Leu Glu Ile Asp Gln Leu Arg Ser Ala Ser Arg
   385 390 395 400
   Glu Met Tyr Glu Arg Glu Asn Arg Asn Leu Arg Glu Ala Arg Asp Asn
   405 410 415
   Ala Leu Ala Glu Lys Asn Arg Ala Val Ala Ala Glu Lys Asp Ala Leu
   420 425 430
   Gly Lys His Glu Gln Leu Leu Asp Arg Tyr Arg Glu Leu Gln Leu Ser
   435 440 445
   Thr Glu Ser Lys Val Ser Glu Phe Leu His Gln Ser Lys Leu Lys Ser
   450 455 460
   Phe Glu Ser Glu Arg Val Gln Leu Leu Gln Glu Glu Thr Ala Arg Asn
   465 470 475 480
   Leu Thr Gln Cys Gln Leu Glu Cys Glu Lys Tyr Gln Lys Lys Leu Glu
   485 490 495
   Val Leu Thr Lys Glu Phe Tyr Ser Leu Gln Thr Ser Ser Glu Lys Arg
   500 505 510
   Ile Thr Glu Leu Glu Ala Gln Asn Ser Glu His Gln Ala Arg Leu Asp
               515                 520                 525
   Ile Tyr Glu Lys Leu Glu Lys Glu Leu Asp Glu Ile Ile Met Gln Thr
           530                 535                 540
   Ala Glu Ile Glu Asn Glu Asp Glu Ala Glu Arg Ile Leu Tyr Ser Tyr
   545                 550                 555                 560
   Gly Tyr Gly Ala Asn Val Pro Thr Thr Ala Lys Arg Arg Leu Lys Gln
                   565                 570                 575
   Ser Val His Leu Ala Arg Arg Val Leu Gln Leu Glu Lys Gln Asn Ser
               580                 585                 590
   Leu Ile Leu Lys Asp Leu Asp His Gln Lys Asn Gln Val Arg Gln Leu
           595                 600                 605
   Ser Gln Glu Leu Asp Arg Ala Asn Ser Leu Leu Asn Gln Thr Gln Gln
       610                 615                 620
   Pro Tyr Arg Tyr Leu Ile Glu Ser Val Arg Gln Arg Asp Ala Lys Ile
   625                 630                 635                 640
   Asp Ser Leu Met Lys Ser Thr Ala Gln Leu Glu Lys Asp Val Ser Asn
                   645                 650                 655
   Leu Asn Lys Glu Lys Ser Ala Leu Leu Gln Thr Lys Asn Gln Met Ala
               660                 665                 670
   Leu Asp Leu Glu Gln Leu Leu Ser His Arg Glu Glu Phe Ala Ala Met
           675                 680                 685
   Lys Gln Ile Ile Ile Asn Met Cys Ser Lys His Ser Glu Asn Asn Leu
       690                 695                 700
   Phe Leu Thr Lys Met Glu Ser Lys Ser Val Thr Glu Asn Gln Ala Lys
   705                 710                 715                 720
   Thr Leu Asn Met Pro Arg Glu His Glu Glu Asn Ile Phe Ile Pro Lys
                   725                 730                 735
   Pro Thr Leu Phe Thr Lys Lys Glu Ala Gln Glu Trp Pro Lys Ser Gln
               740                 745                 750
   Lys Met Lys Thr
           755
<210> 5
   <211> 2719
   <212> DNA
   <213> mouse
<400> 5
   acggccagtg aattgtaata cgactcacta tagggcgaat tgggccctct agatgcatgc 60
   tcgagcggcc gccagtgtga tggatatctg cagaattcgc ccttggttgt cttggttacg 120
   ggtcctaacg gtccggtagc cccgagatac ctgttgaggg gtggcagcct gagctgctga 180
   ctgaagacgc cattggttct tccaagagtc cgggtgaagc tggtgttatc cttatatgcg 240
   gagttactga ccattgagag aagattgatt caaataataa aatgtctcgc aaaattgcca 300
   aggaaccaaa aaaagtaaat atctctagtt ctctggagtc tgaagatatt agtttggaaa 360
   caaccattca tacagatgat gtctcatcat cagaggagcg agaaggtaaa gtcaaaatca 420
   ccaggcagtt aatcgaaaga aaagagatac ttcataatat tcagttactg aaaatcgagc 480
   tatcccagaa aaacatgatg atcgacaact tgaaaatgga ttatcttaca aagattgagg 540
   agctagagga aaaacttaat gacgcccttc accagaagca gctgctaact ttgcgattag 600
   acaatcagtt gactattcaa cagaaagatg ccaaaaaata tcaagaacta atgaaacaag 660
   aaatggaaac cattttattg cgacagaagc aactggaaga aacaaaccat cagctgagag 720
   aaaaggctgg agatgttcgc cgaaatctgc gagactttga gctgacagaa gagcagtatg 780
   tgaagctaaa atcttttcct gaagatcaac tctctattcc tgaatatgta tctattcgct 840
   tctatgagct cgtgaaccca ttaagaaagg aagtctgtga gctacaggtg aagaagagtg 900
   aactctctga agaactgagt acaagtaaag gccaactgaa gcagctgacg gagacatatg 960
   aagaagatcg aagaaacaac gctgaacttc taattcgatg tcaacgtttg accttagaat 1020
   tagcagacac aaaacagtta gttcagcaag gtgattaccg tcaagagaac tatgacaaag 1080
   tgaagagtga acgcgatgct ctggaacagg acgtactcga gcttagaaga aaacacgaag 1140
   tacttgaagc ctctcacata gctcaagcta aggaaaggaa tgaattatca aaggaggtca 1200
   gcagcctgca gcagacagtc accctgctgc agaaggataa agactacctc aatcgccaaa 1260
   acatggaact cagtgtacgc tgtgcccatg aggaggatcg gctggaaagg ctgcaagttc 1320
   aactggaaga caccaaaaag gctagagaag agatgtatga gaaatatgtc acgtccagag 1380
   accattataa aacagaatat gaaaataaac tacatgatga actggaacaa atcaaattga 1440
   aaactaatct agaaattgat cagcttcgaa gtgcctctag ggaaatgtat gaacgagaaa 1500
   acagaaatct ccgtgaagca agggataatg cactcgctga aaagaaccga gcagtggcag 1560
   cggaaaagga cgctctggga aagcatgagc agctcctaga caggtacaga gaactccagc 1620
   tcagtacaga gagcaaggta tctgagtttc tccatcagag caagttgaag tcctttgaaa 1680
   gtgagcgtgt tcaactcctg caagaggaaa ctgcaagaaa tctcacgcag tgccagttgg 1740
   agtgtgaaaa atatcagaag aaattggagg ttttaactaa agaattttat agtctccaaa 1800
   cttcttctga aaaacgcatt accgaactcg aggcacagaa ctcagagcat caggcaaggt 1860
   tagacattta cgagaagttg gaaaaggagc ttgatgagat aataatgcaa acagcagaaa 1920
   ttgaaaatga agatgaggct gaaagaattc tttattccta tggttatggt gctaatgttc 1980
   ccacaacagc taaaagacga ctaaagcaaa gtgtccactt ggcaagaaga gttcttcagt 2040
   tagaaaaaca aaattcatta attttaaaag atctggacca tcaaaagaac caagtaagac 2100
   agctttcaca agagcttgac agagccaatt cactgttgaa tcagactcag cagccctaca 2160
   gatacctcat agagtctgtg cgacagagag atgccaagat tgactcactg atgaagtcta 2220
   cagctcaact tgagaaagat gtcagcaact taaataaaga gaagtcagcc ctgctgcaga 2280
   cgaagaacca gatggcactg gatctggagc agctcctcag tcaccgcgag gaatttgcag 2340
   ctatgaagca gatcatcatt aatatgtgta gtaaacattc tgagaacaac ttatttctta 2400
   cgaaaatgga atcaaaaagt gtgacagaaa atcaagcaaa gactttgaat atgccaagag 2460
   aacatgaaga gaatatattt atacccaagc caacgctctt tactaaaaag gaagcacaag 2520
   agtggcccaa gagtcagaag atgaagacct agtgtttggt gttgaagaaa gctggagcct 2580
   aacccagcca ggccctcggc tctccattgg aacaggcctg tgttatcatg tactcctgaa 2640
   gtgcaatgtc tgttggaagg gcgaattcca gcacactggc ggccgttact agtggatccg 2700
   agctcggtac caagctggc                                              2719
<210> 6
   <211> 298
   <212> PRT
   <213> Artificial Sequence
<220>
   <223> Description of Artificial Sequence:fragment
<400> 6
   Met Ser Arg Lys Ile Ser Lys Glu Ser Lys Lys Val Asn Ile Ser Ser
   1 5 10 15
   Ser Leu Glu Ser Glu Asp Ile Ser Leu Glu Thr Thr Val Pro Thr Asp
   20 25 30
   Asp Ile Ser Ser Ser Glu Glu Arg Glu Gly Lys Val Arg Ile Thr Arg
   35 40 45
   Gln Leu Ile Glu Arg Lys Glu Leu Leu His Asn Ile Gln Leu Leu Lys
   50 55 60
   Ile Glu Leu Ser Gln Lys Thr Met Met Ile Asp Asn Leu Lys Val Asp
   65 70 75 80
   Tyr Leu Thr Lys Ile Glu Glu Leu Glu Glu Lys Leu Asn Asp Ala Leu
   85 90 95
   His Gln Lys Gln Leu Leu Thr Leu Arg Leu Asp Asn Gln Leu Ala Phe
   100 105 110
   Gln Gln Lys Asp Ala Ser Lys Tyr Gln Glu Leu Met Lys Gln Glu Met
   115 120 125
   Glu Thr Ile Leu Leu Arg Gln Lys Gln Leu Glu Glu Thr Asn Leu Gln
   130 135 140
   Leu Arg Glu Lys Ala Gly Asp Val Arg Arg Asn Leu Arg Asp Phe Glu
   145 150 155 160
   Leu Thr Glu Glu Gln Tyr Ile Lys Leu Lys Ala Phe Pro Glu Asp Gln
   165 170 175
   Leu Ser Ile Pro Glu Tyr Val Ser Val Arg Phe Tyr Glu Leu Val Asn
   180 185 190
   Pro Leu Arg Lys Glu Ile Cys Glu Leu Gln Val Lys Lys Asn Ile Leu
   195 200 205
   Ala Glu Glu Leu Ser Thr Asn Lys Asn Gln Leu Lys Gln Leu Thr Glu
   210 215 220
   Glu Leu Ala Ala Met Lys Gln Ile Leu Val Lys Met His Ser Lys His
   225 230 235 240
   Ser Glu Asn Ser Leu Leu Leu Thr Lys Thr Glu Pro Lys His Val Thr
   245 250 255
   Glu Asn Gln Lys Ser Lys Thr Leu Asn Val Pro Arg Glu His Glu Asp
   260 265 270
   Asn Ile Phe Thr Pro Lys Pro Thr Leu Phe Thr Lys Lys Glu Ala Pro
   275 280 285
   Glu Trp Ser Lys Lys Gln Lys Met Lys Thr
   290 295
<210> 7
   <211> 957
   <212> DNA
   <213> Artificial Sequence
<220>
   <223> Description of Artificial Sequence:fragment
<400> 7
   atgtctcgaa aaatttcaaa ggagtcaaaa aaagtgaaca tctctagttc tctggaatct 60
   gaagatatta gtttagaaac aacagttcct acggatgata tttcctcatc agaagagcga 120
   gagggcaaag tcagaatcac caggcagcta attgaacgaa aagaactact tcataatatt 180
   cagttactaa aaattgagct atcccagaaa actatgatga tcgacaattt gaaagtggat 240
   tatcttacaa agattgaaga attggaggag aaacttaatg atgcacttca ccagaagcag 300
   ctactaacat tgagattaga caaccaattg gcttttcaac agaaagatgc cagcaaatat 360
   caagaattaa tgaaacaaga aatggaaacc attttgttga gacagaaaca actagaagag 420
   acaaatcttc agctaagaga aaaagctgga gatgttcgtc gaaacctgcg tgactttgag 480
   ttgacagaag agcaatatat taaattaaaa gcttttcctg aagatcagct ttctattcct 540
   gaatatgtat ctgttcgctt ctatgagcta gtgaatccat taagaaagga aatctgtgaa 600
   ctacaagtga aaaagaatat cctagcagaa gaattaagta caaacaaaaa ccaactgaag 660
   cagctgacag aggaattggc agcaatgaaa cagattctcg ttaagatgca tagtaaacat 720
   tctgagaaca gcttacttct cactaaaaca gaaccaaaac atgtgacaga aaatcagaaa 780
   tcaaagactt tgaatgtgcc taaagagcat gaagacaata tatttacacc taaaccaaca 840
   ctctttacta aaaaagaagc acctgagtgg tctaagaaac aaaagatgaa gacctagtgt 900
   tttggatggg aagcacctgt agaccattat atactcctga agttcttttt ctgatgg    957
<210> 8
   <211> 297
   <212> PRT
   <213> Artificial Sequence
<220>
   <223> Description of Artificial Sequence:fragment
<400> 8
   Met Ser Arg Lys Ile Ala Lys Glu Pro Lys Lys Val Asn Ile Ser Ser
   1 5 10 15
   Ser Leu Glu Ser Glu Asp Ile Ser Leu Glu Thr Thr Ile His Thr Asp
   20 25 30
   Asp Val Ser Ser Ser Glu Glu Arg Glu Gly Lys Val Lys Ile Thr Arg
   35 40 45
   Gln Leu Ile Glu Arg Lys Glu Ile Leu His Asn Ile Gln Leu Leu Lys
   50 55 60
   Ile Glu Leu Ser Gln Lys Asn Met Met Ile Asp Asn Leu Lys Met Asp
   65 70 75 80
   Tyr Leu Thr Lys Ile Glu Glu Leu Glu Glu Lys Leu Asn Asp Ala Leu
   85 90 95
   His Gln Lys Gln Leu Leu Thr Leu Arg Leu Asp Asn Gln Leu Thr Ile
   100 105 110
   Gln Gln Lys Asp Ala Lys Lys Tyr Gln Glu Leu Met Lys Gln Glu Met
   115 120 125
   Glu Thr Ile Leu Leu Arg Gln Lys Gln Leu Glu Glu Thr Asn His Gln
   130 135 140
   Leu Arg Glu Lys Ala Gly Asp Val Arg Arg Asn Leu Arg Asp Phe Glu
   145 150 155 160
   Leu Thr Glu Glu Gln Tyr Val Lys Leu Lys Ser Phe Pro Glu Asp Gln
   165 170 175
   Leu Ser Ile Pro Glu Tyr Val Ser Ile Arg Phe Tyr Glu Leu Val Asn
   180 185 190
   Pro Leu Arg Lys Glu Val Cys Glu Leu Gln Val Lys Lys Ser Glu Leu
   195 200 205
   Ser Glu Glu Leu Ser Thr Ser Lys Gly Gln Leu Lys Gln Leu Thr Glu
   210 215 220
   Glu Phe Ala Ala Met Lys Gln Ile Ile Ile Asn Met Cys Ser Lys His
   225 230 235 240
   Ser Glu Asn Asn Leu Phe Leu Thr Lys Met Glu Ser Lys Ser Val Thr
   245 250 255
   Glu Asn Gln Ala Lys Thr Leu Asn Met Pro Arg Glu His Glu Glu Asn
   260 265 270
   Ile Phe Ile Pro Lys Pro Thr Leu Phe Thr Lys Lys Glu Ala Gln Glu
   275 280 285
   Trp Pro Lys Ser Gln Lys Met Lys Thr
   290 295
<210> 9
   <211> 1173
   <212> DNA
   <213> Artificial Sequence
<220>
   <223> Description of Artificial Sequence:fragment
<400> 9
   gacggccgtg aattgtaata cgactcacta tagggcgaat tgggccctct agatgcatgc 60
   tcgagcggcc gccagtgtga tggatatctg cagaattcgc ccttatgtct cgcaaaattg 120
   ccaaggaacc aaaaaaagta aatatctcta gttctctgga gtctgaagat attagtttgg 180
   aaacaaccat tcatacagat gatgtctcat catcagagga gcgagaaggt aaagtcaaaa 240
   tcaccaggca gttaatcgaa agaaaagaga tacttcataa tattcagtta ctgaaaatcg 300
   agctatccca gaaaaacatg atgatcgaca acttgaaaat ggattatctt acaaagattg 360
   aggagctaga ggaaaaactt aatgacgccc ttcaccagaa gcagctgcta actttgcgat 420
   tagacaatca gttgactatt caacagaaag atgccaaaaa atatcaagaa ctaatgaaac 480
   aagaaatgga aaccatttta ttgcgacaga agcaactgga agaaacaaac catcagctga 540
   gagaaaaggc tggagatgtt cgccgaaatc tgcgagactt tgagctgaca gaagagcagt 600
   atgtgaagct aaaatctttt cctgaagatc aactctctat tcctgaatat gtatctattc 660
   gcttctatga gctcgtgaac ccattaagaa aggaagtctg tgagctacag gtgaagaaga 720
   gtgaactctc tgaagaactg agtacaagta aaggccaact gaagcagctg acggaggaat 780
   ttgcagctat gaagcagatc atcattaata tgtgtagtaa acattctgag aacaacttat 840
   ttcttacgaa aatggaatca aaaagtgtga cagaaaatca agcaaagact ttgaatatgc 900
   caagagaaca tgaagagaat atatttatac ccaagccaac gctctttact aaaaaggaag 960
   cacaagagtg gcccaagagt cagaagatga agacctagtg tttggtgttg aagaaagctg 1020
   gagcctaacc cagccaggcc ctcggctctc cattggaaca ggcctgtgtt atcatgtact 1080
   cctgaagtgc aatgtctgtt ggaagggcga attccagcac actggcggcc gttactagtg 1140
   gatccgagct cggtaccaag cttggcgtaa tca 1173
<210> 10
   <211> 87
   <212> PRT
   <213> Artificial Sequence
<220>
   <223> Description of Artificial Sequence:fragment
<400> 10
   Met Ala Leu Asp Leu Glu Gln Leu Leu Asn His Arg Glu Glu Leu Ala
    1               5                  10                  15
   Ala Met Lys Gln Ile Leu Val Lys Met His Ser Lys His Ser Glu Asn
               20                  25                  30
   Ser Leu Leu Leu Thr Lys Thr Glu Pro Lys His Val Thr Glu Asn Gln
           35                  40                  45
   Lys Ser Lys Thr Leu Asn Val Pro Lys Glu His Glu Asp Asn Ile Phe
       50                  55                  60
   Thr Pro Lys Pro Thr Leu Phe Thr Lys Lys Glu Ala Pro Glu Trp Ser
   65                  70                  75                  80
   Lys Lys Gln Lys Met Lys Thr
                   85
<210> 11
   <211> 983
   <212> DNA
   <213> Artificial Sequence
<220>
   <223> Description of Artificial Sequence:fragment
<400> 11
   acgccagctt ggtaccgagc tcggatccac tagtaacggc cgccagtgtg ctggaattcg 60
   cccttctgcc ttgaaatccc ttgttgaggg cctgcaacct tgtgcttccg actggagacg 120
   cctttggtcc ctcggtgtct gcactggctg ctggtcaagg cttcagtgtg gacgaattga 180
   cactttcgag ttgaaaatga agatgaggct gaaagggttc ttttttccta cggctatggt 240
   gctaatgttc ccacaacagc caaaagacga ctaaagcaaa gtgttcactt ggcaagaaga 300
   gtgcttcaat tagaaaaaca aaactcgctg attttaaaag atctggaaca tcgaaaggac 360
   caagtaacac agctttcaca agagcttgac agagccaatt cgctattaaa ccagactcaa 420
   cagccttaca ggtatctcat tgaatcagtg cgtcagagag attctaagat tgattcactg 480
   acggaatcta ttgcacaact tgagaaagat gtcagcaact taaataaaga aaagtcagct 540
   ttactacaga cgaagaatca aatggcatta gatttagaac aacttctaaa tcatcgtgag 600
   gaattggcag caatgaaaca gattctcgtt aagatgcata gtaaacattc tgagaacagc 660
   ttacttctca ctaaaacaga accaaaacat gtgacagaaa atcagaaatc aaagactttg 720
   aatgtgccta aagagcatga agacaatata tttacaccta aaccaacact ctttactaaa 780
   aaagaagcac ctgagtggtc taagaaacaa aagatgaaga cctagtgttt tggatgggaa 840
   gcacctgtag accattatat actcctgaag ttctttttct gatggaaggg cgaattctgc 900
   agatatccat cacactggcg gccgctcgag catgcatcta gagggcccaa ttcgccctat 960
   agtgagtcgt attacaattc act                                         983
<210> 12
   <211> 689
   <212> DNA
   <213> Artificial Sequence
<220>
   <223> Description of Artificial Sequence:fragment
<400> 12
   ggttgtcttg gttacgggtc ctaacggtcc cctgccttga aatcccttgt tgagggcctg 60
   caaccttgtg cttccgactg gagacgcctt tggtccctcg gtgtctgcac tggctgctgg 120
   tcaaggcttc agtgtggagt aattgacact ttcgagcttg acagagccaa ttcgctatta 180
   aaccagactc aacagcctta caggtatctc attgaatcag tgcgtcagag agattctaag 240
   attgattcac tgacggaatc tattgcacaa cttgagaaag atgtcagcaa cttaaataaa 300
   gaaaagtcag ctttactaca gacgaagaat caaatggcat tagatttaga acaacttcta 360
   aatcatcgtg aggaattggc agcaatgaaa cagattctcg ttaagatgca tagtaaacat 420
   tctgagaaca gcttacttct cactaaaaca gaaccaaaac atgtgacaga aaatcagaaa 480
   tcaaagactt tgaatgtgcc taaagagcat gaagacaata tatttacacc taaaccaaca 540
   ctctttacta aaaaagaagc acctgagtgg tctaagaaac aaaagatgaa gacctagtgt 600
   tttggatggg aagcacctgt agaccattat atactcctga agttcttttt ctgatggaaa 660
   acaaaattca gcttaatcgt gtactcagc                                   689
<210> 13
   <211> 1257
   <212> DNA
   <213> Artificial Sequence
<220>
   <223> Description of Artificial Sequence:fragment
<400> 13
   ccatcagaaa aagaacttca ggacgcgtgg gtttatcgcc cagactggag tgcagtggca 60
   tgatcacagt tcattgcaac ctctgcctcc cagggtcaag gactcctccc tcctcagcct 120
   cccaagtagc tggaactgta gctacgcact actgtgcctg gctaattttt gtattttttt 180
   ggtagagaca gggtttcacc atgttgccca ggctagtcta gaacttctgg gctcaagcga 240
   tccacctgcc tagggcctct gaaagtactg ggattggaga tgtgccactg cacccagcca 300
   agaagttaat attttaaaag ttttaaaaac tatttctctt ataacaaagg gttttttcaa 360
   gtcatacatt aaataacatt aatatatgtt gtttattatt tgttttctta aggatctgtt 420
   tatattcttt agagtgtctt ttcatactat aacattagga ggatctttat cctcaaattt 480
   cgaaagacca gaaatacttc attttattgc agttcctgac acatagttaa tgcttggggt 540
   tgacacagtg gtgtgttggt aaatgttaac agccagcttt ccaagaggga tatgagggag 600
   agcttgattt gtaacattgg cttgtatctc ttttataaat actccccacc atggctgact 660
   tcaaactacc aacctaaggt tactgaagat ggagtaaaga ttgtcagcag cataccagta 720
   cttaatgttt gcaccataca gatacaacag acatagcttg acagagccaa ttcgctatta 780
   aaccagactc aacagcctta caggtatctc attgaatcag tgcgtcagag agattctaag 840
   attgattcac tgacggaatc tattgcacaa cttgagaaag atgtcagcaa cttaaataaa 900
   gaaaagtcag ctttactaca gacgaagaat caaatggcat tagatttaga acaacttcta 960
   aatcatcgtg aggaattggc agcaatgaaa cagattctcg ttaagatgca tagtaaacat 1020
   tctgagaaca gcttacttct cactaaaaca gaaccaaaac atgtgacaga aaatcagaaa 1080
   tcaaagactt tgaatgtgcc taaagagcat gaagacaata tatttacacc taaaccaaca 1140
   ctctttacta aaaaagaagc acctgagtgg tctaagaaac aaaagatgaa gacctagtgt 1200
   tttggatggg aagcacctgt agaccattat atactcctga agttcttttt ctgatgg 1257
<210> 14
   <211> 118
   <212> PRT
   <213> Artificial Sequence
<220>
   <223> Description of Artificial Sequence:fragment
<400> 14
   Met Gln Thr Ala Glu Ile Glu Asn Glu Asp Glu Ala Glu Arg Val Leu
    1               5                  10                  15
   Phe Ser Tyr Gly Tyr Gly Ala Asn Val Pro Thr Thr Ala Lys Arg Arg
               20                  25                  30
   Leu Lys Gln Ser Val His Leu Ala Arg Arg Val Leu Gln Leu Glu Lys
           35                  40                  45
   Gln Asn Ser Leu Ile Leu Lys Asp Leu Glu His Arg Lys Asp Gln Val
       50                  55                  60
   Thr Gln Leu Ser Gln Glu Leu Asp Arg Ala Asn Ser Leu Leu Asn Gln
   65                  70                  75                  80
   Thr Gln Gln Pro Tyr Arg Tyr Leu Ile Glu Ser Val Arg Gln Arg Asp
                   85                  90                  95
   Ser Lys Ile Asp Ser Leu Thr Glu Ser Ile Ala Gln Leu Glu Lys Asp
               100                 105                 110
   Val Arg Asn Trp Gln Gln
           115
<210> 15
   <211> 70
   <212> PRT
   <213> Artificial Sequence
<220>
   <223> Description of Artificial Sequence:fragment
<400> 15
   Met Lys Gln Ile Leu Val Lys Met His Ser Lys His Ser Glu Asn Ser
   1 5 10 15
   Leu Leu Leu Thr Lys Thr Glu Pro Lys His Val Thr Glu Asn Gln Lys
   20 25 30
   Ser Lys Thr Leu Asn Val Pro Lys Glu His Glu Asp Asn Ile Phe Thr
   35 40 45
   Pro Lys Pro Thr Leu Phe Thr Lys Lys Glu Ala Pro Glu Trp Ser Lys
   50 55 60
   Lys Gln Glu Met Lys Thr
   65 70
<210> 16
   <211> 1173
   <212> DNA
   <213> Artificial Sequence
<220>
   <223> Description of Artificial Sequence:fragment
<400> 16
   ggttgtcttg gttacgggtc ctaacggtcc cctgccttga aatcccttgt tgagggcctg 60
   caaccttgtg cttccgactg gagacgcctt tggtccctcg gtgtctgcac tggctgctgg 120
   tcaaggcttc agtgtggagt aattgacact ttcgagagac cattataaaa cagaatatga 180
   aaataaacta catgatgaac tagaacaaat cagattgaaa accaaccaag aaattgatca 240
   acttcgaaat gcctctaggg aaatgtatga acgagaaaac agaaatctcc gagaagcaag 300
   ggataatgct gtggctgaaa aggaacgagc agtgatggct gaaaaggatg ctttagaaaa 360
   acacgatcag ctcttagaca ggttttaacc aaagaatttt atagtctcca agcctcttct 420
   gaaaaacgca ttactgaact tcaagcacag aactcagagc atcaagcaag gctagacatt 480
   tatgagaaac tggaaaaaga gcttgatgaa ataataatgc aaactgcaga aattgaaaat 540
   gaagatgagg ctgaaagggt tcttttttcc tacggctatg gtgctaatgt tcccacaaca 600
   gccaaaagac gactaaagca aagtgttcac ttggcaagaa gagtgcttca attagaaaaa 660
   caaaactcgc tgattttaaa agatctggaa catcgaaagg accaagtaac acagctttca 720
   caagagcttg acagagccaa ttcgctatta aaccagactc aacagcctta caggtatctc 780
   attgaatcag tgcgtcagag agattctaag attgattcac tgacggaatc tattgcacaa 840
   cttgagaaag atgtcaggaa ttggcagcaa tgaaacagat tctcgttaag atgcatagta 900
   aacattctga gaacagctta cttctcacta aaacagaacc aaaacatgtg acagaaaatc 960
   agaaatcaaa gactttgaat gtgcctaaag agcatgaaga caatatattt acacctaaac 1020
   caacactctt tactaaaaaa gaagcacctg agtggtctaa gaaacaagag atgaagacct 1080
   agtgttttgg atgggaagca cctgtagacc attatatact cctgaagttc tttttctgat 1140
   ggaaaacaaa attcagctta atcgtgtact cac                              1173
<210> 17
   <211> 185
   <212> PRT
   <213> Artificial Sequence
<220>
   <223> Description of Artificial Sequence:fragment
<400> 17
   Met Lys Gln Glu Met Glu Thr Ile Leu Leu Arg Gln Lys Gln Leu Glu
   1 5 10 15
   Glu Thr Asn Leu Gln Leu Arg Glu Lys Ala Gly Asp Val Arg Arg Asn
   20 25 30
   Leu Arg Asp Phe Glu Leu Thr Glu Glu Gln Tyr Ile Lys Leu Lys Ala
   35 40 45
   Phe Pro Glu Asp Gln Leu Ser Ile Pro Glu Tyr Val Ser Val Arg Phe
   50 55 60
   Tyr Glu Leu Val Asn Pro Leu Arg Lys Glu Ile Cys Glu Leu Gln Val
   65 70 75 80
   Lys Lys Asn Ile Leu Ala Glu Glu Leu Ser Thr Asn Lys Asn Gln Leu
   85 90 95
   Lys Gln Leu Thr Glu Thr Tyr Glu Glu Asp Arg Lys Asn Tyr Ser Glu
   100 105 110
   Val Gln Ile Arg Cys Gln Arg Leu Ala Leu Glu Leu Ala Asp Thr Lys
   115 120 125
   Gln Leu Ile Gln Gln Gly Asp Tyr Arg Gln Glu Asn Tyr Asp Lys Val
   130 135 140
   Lys Ser Glu Arg Asp Ala Leu Glu Gln Glu Val Ile Glu Leu Arg Arg
   145 150 155 160
   Lys His Glu Ile Leu Glu Ala Ser His Met Ile Gln Thr Lys Glu Arg
   165 170 175
   Ser Glu Leu Ser Lys Glu Arg Pro Leu
   180 185
<210> 18
   <211> 1596
   <212> DNA
   <213> Artificial Sequence
<220>
   <223> Description of Artificial Sequence:fragment
<400> 18
   ccgagctcgg atccactagt aacggccgcc agtgtgctgg aattcgccct tctgccttga 60
   aatcccttgt tgagggcctg caaccttgtg cttccgactg gagacgcctt tggtccctcg 120
   gtgtctgcac tggctgctgg tcaaggcttc agtgtggagt aattgacact ttcgagattg 180
   aagaattgga ggagaaactt aatgatgcac ttcaccagaa gcagctacta acattgagat 240
   tagacaacca attggctttt caacagaaag atgccagcaa atatcaagaa ttaatgaaac 300
   aagaaatgga aaccattttg ttgagacaga aacaactaga agagacaaat cttcagctaa 360
   gagaaaaagc tggagatgtt cgtcgaaacc tgcgtgactt tgagttgaca gaagagcaat 420
   atattaaatt aaaagctttt cctgaagatc agctttctat tcctgaatat gtatctgttc 480
   gcttctatga gctagtgaat ccattaagaa aggaaatctg tgaactacaa gtgaaaaaga 540
   atatcctagc agaagaatta agtacaaaca aaaaccaact gaagcagctg acagagacat 600
   atgaggaaga tcgaaaaaac tactctgaag ttcaaattag atgtcaacgt ttggccttag 660
   aattagcaga cacaaaacag ttaattcagc aaggtgacta ccgtcaagag aactatgata 720
   aagtcaagag tgaacgtgat gcacttgaac aggaagtaat tgagcttagg agaaaacatg 780
   aaatacttga agcctctcac atgattcaaa caaaagaacg aagtgaatta tcaaaagaga 840
   gaccattata aaacagaata tgaaaataaa ctacatgatg aactagaaca aatcagattg 900
   aaaaccaacc aagaaattga tcaacttcga aatgcctcta gggaaatgta tgaacgagaa 960
   aacagaaatc tccgagaagc aagggataat gctgtggctg aaaaggaacg agcagtgatg 1020
   gctgaaaagg atgctttaga aaaacacgat cagctcttag acaggtttta accaaagaat 1080
   tttatagtct ccaagcctct tctgaaaaac gcattactga acttcaagca cagaactcag 1140
   agcatcaagc aaggctagac atttatgaga aactggaaaa agagcttgat gaaataataa 1200
   tgcaaactgc agaaagaatt ggcagcaatg aaacagattc tcgttaagat gcatagtaaa 1260
   cattctgaga acagcttact tctcactaaa acagaaccaa aacatgtgac agaaaatcag 1320
   aaatcaaaga ctttgaatgt gcctaaagag catgaagaca atatatttac acctaaacca 1380
   acactcttta ctaaaaaaga agcacctgag tggtctaaga aacaaaagat gaagacctag 1440
   tgttttggat gggaagcacc tgtagaccat tatatactcc tgaagttctt tttctgatgg 1500
   aagggcgaat tctgcagata tccatcacac tggcggccgc tcgagcatgc atctagaggg 1560
   cccaattcgc cctatagtga gtcgtattac aattca                           1596
<210> 19
   <211> 308
   <212> PRT
   <213> Artificial Sequence
<220>
   <223> Description of Artificial Sequence:fragment
<400> 19
<210> 20
   <211> 258
   <212> PRT
   <213> Artificial Sequence
<220>
   <223> Description of Artificial Sequence:fragment
<400> 20
   Met Tyr Glu Arg Glu Asn Arg Asn Leu Arg Glu Ala Arg Asp Asn Ala
   1 5 10 15
   Val Ala Glu Lys Glu Arg Ala Val Met Ala Glu Lys Asp Ala Leu Glu
   20 25 30
   Lys His Asp Gln Leu Leu Asp Arg Tyr Arg Glu Leu Gln Leu Ser Thr
   35 40 45
   Glu Ser Lys Val Thr Glu Phe Leu His Gln Ser Lys Leu Lys Ser Phe
   50 55 60
   Glu Ser Glu Arg Val Gln Leu Leu Gln Glu Glu Thr Ala Arg Asn Leu
   65 70 75 80
   Thr Gln Cys Gln Leu Glu Cys Glu Lys Tyr Gln Lys Lys Leu Glu Val
   85 90 95
   Leu Thr Lys Glu Phe Tyr Ser Leu Gln Ala Ser Ser Glu Lys Arg Ile
   100 105 110
   Thr Glu Leu Gln Ala Gln Asn Ser Glu His Gln Ala Arg Leu Asp Ile
   115 120 125
   Tyr Glu Lys Leu Glu Lys Glu Leu Asp Glu Ile Ile Met Gln Thr Ala
   130 135 140
   Glu Ile Glu Asn Glu Asp Glu Ala Glu Arg Val Leu Phe Ser Tyr Gly
   145 150 155 160
   Tyr Gly Ala Asn Val Pro Thr Thr Ala Lys Arg Arg Leu Lys Gln Ser
   165 170 175
   Val His Leu Ala Arg Arg Val Leu Gln Leu Glu Lys Gln Asn Ser Leu
   180 185 190
   Ile Leu Lys Asp Leu Glu His Arg Lys Asp Gln Val Thr Gln Leu Ser
   195 200 205
   Gln Glu Leu Asp Arg Ala Asn Ser Leu Leu Asn Gln Thr Gln Gln Pro
   210 215 220
   Tyr Arg Tyr Leu Ile Glu Ser Val Arg Gln Arg Asp Ser Lys Ile Asp
   225 230 235 240
   Ser Leu Thr Glu Ser Ile Ala Gln Leu Glu Lys Asp Val Arg Asn Trp
   245 250 255
   Gln Gln
<210> 21
   <211> 2403
   <212> DNA
   <213> Artificial Sequence
<220>
   <223> Description of Artificial Sequence:fragment
<400> 21 .
   gcttggtacc gagctcggat ccactagtaa cggccgccag tgtgctggaa ttcgcccttc 60
   gccttgaaat cccttgttga gggcctgcaa ccttgtgctt ccgactggag acgcctttgg 120
   tccctcggtg tctgcactgg ctgctggtca aggcttcagt gtggagtaat tgacactttc 180
   gagaatatta aaatcaaatt agagaagaaa actgatccat aataataaaa atgtctcgaa 240
   aaatttcaaa ggagtcaaaa aaagtgaaca tctctagttc tctggaatct gaagatatta 300
   gtttagaaac aacagttcct acggatgata tttcctcatc agaagagcga gagggcaaag 360
   tcagaatcac caggcagcta attgaacgaa aagaactact tcataatatt cagttactaa 420
   aaattgagct atcccagaaa actatgatga tcgacaattt gaaagtggat tatcttacaa 480
   agattgaaga attggaggag aaacttaatg atgcacttca ccagaagcag ctactaacat 540
   tgagattaga caaccaattg gcttttcaac agaaagatgc cagcaaatat caagaattaa 600
   tgaaacaaga aatggaaacc attttgttga gacagaaaca actagaagag acaaatcttc 660
   agctaagaga aaaagctgga gatgttcgtc gaaacctgcg tgactttgag ttgacagaag 720
   agcaatatat taaattaaaa gcttttcctg aagatcagct ttctattcct gaatatgtat 780
   ctgttcgctt ctatgagcta gtgaatccat taagaaagga aatctgtgaa ctacaagtga 840
   aaaagaatat cctagcagaa gaattaagta caaacaaaaa ccaactgaag cagctgacag 900
   agacatatga ggaagatcga aaaaactact ctgaagttca aattagatgt caacgtttgg 960
   ccttagaatt agcagacaca aaacagttaa ttcagcaagg tgactaccgt caagagaact 1020
   atgataaagt caagagtgaa cgtgatgcac ttgaacagga agtaattgag cttaggagaa 1080
   aacatgaaat acttgaagcc tctcacatga ttcaaacaaa agaacgaagt gaattatcaa 1140
   aagagagacc attataaaac agaatatgaa aataaactac atgatgaact agaacaaatc 1200
   agattgaaaa ccaaccaaga aattgatcaa cttcgaaatg cctctaggga aatgtatgaa 1260
   cgagaaaaca gaaatctccg agaagcaagg gataatgctg tggctgaaaa ggaacgagca 1320
   gtgatggctg aaaaggatgc tttagaaaaa cacgatcagc tcttagacag gtacagagaa 1380
   ctacaactta gtacagaaag caaagtaaca gaatttctcc atcaaagtaa attaaaatct 1440
   tttgaaagtg agcgtgttca acttctgcaa gaggaaacag caagaaatct cacacagtgt 1500
   caattggaat gtgaaaaata tcagaaaaaa ttggaggttt taaccaaaga attttatagt 1560
   ctccaagcct cttctgaaaa acgcattact gaacttcaag cacagaactc agagcatcaa 1620
   gcaaggctag acatttatga gaaactggaa aaagagcttg atgaaataat aatgcaaact 1680
   gcagaaattg aaaatgaaga tgaggctgaa agggttcttt tttcctacgg ctatggtgct 1740
   aatgttccca caacagccaa aagacgacta aagcaaagtg ttcacttggc aagaagagtg 1800
   cttcaattag aaaaacaaaa ctcgctgatt ttaaaagatc tggaacatcg aaaggaccaa 1860
   gtaacacagc tttcacaaga gcttgacaga gccaattcgc tattaaacca gactcaacag 1920
   ccttacaggt atctcattga atcagtgcgt cagagagatt ctaagattga ttcactgacg 1980
   gaatctattg cacaacttga gaaagatgtc aggaattggc agcaatgaaa cagattctcg 2040
   ttaagatgca tagtaaacat tctgagaaca gcttacttct cactaaaaca gaaccaaaac 2100
   atgtgacaga aaatcagaaa tcaaagactt tgaatgtgcc taaagagcat gaagacaata 2160
   tatttacacc taaaccaaca ctctttacta aaaaagaagc acctgagtgg tctaagaaac 2220
   aaaagatgaa gacctagtgt tttggatggg aagcacctgt agaccattat atactcctga 2280
   agttcttttt ctgatggaag ggcgaattct gcagatatcc atcacactgg cggccgctcg 2340
   agcatgcatc tagagggccc aattcgccct atagtgagtc gtattacaaa tcacggccgt 2400
   cag                                                               2403
<210> 22
   <211> 1880
   <212> DNA
   <213> Artificial Sequence
<220>
   <223> Description of Artificial Sequence:fragment
<400> 22
   ccgagctcgg atccactagt aacggccgcc agtgtgctgg aattcgccct tctgccttga 60
   aatcccttgt tgagggccta caaccttgtg cttccgactg gagacgcctt tggtccctcg 120
   gtgtctgcac tggctgctgg tcaaggcttc agtgtggagt aattgacact ttcgagaata 180
   ttaaaatcaa attagagaag aaaactgatc cataataata aaaatgtctc gaaaaatttc 240
   aaaggagtca aaaaaagtga acatctctag ttctctggaa tctgaagata ttagtttaga 300
   aacaacagtt cctacggatg atatttcctc atcagaagag cgagagggca aagtcagaat 360
   caccaggcag ctaattgaac gaaaagaact acttcataat attcagttac taaaaattga 420
   gctatcccag aaaactatga tgatcgacaa tttgaaagtg gattatctta caaagattga 480
   agaattggag gagaaactta atgatgcact tcaccagaag cagctactaa cattgagatt 540
   agacaaccaa ttggcttttc aacagaaaga tgccagcaaa tatcaagaat taatgaaaca 600
   agaaatggaa accattttgt tgagacagaa acaactagaa gagacaaatc ttcagctaag 660
   agaaaaagct ggagatgttc gtcgaaacct gcgtgacttt gagttgacag aagagcaata 720
   tattaaatta aaagcttttc ctgaagatca gctttctatt cctgaatatg tatctgttcg 780
   cttctatgag ctagtgaatc cattaagaaa ggaaatctgt gaactacaag tgaaaaagaa 840
   tatcctagca gaagaattaa gtacaaacaa aaaccaactg aagcagctga cagagacata 900
   tgaggaagat cgaaaaaact actctgaagt tcaaattaga tgtcaacgtt tggccttaga 960
   attagcagac acaaaacagt taattcagca aggtgactac cgtcaagaga actatgataa 1020
   agtcaagagt gaacgtgatg cacttgaaca ggaagtaatt gagcttagga gaaaacatga 1080
   aatacttgaa gcctctcaca tgattcaaac aaaagaacga agtgaattat caaaagagag 1140
   accattataa aacagaatat gaaaataaac tacatgatga actagaacaa atcagattga 1200
   aaaccaacca agaaattgat caacttcgaa atgcctctag ggaaatgtat gaacgagaaa 1260
   acagaaatct ccgagaagca agggataatg ctgtggctga aaaggaacga gcagtgatgg 1320
   ctgaaaagga tgctttagaa aaacacgatc agctcttaga caggttttaa ccaaagaatt 1380
   ttatagtctc caagcctctt ctgaaaaacg cattactgaa cttcaagcac agaactcaga 1440
   gcatcaagca aggctagaca tttatgagaa actggaaaaa gagcttgatg aaataataat 1500
   gcaaactgca gaaagaattg gcagcaatga aacagattct cgttaagatg catagtaaac 1560
   attctgagaa cagcttactt ctcactaaaa cagaaccaaa acatgtgaca gaaaatcaga 1620
   aatcaaagac tttgaatgtg cctaaagagc atgaagacaa tatatttaca cctaaaccaa 1680
   cactctttac taaaaaagaa gcacctgagt ggtctaagaa acaaaagatg aagacctagt 1740
   gttttggatg ggaagcacct gtagaccatt atatactcct gaagttcttt ttctgatgga 1800
   agggcgaatt ctgcagatat ccatcacact ggcggccgct cgagcatgca tctagagggc 1860
   ccaattcgcc ctatagtgag 1880
<210> 23
   <211> 157
   <212> PRT
   <213> Artificial Sequence
<220>
   <223> Description of Artificial Sequence:fragment
<400> 23
   Met Ser Arg Lys Ile Ala Lys Glu Pro Lys Lys Val Asn Ile Ser Ser
    1               5                  10                  15
   Ser Leu Glu Ser Glu Asp Ile Ser Leu Glu Thr Thr Ile His Thr Asp
               20                  25                  30
   Asp Val Ser Ser Ser Glu Glu Arg Glu Gly Lys Val Lys Ile Thr Arg
           35                  40                  45
   Gln Leu Ile Glu Arg Lys Glu Ile Leu His Asn Ile Gln Leu Leu Lys
       50                  55                  60
   Ile Glu Leu Ser Gln Lys Asn Met Met Ile Asp Asn Leu Lys Met Asp
   65                  70                  75                  80
   Tyr Leu Thr Lys Glu Phe Ala Ala Met Lys Gln Ile Ile Ile Asn Met
                   85                  90                  95
   Cys Ser Lys His Ser Glu Asn Asn Leu Phe Leu Thr Lys Met Glu Ser
               100                 105                 110
   Lys Ser Val Thr Glu Asn Gln Ala Lys Thr Leu Asn Met Pro Arg Glu
           115                 120                 125
   His Glu Glu Asn Ile Phe Ile Pro Lys Pro Thr Leu Phe Thr Lys Lys
       130                 135                 140
   Glu Ala Gln Glu Trp Pro Lys Ser Gln Lys Met Lys Thr
   145                 150                 155
<210> 24
   <211> 741
   <212> DNA
   <213> Artificial Sequence
<220>
   <223> Description of Artificial Sequence:fragment
<400> 24
   gtgaattgta atacgactca ctatagggcg aattgggccc tctagatgca tgctcgagcg 60
   gccgccagtg tgatggatat ctgcagaatt cgcccttatg tctcgcaaaa ttgccaagga 120
   accgaaaaaa gtaaatatct ctagttctct ggagtctgaa gatattagtt tggaaacaac 180
   cattcataca gatgatgtct catcatcaga ggagcgagaa ggtaaagtca aaatcaccag 240
   gcagttaatc gaaagaaaag agatacttca taatattcag ttactgaaaa tcgagctatc 300
   ccagaaaaac atgatgatcg acaacttgaa aatggattat cttacaaagg aatttgcagc 360
   tatgaagcag atcatcatta atatgtgtag taaacattct gagaacaact tatttcttac 420
   gaaaatggaa tcaaaaagtg tgacagaaaa tcaagcaaag actttgaata tgccaagaga 480
   acatgaagag aatatattta tacccaagcc aacgctcttt actaaaaagg aagcacaaga 540
   gtggcccaag agtcagaaga tgaagaccta gtgtttggtg ttgaagaaag ctggagccta 600
   acccagccag gccctcggct ctccattgga acaggcctgt gttatcatgt actcctgaag 660
   tgcaatgtct gttggaaggg cgaattccag cacactggcg gccgttacta gtggatccga 720
   gctcggtacc aagcttggcg t                                           741
<210> 25
   <211> 256
   <212> PRT
   <213> Artificial Sequence
<220>
   <223> Description of Artificial Sequence:fragment
<400> 25
   Met Ser Arg Lys Ile Ala Lys Glu Pro Lys Lys Val Asn Ile Ser Ser
   1 5 10 15
   Ser Leu Glu Ser Glu Asp Ile Ser Leu Glu Thr Thr Ile His Thr Asp
   20 25 30
   Asp Val Ser Ser Ser Glu Glu Arg Glu Gly Lys Val Lys Ile Thr Arg
   35 40 45
   Gln Leu Ile Glu Arg Lys Glu Ile Leu His Asn Ile Gln Leu Leu Lys
   50 55 60
   Ile Glu Leu Ser Gln Lys Asn Met Met Ile Asp Asn Leu Lys Met Asp
   65 70 75 80
   Tyr Leu Thr Lys Ile Glu Glu Leu Glu Glu Lys Leu Asn Asp Ala Leu
   85 90 95
   His Gln Lys Gln Leu Leu Thr Leu Arg Leu Asp Asn Gln Leu Thr Ile
   100 105 110
   Gln Gln Lys Asp Ala Lys Lys Tyr Gln Glu Leu Met Lys Gln Glu Met
   115 120 125
   Glu Thr Ile Leu Leu Arg Gln Arg Asp Ala Lys Ile Asp Ser Leu Met
   130 135 140
   Lys Ser Thr Ala Gln Leu Glu Lys Asp Val Ser Asn Leu Asn Lys Glu
   145 150 155 160
   Lys Ser Ala Leu Leu Gln Thr Lys Asn Gln Met Ala Leu Asp Leu Glu
   165 170 175
   Gln Leu Leu Ser His Arg Glu Glu Phe Ala Ala Met Lys Gln Ile Ile
   180 185 190
   Ile Asn Met Cys Ser Lys His Ser Glu Asn Asn Leu Phe Leu Thr Lys
   195 200 205
   Met Glu Ser Lys Ser Val Thr Glu Asn Gln Ala Lys Thr Leu Asn Met
   210 215 220
   Pro Arg Glu His Glu Glu Asn Ile Phe Ile Pro Lys Pro Thr Leu Phe
   225 230 235 240
   Thr Lys Lys Glu Ala Gln Glu Trp Pro Lys Ser Gln Lys Met Lys Thr
   245 250 255
<210> 26
   <211> 875
   <212> DNA
   <213> Artificial Sequence
<220>
   <223> Description of Artificial Sequence:fragment
<400> 26
   atgtctcgca aaattgccaa ggaaccaaaa aaagtaaata tctctagttc tctggagtct 60
   gaagatatta gtttggaaac aaccattcat acagatgatg tctcatcatc agaggagcga 120
   gaaggtaaag tcaaaatcac caggcagtta atcgaaagaa aagagatact tcataatatt 180
   cagttactga aaatcgagct atcccagaaa aacatgatga tcgacaactt gaaaatggat 240
   tatcttacaa agattgagga gctagaggaa aaacttaatg acgcccttca ccagaagcag 300
   ctgctaactt tgcgattaga caatcagttg actattcaac agaaagatgc caaaaaatat 360
   caagaactaa tgaaacaaga aatggaaacc attttattgc gacagagaga tgccaagatt 420
   gactcactga tgaagtctac agctcaactt gagaaagatg tcagcaactt aaataaagag 480
   aagtcagccc tgctgcagac gaagaaccag atggcactgg atctggagca gctcctcagt 540
   caccgcgagg aatttgcagc tatgaagcag atcatcatta atatgtgtag taaacattct 600
   gagaacaact tatttcttac gaaaatggaa tcaaaaagtg tgacagaaaa tcaagcaaag 660
   actttgaata tgccaagaga acatgaagag aatatattta tacccaagcc aacgctcttt 720
   actaaaaagg aagcacaaga gtggcccaag agtcagaaga tgaagaccta gtgtttggtg 780
   ttgaagaaag ctggagccta acccagccag gccctcggct ctccattggg acaggcctgt 840
   gttatcatgt actcctgaag tgcaatgtct gttgg 875
<210> 27
   <211> 229
   <212> PRT
   <213> Artificial Sequence
<220>
   <223> Description of Artificial Sequence:fragment
<400> 27
   Met Ser Arg Lys Ile Ala Lys Glu Pro Lys Lys Val Asn Ile Ser Ser
   1 5 10 15
   Ser Leu Glu Ser Glu Asp Ile Ser Leu Glu Thr Thr Ile His Thr Asp
   20 25 30
   Asp Val Ser Ser Ser Glu Glu Arg Glu Gly Lys Val Lys Ile Thr Arg
   35 40 45
   Gln Leu Ile Glu Arg Lys Glu Ile Leu His Asn Ile Gln Leu Leu Lys
   50 55 60
   Ile Glu Leu Ser Gln Lys Asn Met Met Ile Asp Asn Leu Lys Met Asp
   65 70 75 80
   Tyr Leu Thr Lys Leu Asp Arg Ala Asn Ser Leu Leu Asn Gln Thr Gln
   85 90 95
   Gln Pro Tyr Arg Tyr Leu Ile Glu Ser Val Arg Gln Arg Asp Ala Lys
   100 105 110
   Ile Asp Ser Leu Met Lys Ser Thr Ala Gln Leu Glu Lys Asp Val Ser
   115 120 125
   Asn Leu Asn Lys Glu Lys Ser Ala Leu Leu Gln Thr Lys Asn Gln Met
   130 135 140
   Ala Leu Asp Leu Glu Gln Leu Leu Ser His Arg Glu Glu Phe Ala Ala
   145 150 155 160
   Met Lys Gln Ile Ile Ile Asn Met Cys Ser Lys His Ser Glu Asn Asn
   165 170 175
   Leu Phe Leu Thr Lys Met Glu Ser Lys Ser Val Thr Glu Asn Gln Ala
   180 185 190
   Lys Thr Leu Asn Met Pro Arg Glu His Glu Glu Asn Ile Phe Ile Pro
   195 200 205
   Lys Pro Thr Leu Phe Thr Lys Lys Glu Ala Gln Glu Trp Pro Lys Ser
   210 215 220
   Gln Glu Met Lys Thr
   225
<210> 28
   <211> 908
   <212> DNA
   <213> Artificial Sequence
<220>
   <223> Description of Artificial Sequence:fragment
<400> 28
   cctgagctgc tgactgaaga cgccattggt tcttccaaga gtccgggtga agctggtgtt 60
   atccttatat gcggagttac tgaccattga gagaagattg attcaaataa taaaatgtct 120
   cgcaaaattg ccaaggaacc aaaaaaagta aatatctcta gttctctgga gtctgaagat 180
   attagtttgg aaacaaccat tcatacagat gatgtctcat catcagagga gcgagaaggt 240
   aaagtcaaaa tcaccaggca gttaatcgaa agaaaagaga tacttcataa tattcagtta 300
   ctgaaaatcg agctatccca gaaaaacatg atgatcgaca acttgaaaat ggattatctt 360
   acaaagcttg acagagccaa ttcactgttg aatcagactc agcagcccta cagatacctc 420
   atagagtctg tgcgacagag agatgccaag attgactcac tgatgaagtc tacagctcaa 480
   cttgagaaag atgtcagcaa cttaaataaa gagaagtcag ccctgctgca gacgaagaac 540
   cagatggcac tggatctgga gcagctcctc agtcaccgcg aggaatttgc agctatgaag 600
   cagatcatca ttaatatgtg tagtaaacat tctgagaaca acttatttct tacgaaaatg 660
   gaatcaaaaa gtgtgacaga aaatcaagca aagactttga atatgccaag agaacatgaa 720
   gagaatatat ttatacccaa gccaacgctc tttactaaaa aggaagcaca agagtggccc 780
   aagagtcagg agatgaagac ctagtgtttg gtgttgaaga aagctggagc ctaacccagc 840
   caggccctcg gctctccatt ggaacaggcc tgtgttatca tgtactcctg aagtgcaatg 900
   tctgttgg                                                           908
<210> 29
   <211> 512
   <212> PRT
   <213> Artificial Sequence
<220>
   <223> Description of Artificial Sequence:fragment
<400> 29
   Met Ser Arg Lys Ile Ala Lys Glu Pro Lys Lys Val Asn Ile Ser Ser
    1               5                  10                  15
   Ser Leu Glu Ser Glu Asp Ile Ser Leu Glu Thr Thr Ile His Thr Asp
               20                  25                  30
   Asp Val Ser Ser Ser Glu Glu Arg Glu Gly Lys Val Lys Ile Thr Arg
           35                  40                  45
   Gln Leu Ile Glu Arg Lys Glu Ile Leu His Asn Ile Gln Leu Leu Lys
       50                  55                  60
   Ile Glu Leu Ser Gln Lys Asn Met Met Ile Asp Asn Leu Lys Met Asp
   65                  70                  75                  80
   Tyr Leu Thr Lys Ile Glu Glu Leu Glu Glu Lys Leu Asn Asp Ala Leu
                   85                  90                  95
   His Gln Lys Gln Leu Leu Thr Leu Arg Leu Asp Asn Gln Leu Thr Ile
               100                 105                 110
   Gln Gln Lys Asp Ala Lys Lys Tyr Gln Glu Leu Met Lys Gln Glu Met
   115 120 125
   Glu Thr Ile Leu Leu Arg Gln Lys Gln Leu Glu Glu Thr Asn His Gln
   130 135 140
   Leu Arg Glu Lys Ala Gly Asp Val Arg Arg Asn Leu Arg Asp Phe Glu
   145 150 155 160
   Leu Thr Glu Glu Gln Tyr Val Lys Leu Lys Ser Phe Pro Glu Asp Gln
   165 170 175
   Leu Ser Ile Pro Glu Tyr Val Ser Ile Arg Phe Tyr Glu Leu Val Asn
   180 185 190
   Pro Leu Arg Lys Glu Val Cys Glu Leu Gln Val Lys Lys Ser Glu Leu
   195 200 205
   Ser Glu Glu Leu Ser Thr Ser Lys Gly Gln Leu Lys Gln Leu Thr Glu
   210 215 220
   Thr Tyr Glu Glu Asp Arg Arg Asn Asn Ala Glu Leu Leu Ile Arg Cys
   225 230 235 240
   Gln Arg Leu Thr Leu Glu Leu Ala Asp Thr Lys Gln Leu Val Gln Gln
   245 250 255
   Gly Asp Tyr Arg Gln Glu Asn Tyr Asp Lys Val Lys Ser Glu Arg Asp
   260 265 270
   Ala Leu Glu Gln Asp Val Leu Glu Leu Arg Arg Lys His Glu Val Leu
   275 280 285
   Glu Ala Ser His Ile Ala Gln Ala Lys Glu Arg Asn Glu Leu Ser Lys
   290 295 300
   Glu Val Ser Ser Leu Gln Gln Thr Val Thr Leu Leu Gln Lys Asp Lys
   305 310 315 320
   Asp Tyr Leu Asn Arg Gln Asn Met Glu Leu Ser Val Arg Cys Ala His
   325 330 335
   Glu Glu Asp Arg Leu Glu Arg Leu Gln Val Gln Leu Glu Asp Thr Lys
   340 345 350
   Lys Ala Arg Glu Glu Met Tyr Glu Lys Tyr Val Thr Ser Arg Asp His
   355 360 365
   Tyr Lys Thr Glu Tyr Glu Asn Lys Leu His Asp Glu Leu Glu Gln Ile
   370 375 380
   Lys Leu Lys Thr Asn Leu Glu Ile Asp Gln Leu Arg Ser Ala Ser Arg
   385 390 395 400
   Glu Met Tyr Glu Arg Glu Asn Arg Asn Leu Arg Glu Ala Arg Asp Asn
   405 410 415
   Ala Leu Ala Glu Glu Asn Arg Ala Val Ala Ala Glu Lys Asp Ala Leu
   420 425 430
   Gly Lys His Glu Gln Leu Leu Asp Arg Tyr Arg Glu Leu Gln Leu Ser
   435 440 445
   Thr Glu Ser Lys Val Ser Glu Phe Leu His Gln Ser Lys Leu Lys Ser
   450 455 460
   Phe Glu Ser Glu Arg Val Gln Leu Leu Gln Glu Glu Thr Ala Arg Asn
   465 470 475 480
   Leu Thr Gln Cys Gln Leu Glu Cys Glu Lys Tyr Gln Lys Lys Leu Glu
   485 490 495
   Thr Lys Lys Glu Ala Gln Glu Trp Pro Lys Ser Gln Lys Met Lys Thr
   500 505 510
<210> 30
   <211> 1806
   <212> DNA
   <213> Artificial Sequence
<220>
   <223> Description of Artificial Sequence:fragment
<400> 30
   gtgaattgta atacgactca ctatagggcg aattgggccc tctagatgca tgctcgagcg 60
   gccgccagtg tgatggatat ctgcagaatt cgcccttatg tctcgcaaaa ttgccaagga 120
   accaaaaaaa gtaaatatct ctagttctct ggagtctgaa gatattagtt tggaaacaac 180
   cattcataca gatgatgtct catcatcaga ggagcgagaa ggtaaagtca aaatcaccag 240
   gcagttaatc gaaagaaaag agatacttca taatattcag ttactgaaaa tcgagctatc 300
   ccagaaaaac atgatgatcg acaacttgaa aatggattat cttacaaaga ttgaggagct 360
   agaggaaaaa cttaatgacg cccttcacca gaagcagctg ctaactttgc gattagacaa 420
   tcagttgact attcaacaga aagatgccaa aaaatatcaa gaactaatga aacaagaaat 480
   ggaaaccatt ttattgcgac agaagcaact ggaagaaaca aaccatcagc tgagagaaaa 540
   ggctggagat gttcgccgaa atctgcgaga ctttgagctg acagaagagc agtatgtgaa 600
   gctaaaatct tttcccgaag atcaactctc tattcctgaa tatgtatcta ttcgcttcta 660
   tgagctcgtg aacccattaa gaaaggaagt ctgtgagcta caggtgaaga agagtgaact 720
   ctctgaagaa ctgagtacaa gtaaaggcca actgaagcag ctgacggaga catatgaaga 780
   agatcgaaga aacaacgctg aacttctaat tcgatgtcaa cgtttgacct tagaattagc 840
   agacacaaaa cagttagttc agcaaggtga ttaccgtcaa gagaactatg acaaagtgaa 900
   gagtgaacgc gatgctctgg aacaggacgt actcgagctt agaagaaaac acgaagtact 960
   tgaagcctct cacatagctc aagctaagga aaggaatgaa ttatcaaagg aggtcagcag 1020
   cctgcagcag acagtcaccc tgctgcagaa ggataaagac tacctcaatc gccaaaacat 1080
   ggaactcagt gtacgctgtg cccatgagga ggatcggctg gaaaggctgc aagttcaact 1140
   ggaagacacc aaaaaggcta gagaagagat gtatgagaaa tatgtcacgt ccagagacca 1200
   ttataaaaca gaatatgaaa ataaactaca tgatgaactg gaacaaatca aattgaaaac 1260
   taatctagaa attgatcagc ttcgaagtgc ctctagggaa atgtatgaac gagaaaacag 1320
   aaatctccgt gaagcaaggg ataatgcact cgctgaagag aaccgagcag tggcagcgga 1380
   aaaggacgct ctgggaaagc atgagcagct cctagacagg tacagagaac tccagctcag 1440
   tacagagagc aaggtatctg agtttctcca tcagagcaag ttgaagtcct ttgaaagtga 1500
   gcgtgttcaa ctcctgcaag aggaaactgc aagaaatctc acgcagtgcc agttggagtg 1560
   tgaaaaatat cagaagaaat tggagactaa aaaggaagca caagagtggc ccaagagtca 1620
   gaagatgaag acctagtgtt tggtgttgaa gaaagctgga gcctaaccca gccaggccct 1680
   cggctctcca tcggaacagg cctgtgttat catgtactcc tgaagtgcaa tgtctgttgg 1740
   aagggcgaat tccagcacac tggcggccgt tactagtgga tccgagctcg gtaccaagct 1800
   tggcgt                                                            1806
<210> 31
   <211> 569
   <212> PRT
   <213> Artificial Sequence
<220>
   <223> Description of Artificial Sequence:fragment
<400> 31
   Met Ser Arg Lys Ile Ala Lys Glu Pro Lys Lys Val Asn Ile Ser Ser
    1               5                  10                  15
   Ser Leu Glu Ser Glu Asp Ile Ser Leu Glu Thr Thr Ile His Thr Asp
               20                  25                  30
   Asp Val Ser Ser Ser Glu Glu Arg Glu Gly Lys Val Lys Ile Thr Arg
           35                  40                  45
   Gln Leu Ile Glu Arg Lys Glu Ile Leu His Asn Ile Gln Leu Leu Lys
       50                  55                  60
   Ile Glu Leu Ser Gln Lys Asn Met Met Ile Asp Asn Leu Lys Met Asp
   65                  70                  75                  80
   Tyr Leu Thr Lys Ile Glu Glu Leu Glu Glu Lys Leu Asn Asp Ala Leu
                   85                  90                  95
   His Gln Lys Gln Leu Leu Thr Leu Arg Leu Asp Asn Gln Leu Thr Ile
               100                 105                 110
   Gln Gln Lys Asp Ala Lys Lys Tyr Gln Glu Leu Met Lys Gln Glu Met
           115                 120                 125
   Glu Thr Ile Leu Leu Arg Gln Lys Gln Leu Glu Glu Thr Asn His Gln
       130                 135                 140
   Leu Arg Glu Lys Ala Gly Asp Val Arg Arg Asn Leu Arg Asp Phe Glu
   145                 150                 155                 160
   Leu Thr Glu Glu Gln Tyr Val Lys Leu Lys Ser Phe Pro Glu Asp Gln
                   165                 170                 175
   Leu Ser Ile Pro Glu Tyr Val Ser Ile Arg Phe Tyr Glu Leu Val Asn
               180                 185                 190
   Pro Leu Arg Lys Glu Val Cys Glu Leu Gln Val Lys Lys Ser Glu Leu
           195                 200                 205
   Ser Glu Glu Leu Ser Thr Ser Lys Gly Gln Leu Lys Gln Leu Thr Glu
       210                 215                 220
   Thr Tyr Glu Glu Asp Arg Arg Asn Asn Ala Glu Leu Leu Ile Arg Cys
   225                 230                 235                 240
   Gln Arg Leu Thr Leu Glu Leu Ala Asp Thr Lys Gln Leu Val Gln Gln
                   245                 250                 255
   Gly Asp Tyr Arg Gln Glu Asn Tyr Asp Lys Val Lys Ser Glu Arg Asp
               260                 265                 270
   Ala Leu Glu Gln Asp Val Leu Glu Leu Arg Arg Lys His Glu Val Leu
           275                 280                 285
   Glu Ala Ser His Ile Ala Gln Ala Lys Glu Arg Asn Glu Leu Ser Lys
       290                 295                 300
   Glu Val Ser Ser Leu Gln Gln Thr Val Thr Leu Leu Gln Lys Asp Lys
   305                 310                 315                 320
   Asp Tyr Leu Asn Arg Gln Asn Met Glu Leu Ser Val Arg Cys Ala His
                   325                 330                 335
   Glu Glu Asp Arg Leu Glu Arg Leu Gln Val Gln Leu Glu Asp Thr Lys
   340 345 350
   Lys Ala Arg Glu Glu Met Tyr Glu Lys Tyr Val Thr Ser Arg Asp His
   355 360 365
   Tyr Lys Thr Glu Tyr Glu Asn Lys Leu His Asp Glu Leu Glu Gln Ile
   370 375 380
   Lys Leu Lys Thr Asn Leu Glu Ile Asp Gln Leu Arg Ser Ala Ser Arg
   385 390 395 400
   Glu Met Tyr Glu Arg Glu Asn Arg Asn Leu Arg Glu Ala Arg Asp Asn
   405 410 415
   Ala Leu Ala Glu Lys Asn Arg Ala Val Ala Ala Glu Lys Asp Ala Leu
   420 425 430
   Gly Lys His Glu Gln Leu Leu Asp Arg Tyr Arg Glu Leu Gln Leu Ser
   435 440 445
   Thr Glu Ser Lys Val Ser Glu Phe Leu His Gln Ser Lys Leu Lys Ser
   450 455 460
   Phe Glu Ser Glu Arg Val Gln Leu Leu Gln Glu Glu Thr Ala Arg Asn
   465 470 475 480
   Leu Thr Gln Cys Gln Leu Glu Cys Glu Lys Tyr Gln Lys Lys Leu Glu
   485 490 495
   Glu Phe Ala Ala Met Lys Gln Ile Ile Ile Asn Met Cys Ser Lys His
   500 505 510
   Ser Glu Asn Asn Leu Phe Leu Thr Lys Met Glu Ser Lys Ser Val Thr
   515 520 525
   Glu Asn Gln Ala Lys Thr Leu Asn Met Pro Arg Glu His Glu Glu Asn
   530 535 540
   Ile Phe Ile Pro Lys Pro Thr Leu Phe Thr Lys Lys Glu Ala Gln Glu
   545 550 555 560
   Trp Pro Lys Ser Gln Lys Met Lys Thr
   565
<210> 32
   <211> 86
   <212> PRT
   <213> Artificial Sequence
<220>
   <223> Description of Artificial Sequence:fragment
<400> 32
   Met Asn Tyr Gln Arg Arg Ser Ala Ala Cys Ser Arg Gln Ser Pro Cys
    1               5                  10                  15
   Cys Arg Arg Ile Lys Thr Thr Ser Ile Ala Lys Thr Trp Asn Ser Val
               20                  25                  30
   Tyr Ala Val Pro Met Arg Arg Ile Gly Trp Lys Gly Cys Lys Phe Asn
           35                  40                  45
   Trp Lys Thr Pro Lys Arg Leu Glu Lys Arg Cys Met Arg Asn Met Ser
       50                  55                  60
   Arg Pro Glu Thr Ile Ile Lys Gln Asn Met Lys Ile Asn Tyr Met Met
   65                  70                  75                  80
   Asn Trp Asn Lys Ser Asn
                   85
<210> 33
   <211> 1963
   <212> DNA
   <213> Artificial Sequence
<220>
   <223> Description of Artificial Sequence:fragment
<400> 33
   cgactcacta tagggcgaat tgggccctct agatgcatgc tcgagcggcc gccagtgtga 60
   tggatatctg cagaattcgc ccttatgtct cgcaaaattg ccaaggaacc aaaaaaagta 120
   aatatctcta gttctctgga gtctgaagat attagtttgg aaacaaccat tcatacagat 180
   gatgtctcat catcagagga gcgagaaggt aaagtcaaaa tcaccaggca gttaatcgaa 240
   agaaaagaga tacttcataa tattcagtta ctgaaaatcg agctatccca gaaaaacatg 300
   atgatcgaca acttgaaaat ggattatctt acaaagattg aggagctaga ggaaaaactt 360
   aatgacgccc ttcaccagaa gcagctgcta actttgcgat tagacaatca gttgactatt 420
   caacagaaag atgccaaaaa atatcaagaa ctaatgaaac aagaaatgga aaccatttta 480
   ttgcgacaga agcaactgga agaaacaaac catcagctga gagaaaaggc tggagatgtt 540
   cgccgaaatc tgcgagactt tgagctgaca gaagagcagt atgtgaagct aaaatctttt 600
   cctgaagatc aactctctat tcctgaatat gtatctattc gcttctatga gctcgtgaac 660
   ccattaagaa aggaagtctg tgagctacag gtgaagaaga gtgaactctc tgaagaactg 720
   agtacaagta aaggccaact gaagcagctg acggagacat atgaagaaga tcgaagaaac 780
   aacgctgaac ttctaattcg atgtcaacgt ttgaccttag aattagcaga cacaaaacag 840
   ttagttcagc aaggtgatta ccgtcaagag aactatgaca aagtgaagag tgaacgcgat 900
   gctctggaac aggacgtact cgagcttaga agaaaacacg aagtacttga agcctctcac 960
   atagctcaag ctaaggaaag gaatgaatta tcaaaggagg tcagcagcct gcagcagaca 1020
   gtcaccctgc tgcagaagga taaagactac ctcaatcgcc aaaacatgga actcagtgta 1080
   cgctgtgccc atgaggagga tcggctggaa aggctgcaag ttcaactgga agacaccaaa 1140
   aaggctagag aagagatgta tgagaaatat gtcacgtcca gagaccatta taaaacagaa 1200
   tatgaaaata aactacatga tgaactggaa caaatcaaat tgaaaactaa tctagaaatt 1260
   gatcagcttc gaagtgcctc tagggaaatg tatgaacgag aaaacagaaa tctccgtgaa 1320
   gcaagggata atgcactcgc tgaaaagaac cgagcagtgg cagcggaaaa ggacgctctg 1380
   ggaaagcatg agcagctcct agacaggtac agagaactcc agctcagtac agagagcaag 1440
   gtatctgagt ttctccatca gagcaagttg aagtcctttg aaagtgagcg tgttcaactc 1500
   ctgcaagagg aaactgcaag aaatctcacg cagtgccagt tggagtgtga aaaatatcag 1560
   aagaaattgg aggaatttgc agctatgaag cagatcatca ttaatatgtg tagtaaacat 1620
   tctgagaaca acttatttct tacgaaaatg gaatcaaaaa gtgtgacaga aaatcaagca 1680
   aagactttga atatgccaag agaacatgaa gagaatatat ttatacccaa gccaacgctc 1740
   tttactaaaa aggaagcaca agagtggccc aagagtcaga agatgaagac ctagtgtttg 1800
   gtgttgaaga aagctggagc ctaacccagc caggccctcg gctctccatt ggaacaggcc 1860
   tgtgttatca tgtactcctg aagtgcaatg tctgttggaa gggcgaattc cagcacactg 1920
   gcggccgtta ctagtggatc cgagctcggt accaagcttg gcg 1963
<210> 34
   <211> 309
   <212> PRT
   <213> Artificial Sequence
<220>
   <223> Description of Artificial Sequence:fragment
<400> 34
   Met Tyr Glu Lys Tyr Val Thr Ser Arg Asp His Tyr Lys Thr Glu Tyr
   1 5 10 15
   Glu Asn Lys Leu His Asp Glu Leu Glu Gln Ile Lys Leu Lys Thr Asn
   20 25 30
   Leu Glu Ile Asp Gln Leu Arg Ser Ala Ser Arg Glu Met Tyr Glu Arg
   35 40 45
   Glu Asn Arg Asn Leu Arg Glu Ala Arg Asp Asn Ala Leu Ala Glu Lys
   50 55 60
   Asn Arg Ala Val Ala Ala Glu Lys Asp Ala Leu Gly Lys His Glu Gln
   65 70 75 80
   Leu Leu Asp Arg Tyr Arg Glu Leu Gln Leu Ser Thr Glu Ser Lys Val
   85 90 95
   Ser Glu Phe Leu His Gln Ser Lys Leu Lys Ser Phe Glu Ser Glu Arg
               100                 105                 110
   Val Gln Leu Leu Gln Glu Glu Thr Ala Arg Asn Leu Thr Gln Cys Gln
           115                 120                 125
   Leu Glu Cys Glu Lys Tyr Gln Lys Lys Leu Glu Val Leu Thr Lys Glu
       130                 135                 140
   Phe Tyr Ser Leu Gln Thr Ser Ser Glu Lys Arg Ile Thr Glu Leu Glu
   145                 150                 155                 160
   Ala Gln Asn Ser Glu His Gln Ala Arg Leu Asp Ile Tyr Glu Lys Leu
                   165                 170                 175
   Glu Lys Glu Leu Asp Glu Ile Ile Met Gln Thr Ala Glu Ile Glu Asn
               180                 185                 190
   Glu Asp Glu Ala Glu Arg Ile Leu Tyr Ser Tyr Gly Tyr Gly Ala Asn
           195                 200                 205
   Val Pro Thr Thr Ala Lys Arg Arg Leu Lys Gln Ser Val His Leu Ala
       210                 215                 220
   Arg Arg Val Leu Gln Leu Glu Lys Gln Asn Ser Leu Ile Leu Lys Asp
   225                 230                 235                 240
   Leu Glu Gln Ile Ile Ile Asn Met Cys Ser Lys His Ser Glu Asn Asn
                   245                 250                 255
   Leu Phe Leu Thr Lys Met Glu Ser Ile Ser Val Thr Glu Asn Gln Thr
               260                 265                 270
   Lys Thr Leu Asn Met Pro Arg Glu His Glu Glu Asn Ile Phe Ile Pro
           275                 280                 285
   Lys Pro Thr Leu Phe Thr Lys Lys Glu Ala Gln Glu Trp Pro Lys Ser
       290                 295                 300
   Gln Lys Met Lys Thr
   305
<210> 35
   <211> 1169
   <212> DNA
   <213> Artificial Sequence
<220>
   <223> Description of Artificial Sequence:fragment
<400> 35
   ggttgtcttg gtgaccgatc ctaacggtcc ggtagccccg agatacctgt tgaggggtgg 60
   cagcctgagc tgctgactga agacaccaaa aaggctagag aagagatgta tgagaaatat 120
   gtcacgtcca gagaccatta taaaacagaa tatgaaaata aactacatga tgaactggaa 180
   caaatcaaat tgaaaactaa tctagaaatt gatcagcttc gaagtgcctc tagggaaatg 240
   tatgaacgag aaaacagaaa tctccgtgaa gcaagggata atgcactcgc tgaaaagaac 300
   cgagcagtgg cagcggaaaa ggacgctctg ggaaagcatg agcagctcct agacaggtac 360
   agagaactcc agctcagtac agagagcaag gtatctgagt ttctccatca gagcaagttg 420
   aagtcctttg aaagtgagcg tgttcaactc ctgcaagagg aaactgcaag aaatctcacg 480
   cagtgccagt tggagtgtga aaaatatcag aagaaattgg aggttttaac taaagaattt 540
   tatagtctcc aaacttcttc tgaaaaacgc attaccgaac tcgaggcaca gaactcagag 600
   catcaggcaa ggttagacat ttacgagaag ttggaaaagg agcttgatga gataataatg 660
   caaacagcag aaattgaaaa tgaagatgag gctgaaagaa ttctttattc ctatggttat 720
   ggtgctaatg ttcccacaac agctaaaaga cgactaaagc aaagtgtcca cttggcaaga 780
   agagttcttc agttagaaaa acaaaattca ttaattttaa aagacctgga gcagatcatc 840
   attaatatgt gtagtaaaca ttctgagaac aacttatttc ttacgaaaat ggaatcaata 900
   agtgtgacag aaaatcaaac aaagactttg aatatgccaa gagaacatga agagaatata 960
   tttataccca agccaacgct ctttactaaa aaggaagcac aagagtggcc caagagtcag 1020
   aagatgaaga cctagtgttt ggtgttgaag aaagctggag cctaacccag ccaggccctc 1080
   ggctctccat tggaacaggc ctgtgttatc atgtactcct gaagtgcaat gtctgttggg 1140
   gagaaaggct cacttcaatc atgtacttg 1169
<210> 36
   <211> 641
   <212> PRT
   <213> Artificial Sequence
<220>
   <223> Description of Artificial Sequence:fragment
<400> 36
   Met Ser Arg Lys Ile Ala Lys Glu Pro Lys Lys Val Asn Ile Ser Ser
   1 5 10 15
   Ser Leu Glu Ser Glu Asp Ile Ser Leu Glu Thr Thr Ile His Thr Asp
   20 25 30
   Asp Val Ser Ser Ser Glu Glu Arg Glu Gly Lys Val Lys Ile Thr Arg
   35 40 45
   Gln Leu Ile Glu Arg Lys Glu Ile Leu His Asn Ile Gln Leu Leu Lys
   50 55 60
   Ile Glu Leu Ser Gln Lys Asn Met Met Ile Asp Asn Leu Lys Met Asp
   65 70 75 80
   Tyr Leu Thr Lys Ile Glu Glu Leu Glu Glu Lys Leu Asn Asp Ala Leu
   85 90 95
   His Gln Lys Gln Leu Leu Thr Leu Arg Leu Asp Asn Gln Leu Thr Ile
   100 105 110
   Gln Gln Lys Asp Ala Lys Lys Tyr Gln Glu Leu Met Lys Gln Glu Met
   115 120 125
   Glu Thr Ile Leu Leu Arg Gln Lys Gln Leu Glu Glu Thr Asn His Gln
   130 135 140
   Leu Arg Glu Lys Ala Gly Asp Val Arg Arg Asn Leu Arg Asp Phe Glu
   145 150 155 160
   Leu Thr Glu Glu Gln Tyr Val Lys Leu Lys Ser Phe Pro Glu Asp Gln
   165 170 175
   Leu Ser Ile Pro Glu Tyr Val Ser Ile Arg Phe Tyr Glu Leu Val Asn
   180 185 190
   Pro Leu Arg Lys Glu Val Cys Glu Leu Gln Val Lys Lys Ser Glu Leu
   195 200 205
   Ser Glu Glu Leu Ser Thr Ser Lys Gly Gln Leu Lys Gln Leu Thr Glu
   210 215 220
   Thr Tyr Glu Glu Asp Arg Arg Asn Asn Ala Glu Leu Leu Ile Arg Cys
   225 230 235 240
   Gln Arg Leu Thr Leu Glu Leu Ala Asp Thr Lys Gln Leu Val Gln Gln
   245 250 255
   Gly Asp Tyr Arg Gln Glu Asn Tyr Asp Lys Val Lys Ser Glu Arg Asp
   260 265 270
   Ala Leu Glu Gln Asp Val Leu Glu Leu Arg Arg Lys His Glu Val Leu
   275 280 285
   Glu Ala Ser His Ile Ala Gln Ala Lys Glu Arg Asn Glu Leu Ser Lys
   290 295 300
   Glu Val Ser Ser Leu Gln Gln Thr Val Thr Leu Leu Gln Lys Asp Lys
   305 310 315 320
   Asp Tyr Leu Asn Arg Gln Asn Met Glu Leu Ser Val Arg Cys Ala His
   325 330 335
   Glu Glu Asp Arg Leu Glu Arg Leu Gln Val Gln Leu Glu Asp Thr Lys
   340 345 350
   Lys Ala Arg Glu Glu Met Tyr Glu Lys Tyr Val Thr Ser Arg Asp His
   355 360 365
   Tyr Lys Thr Glu Tyr Glu Asn Lys Leu His Asp Glu Leu Glu Gln Ile
   370 375 380
   Lys Leu Lys Thr Asn Leu Glu Ile Asp Gln Leu Arg Ser Ala Ser Arg
   385 390 395 400
   Glu Met Tyr Glu Arg Glu Asn Arg Asn Leu Arg Glu Ala Arg Asp Asn
   405 410 415
   Ala Leu Ala Glu Lys Asn Arg Ala Val Ala Ala Lys Lys Asp Ala Leu
   420 425 430
   Gly Lys His Glu Gln Leu Leu Asp Arg Tyr Arg Glu Leu Gln Leu Ser
   435 440 445
   Thr Glu Ser Lys Val Ser Glu Phe Leu His Gln Ser Lys Leu Lys Ser
   450 455 460
   Phe Glu Ser Glu Arg Val Gln Leu Leu Gln Glu Glu Thr Ala Arg Asn
   465 470 475 480
   Leu Thr Gln Cys Gln Leu Glu Cys Glu Lys Tyr Gln Lys Lys Leu Glu
   485 490 495
   Leu Asp Arg Ala Asn Ser Leu Leu Asn Gln Thr Gln Gln Pro Tyr Arg
   500 505 510
   Tyr Leu Ile Glu Ser Val Arg Gln Arg Asp Ala Lys Ile Asp Ser Leu
   515 520 525
   Met Lys Ser Thr Ala Gln Leu Glu Glu Asp Val Ser Asn Leu Asn Lys
   530 535 540
   Glu Lys Ser Ala Leu Leu Gln Thr Lys Asn Gln Met Ala Leu Asp Leu
   545 550 555 560
   Glu Gln Leu Leu Ser His Arg Glu Glu Phe Ala Ala Met Lys Gln Ile
   565 570 575
   Ile Ile Asn Met Cys Ser Lys His Ser Glu Asn Asn Leu Phe Leu Thr
   580 585 590
   Lys Met Glu Ser Lys Ser Val Thr Glu Asn Gln Ala Lys Thr Leu Asn
           595                 600                 605
   Met Pro Arg Glu His Glu Glu Asn Ile Phe Ile Pro Lys Pro Thr Leu
       610                 615                 620
   Phe Thr Lys Lys Glu Ala Gln Glu Trp Pro Lys Ser Gln Lys Met Lys
   625                 630                 635                 640
   Thr
<210> 37
   <211> 2144
   <212> DNA
   <213> Artificial Sequence
<220>
   <223> Description of Artificial Sequence:fragment
<400> 37
   cctgagctgc tgactgaaga cgccattggt tcttccaaga gtccgggtga agctggtgtt 60
   atccttatat gcggagttac tgaccattga gagaagattg attcaaataa taaaatgtct 120
   cgcaaaattg ccaaggaacc aaaaaaagta aatatctcta gttctctgga gtctgaagat 180
   attagtttgg aaacaaccat tcatacagat gatgtctcat catcagagga gcgagaaggt 240
   aaagtcaaaa tcaccaggca gttaatcgaa agaaaagaga tacttcataa tattcagtta 300
   ctgaaaatcg agctatccca gaaaaacatg atgatcgaca acttgaaaat ggattatctt 360
   acaaagattg aggagctaga ggaaaaactt aatgacgccc ttcaccagaa gcagctgcta 420
   actttgcgat tagacaatca gttgactatt caacagaaag atgccaaaaa atatcaagaa 480
   ctaatgaaac aagaaatgga aaccatttta ttgcgacaga agcaactgga agaaacaaac 540
   catcagctga gagaaaaggc tggagatgtt cgccgaaatc tgcgagactt tgagctgaca 600
   gaagagcagt atgtgaagct aaaatctttt cctgaagatc aactctctat tcctgaatat 660
   gtatctattc gcttctatga gctcgtgaac ccattaagaa aggaagtctg tgagctacag 720
   gtgaagaaga gtgaactctc tgaagaactg agtacaagta aaggccaact gaagcagctg 780
   acggagacat atgaagaaga tcgaagaaac aacgctgaac ttctaattcg atgtcaacgt 840
   ttgaccttag aattagcaga cacaaaacag ttagttcagc aaggtgatta ccgtcaagag 900
   aactatgaca aagtgaagag tgaacgcgat gctctggaac aggacgtact cgagcttaga 960
   agaaaacacg aagtacttga agcctcacac atagctcaag ctaaggaaag gaatgaatta 1020
   tcaaaggagg tcagcagcct gcagcagaca gtcaccctgc tgcagaagga taaagactac 1080
   ctcaatcgcc aaaacatgga actcagtgta cgctgtgccc atgaggagga tcggctggaa 1140
   aggctgcaag ttcaactgga agacaccaaa aaggctagag aagagatgta tgagaaatat 1200
   gtcacgtcca gagaccatta taaaacagaa tatgaaaata aactacatga tgaactggaa 1260
   caaatcaaat tgaaaactaa tctagaaatt gatcagcttc gaagtgcctc tagggaaatg 1320
   tatgaacgag aaaacagaaa tctccgtgaa gcaagggata atgcactcgc tgaaaagaac 1380
   cgagcagtgg cagcgaaaaa ggacgctctg ggaaagcatg agcagctcct agacaggtac 1440
   agagaactcc agctcagcac agagagcaag gtatctgagt ttctccatca gagcaagttg 1500
   aagtcctttg aaagtgagcg tgttcaactc ctgcaagagg aaactgcaag aaatctcacg 1560
   cagtgccagt tggagtgtga aaaatatcag aagaaattgg agcttgacag agccaattca 1620
   ctgttgaatc agactcagca gccctacaga tacctcatag agtctgtgcg acagagagat 1680
   gccaagattg actcactgat gaagtctaca gctcaacttg aggaagatgt cagcaactta 1740
   aataaagaga agtcagccct gctgcagacg aagaaccaga tggcactgga tctggagcag 1800
   ctcctcagtc accgcgagga atttgcagct atgaagcaga tcatcattaa tatgtgtagt 1860
   aaacattctg agaacaactt atttcttacg aaaatggaat caaaaagtgt gacagaaaat 1920
   caagcaaaga ctttgaatat gccaagagaa catgaagaga atatatttat acccaagcca 1980
   acgctcttta ctaaaaagga agcacaagag tggcccaaga gtcagaagat gaagacctag 2040
   tgtttggtgt tgaagaaggc tggagcctaa cccagccagg ccctcggctc tccattggaa 2100
   caggcctgtg ttatcatgta ctcctgaagt gcaatgtctg ttgg 2144